(19) Europäisches Patentamt
European Patent Office
Office européen des brevets

(11) EP 4 259 617 B1

(12) # EUROPEAN PATENT SPECIFICATION

(45) Date of publication and mention of the grant of the patent:
**23.10.2024 Bulletin 2024/43**

(21) Application number: **21823605.7**

(22) Date of filing: **09.12.2021**

(51) International Patent Classification (IPC):
***C07D 401/04*** (2006.01) ***A61P 7/00*** (2006.01)
***A61P 9/00*** (2006.01) ***A61P 11/00*** (2006.01)
***A61P 13/12*** (2006.01) ***A61K 31/496*** (2006.01)

(52) Cooperative Patent Classification (CPC):
**C07D 401/04; A61P 7/00; A61P 9/00; A61P 11/00; A61P 13/12**

(86) International application number:
**PCT/EP2021/084987**

(87) International publication number:
**WO 2022/122914 (16.06.2022 Gazette 2022/24)**

(54) **SUBSTITUTED PYRAZOLO PIPERIDINE CARBOXYLIC ACIDS**

SUBSTITUIERTE PYRAZOLOPIPERIDINCARBONSÄUREN

ACIDES PYRAZOLO PIPÉRIDINE CARBOXYLIQUES SUBSTITUÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**ME**
Designated Validation States:
**MA TN**

(30) Priority: **10.12.2020 EP 20213020**

(43) Date of publication of application:
**18.10.2023 Bulletin 2023/42**

(73) Proprietor: **Bayer Aktiengesellschaft**
**51373 Leverkusen (DE)**

(72) Inventors:
- **VAKALOPOULOS, Alexandros**
  **40721 Hilden (DE)**
- **COLLIN-KRÖPELIN, Marie-Pierre**
  **79576 Weil am Rhein (DE)**
- **ORTEGA HERNANDEZ, Nuria**
  **42281 Wuppertal (DE)**
- **DIESKAU, Andre**
  **42115 Wuppertal (DE)**
- **BOULTADAKIS-ARAPINIS, Melissa**
  **40215 Düsseldorf (DE)**
- **CANDISH, Lisa**
  **42115 Wuppertal (DE)**
- **STELLFELD, Timo**
  **14197 Berlin (DE)**
- **MATHAR, Ilka**
  **40235 Düsseldorf (DE)**
- **HOFMEISTER, Lucas, Hudson**
  **10245 Berlin (DE)**
- **SANDNER, Peter**
  **42113 Wuppertal (DE)**
- **WUNDER, Frank**
  **42117 Wuppertal (DE)**
- **DIETZ, Lisa**
  **42111 Wuppertal (DE)**
- **WEBSTER, Robert, Alan**
  **13467 Berlin (DE)**
- **SCHMECK, Carsten**
  **45472 Mülheim (DE)**
- **MONDRITZKI, Thomas**
  **45130 Essen (DE)**

(74) Representative: **BIP Patents**
**c/o Bayer Intellectual Property GmbH**
**Alfred-Nobel-Straße 50**
**40789 Monheim am Rhein (DE)**

(56) References cited:
**WO-A1-2012/058132 WO-A1-2015/095515**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BUYS EMMANUEL ET AL: "New insights into the role of soluble guanylate cyclase in blood pressure regulation :", CURRENT OPINION IN NEPHROLOGY & HYPERTENSION, vol. 23, no. 2, 1 March 2014 (2014-03-01), GB, pages 135 - 142, XP055883234, ISSN: 1062-4821, Retrieved from the Internet <URL:https://www.ncbi.nlm.nih.gov/pmc/articles/PMC4102174/pdf/nihms-611318.pdf> DOI: 10.1097/01.mnh.0000441048.91041.3a**

## Description

The invention relates to substituted pyrazolo piperidine carboxylic acids, their salts and to processes for their preparation, and also to their use for preparing medicaments for the treatment and/or prophylaxis of diseases, in particular cardiovascular and cardiac diseases, preferably heart failure with reduced and preserved ejection fraction (HFrEF, HFmrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU).

In addition, the same above-mentioned pathophysiological mechanisms are effective when blood transfusions (for example by storage etc. with an elevated concentration of free Hb) are administered to patients having a transfusion indication.

Furthermore, in the future the combination of an sGC activator with a synthetic Hb-based oxygen carrier may mitigate the side effects hitherto observed [Weiskopf, Anaesthesia & Analgesia, 110:3; 659-661, 2010] which are caused by reduced availability of NO, thus allowing clinical application.

One of the most important cellular transmission systems in mammalian cells is cyclic guanosine monophosphate (cGMP). Together with nitric oxide (NO), which is released from the endothelium and transmits hormonal and mechanical signals, it forms the NO/cGMP system. Guanylate cyclases catalyse the biosynthesis of cGMP from guanosine triphosphate (GTP). The representatives of this family disclosed to date can be divided both according to structural features and according to the type of ligands into two groups: the particulate guanylate cyclases which can be stimulated by natriuretic peptides, and the soluble guanylate cyclases which can be stimulated by NO. The soluble guanylate cyclases consist of two subunits and very probably contain one haem per heterodimer, which is part of the regulatory site. The latter is of central importance for the mechanism of activation. NO is able to bind to the iron atom of haem and thus markedly increase the activity of the enzyme. Haem-free preparations cannot, by contrast, be stimulated by NO. Carbon monoxide (CO) is also able to attach to the central iron atom of haem, but the stimulation by CO is distinctly less than that by NO.

**[0005]** Through the production of cGMP and the regulation, resulting therefrom, of phosphodiesterases, ion channels and protein kinases, guanylate cyclase plays a crucial part in various physiological processes, in particular in the relaxation and proliferation of smooth muscle cells, in platelet aggregation and adhesion and in neuronal signal transmission, and in disorders caused by an impairment of the aforementioned processes. Under pathophysiological conditions, the NO/cGMP system may be suppressed, which may lead for example to high blood pressure, platelet activation, increased cellular proliferation and fibrosis, endothelial dysfunction, atherosclerosis, angina pectoris, heart failure, thromboses, stroke and myocardial infarction.

A possible way of treating such disorders which is independent of NO and aims at influencing the cGMP signaling pathway in organisms is a promising approach because of the high efficiency and few side effects which are to be expected.

Compounds, such as organic nitrates, whose effect is based on NO have to date been exclusively used for the therapeutic stimulation of soluble guanylate cyclase. NO is produced by bioconversion and activates soluble guanylate cyclase by attaching to the central iron atom of haem. Besides the side effects, the development of tolerance is one of the crucial disadvantages of this mode of treatment [O.V. Evgenov et al., Nature Rev. Drug Disc. 5 (2006), 755].

Substances which directly stimulate soluble guanylate cyclase, i.e. without previous release of NO, have been identified in recent years. The indazole derivative YC-1 was the first NO-independent but haem-dependent sGC stimulator described [Evgenov et al., ibid.]. Based on YC-1, further substances were discovered which are more potent than YC-1 and show no relevant inhibition of phosphodiesterases (PDE). This led to the identification of the pyrazolopyridine derivatives BAY 41-2272, BAY 41-8543, BAY 63-2521 and BAY 102-1189. Together with the recently published structurally different substances CMF-1571 and A-350619, these compounds form the new class of the sGC stimulators [Evgenov et al., ibid.]. A common characteristic of this substance class is a NO-independent and selective activation of the haem-containing sGC. In addition, the sGC stimulators in combination with NO have a synergistic effect on sGC activation based on a stabilization of the nitrosyl-haem complex. The exact binding site of the sGC stimulators at the sGC is still being debated. If the haem group is removed from the soluble guanylate cyclase, the enzyme still has a detectable catalytic basal activity, i.e. cGMP is still being formed. The remaining catalytic basal activity of the haem-free enzyme cannot be stimulated by any of the stimulators mentioned above [Evgenov et al., ibid.].

In addition, NO- and haem-independent sGC activators, with BAY 5 8-2667 as prototype of this class, have been identified. Common characteristics of these substances are that in combination with NO they only have an additive effect on enzyme activation, and that the activation of the oxidized or haem-free enzyme is markedly higher than that of the haem-containing enzyme [Evgenov et al., ibid.; J.P. Stasch et al., Br. J. Pharmacol. 136 (2002), 773; J.P. Stasch et al., J. Clin. Invest. 116 (2006), 2552]. Spectroscopic studies show that BAY 58-2667 displaces the oxidized haem group which, as a result of the weakening of the iron-histidine bond, is attached only weakly to the sGC. It has also been

shown that the characteristic sGC haem binding motif Tyr-x-Ser-x-Arg is absolutely essential both for the interaction of the negatively charged propionic acids of the haem group and for the action of BAY 58-2667. Against this background, it is assumed that the binding site of BAY 58-2667 at the sGC is identical to the binding site of the haem group [J.P. Stasch et al., J. Clin. Invest. 116 (2006), 2552].

The sGC activator Runcaciguat (Hahn et al., Drugs Future 43 (2018), 738, WO 2012/139888) is in clinical development by BAYER (https://www.clinicaltrials.gov/NCT04507061). our understanding of the redox equilibrium of the sGC in health and diseases is limited. Therefore, the treatment potential of sGC activators is not fully clear yet. However, since oxidative stress could render the sGC enzyme heme-free the sGC activators, sGC activators might have an even broader treatment potential which still needs to be identified and proved in the future.

The compounds described in the present invention are now likewise capable of activating the haem-free form of soluble guanylate cyclase. This is also confirmed by the fact that these novel activators firstly have no synergistic action with NO at the haem-containing enzyme and that secondly their action cannot be blocked by the haem-dependent inhibitor of soluble guanylate cyclase, 1H-1,2,4-oxadiazolo[4,3-a]-quinoxalin-1-one (ODQ), but is even potentiated by this inhibitor [cf. O.V. Evgenov et al., Nature Rev. Drug Disc. 5 (2006), 755; J.P. Stasch et al., J. Clin. Invest. 116 (2006), 2552].

In WO 2012/058132 substituted pyrazolo pyridine carboxylic acids are diclosed as sGC activators. In contrast to the compounds according to the present invention these compounds do have a heteroaromatic pyridine moiety linking the pyrazole carboxylic acid to the rest of the molecule. Furthermore the pyridine nitrogen has another position than the piperidine nitrogen of the compounds according to the present invention. However these compounds do only show mediocre pharmacokinetic properties, like e.g. moderate clearance (CL) and intermediate half-life and mean residence time (MRT) after intraveneous (iv) administration in preclinical pharmacokinetic models.

It is therefore an object of the present invention to provide novel sGC activator compounds for the treatment and / or prophylaxis of diseases, in particular cardiovascular and cardiac diseases, preferably heart failure with reduced and preserved ejection fraction (HFrEF, HFmrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU), in humans and animals, which compounds show a good pharmacokinetic behavior with a good pharmacological activity profile as well as beneficial physico chemical properties (e.g. solubility).

Surprisingly, it has now been found that certain substituted pyrazolo piperidine carboxylic acids as well as their corresponding salts represent highly potent sGC activators with good pharmacokinetic behavior with a good pharmacological activity profile as well as beneficial physico chemical properties (e.g. solubility).

The invention provides compounds of the formula (I)

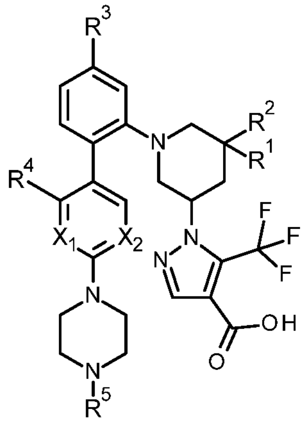 (I),

in which

$R^1$ represents hydrogen or halogen,

$R^2$ represents hydrogen or halogen,

$R^3$ represents chloro or trifluoromethyl,

$R^4$ represents hydrogen or $C_1$-$C_4$-alkyl

$R^5$ represents $C_1$-$C_6$-alkyl

$X_1$ represents nitrogen or carbon

$X_2$ represents nitrogen or carbon

and the salts thereof, the solvates thereof and the solvates of the salts thereof.

The term "substituted" means that one or more hydrogen atoms on the designated atom or group are replaced with a selection from the indicated group, provided that the designated atom's normal valence under the existing circumstances is not exceeded. Combinations of substituents and/or variables are permissible.

As used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".

In the context of the present invention, unless specified otherwise, the substituents are defined as follows:
The term "halogen" or "halogeno" like in combinations e.g. in halogenoalkyl means a fluorine, chlorine, bromine or iodine atom, particularly a fluorine, chlorine or bromine atom, even more particularly fluorine or chlorine.

The term "$C_1$-$C_4$-alkyl", "$C_1$-$C_5$-alkyl" and "$C_1$-$C_6$-alkyl" means a linear or branched, saturated, monovalent hydrocarbon group having 1, 2, 3, or 4 carbon atoms, 1, 2, 3, 4 or 5 carbon atoms, and 1, 2, 3, 4, 5 or 6 carbon atoms, *e.g.* a methyl, ethyl, propyl, isopropyl, *n*-butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("$C_1$-$C_4$-alkyl"), *e.g.* a methyl, ethyl, propyl, isopropyl, butyl, sec-butyl isobutyl, or *tert*-butyl group, more particularly 1, 2 or 3 carbon atoms ("$C_1$-$C_3$-alkyl"), e.g. a methyl, ethyl, n-propyl or isopropyl group.

The term "$C_1$-$C_6$-halogenoalkyl", "$C_2$-$C_6$-halogenoalkyl", "$C_1$-$C_4$-halogenoalkyl", "$C_2$-$C_4$-halogenoalkyl", "Ci-Cs-halogenoalkyl" and "$C_1$-$C_2$-halogenoalkyl" represents a linear or branched, saturated, monovalent hydrocarbon group in which the term "alkyl" is as defined *supra,* and in which one or more of the hydrogen atoms are replaced, identically or differently, with a halogen atom. Particularly, said halogen atom is a fluorine atom. Said $C_1$-$C_6$-halogenoalkyl group is, for example fluoromethyl, difluoromethyl, trifluoromethyl, 2-fluoroethyl, 2,2-difluoroethyl, 2,2,2-trifluoroethyl, pentafluoroethyl, 3,3,3-tri-fluoropropan-1-yl, 1,1,1-trifluoropropan-2-yl, 1,3-difluoropropan-2-yl, 3-fluoropropan-1-yl, 1,1,1-trifluo-robutan-2-yl, and 3,3,3-trifluoro-1-methyl-propan-1-yl.

The term "$C_1$-$C_4$-halogenoalkoxy" and "$C_1$-$C_3$-halogenoalkoxy" represents a linear or branched, saturated, monovalent $C_1$-$C_4$-alkoxy or $C_1$-$C_3$-alkoxy group (where alkoxy represents a straight-chain or branched, saturated, monovalent alkoxy radical having 1 to 4 or 1 to 3 carbon atoms, by way of example and with preference methoxy, ethoxy, n-propoxy, isopropoxy), in which one or more of the hydrogen atoms is replaced, identically or differently, with a halogen atom. Particularly, said halogen atom is a fluorine atom. Said $C_1$-$C_3$-halogenoalkoxy group is, for example, fluoromethoxy, difluoromethoxy, trifluoromethoxy, 2,2,2-trifluoroethoxy or pentafluoroethoxy.

The term "$C_3$-$C_6$-cycloalkyl" means a saturated, monovalent, monocyclic hydrocarbon ring which contains 3, 4, 5 or 6 carbon atoms. Said $C_3$-$C_6$-cycloalkyl group is for example a cyclopropyl, cyclobutyl, cyclopentyl or cyclohexyl group.

Compounds according to the invention are the compounds of the formula (I) and the salts, solvates and solvates of the salts thereof, and also the compounds encompassed by formula (I) and specified hereinafter as working example(s), and the salts, solvates and solvates of the salts thereof, to the extent that the compounds encompassed by formula (I) and specified hereinafter are not already salts, solvates and solvates of the salts.

The inventive compounds may, depending on their structure, exist in different stereoisomeric forms, i.e. in the form of configurational isomers or else, if appropriate, of conformational isomers (enantiomers and/or diastereomers, including those in the case of rotamers and atropisomers). The present invention therefore encompasses the enantiomers and diastereomers, and the respective mixtures thereof. The stereoisomerically uniform constituents can be isolated from such mixtures of enantiomers and/or diastereomers in a known manner; chromatography processes are preferably used for this, especially HPLC chromatography on an achiral or chiral phase.

The present invention includes all possible tautomers of the compounds of the present invention as single tautomers, or as any mixture of said tautomers, in any ratio.

In the context of the present invention, the term "enantiomerically pure" is understood to mean that the compound in question with respect to the absolute configuration of the chiral centre is present in an enantiomeric excess of more than 95%, preferably more than 97%. The enantiomeric excess (ee value) is calculated in this case by evaluation of the corresponding HPLC chromatogram on a chiral phase with the aid of the formula below:

$$ee = [E^A (area\%) - E^B (area\%)] \times 100\% / [E^A (area\%) + E^B (area\%)]$$

($E^A$: enantiomer in excess, $E^B$: enantiomer in deficiency)

The present invention also encompasses all suitable isotopic variants of the compounds according to the invention. An isotopic variant of an inventive compound is understood here as meaning a compound in which at least one atom within the inventive compound has been exchanged for another atom of the same atomic number, but with a different atomic mass than the atomic mass which usually or predominantly occurs in nature. Examples of isotopes which can be incorporated into a compound according to the invention are those of hydrogen, carbon, nitrogen, oxygen, phosphorus, sulfur, fluorine, chlorine, bromine and iodine, such as $^{2}H$ (deuterium), $^{3}H$ (tritium), $^{13}C$, $^{14}C$, $^{15}N$, $^{17}0$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$ $^{124}I$, $^{129}I$ and $^{131}I$. Particular isotopic variants of a compound according to the invention, especially those in which one or more radioactive isotopes have been incorporated, may be beneficial, for example, for the examination of the mechanism of action or of the active ingredient distribution in the body; due to comparatively easy preparability and detectability, especially compounds labelled with $^{3}H$ or $^{14}C$ isotopes are suitable for this purpose. In addition, the incorporation of isotopes, for example of deuterium, may lead to particular therapeutic benefits as a consequence of greater metabolic stability of the compound, for example an extension of the half-life in the body or a reduction in the active dose required; such modifications of the inventive compounds may therefore in some cases also constitute a preferred embodiment of the present invention. Isotopic variants of the compounds according to the invention can be prepared by the processes known to those skilled in the art, for example by the methods described further below and the procedures described in the working examples, by using corresponding isotopic modifications of the respective reagents and/or starting compounds.

Preferred salts in the context of the present invention are physiologically acceptable salts of the compounds according to the invention. However, the invention also encompasses salts which themselves are unsuitable for pharmaceutical applications but which can be used, for example, for the isolation or purification of the compounds according to the invention.

Physiologically acceptable salts of the compounds according to the invention include acid addition salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalenedisulfonic acid, acetic acid, trifluoroacetic acid, propionic acid, lactic acid, tartaric acid, malic acid, citric acid, fumaric acid, maleic acid and benzoic acid.

Physiologically acceptable salts of the compounds according to the invention also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, ethyldiisopropylamine, monoethanolamine, diethanolamine, triethanolamine, dicyclohexylamine, dimethylaminoethanol, procaine, dibenzylamine, *N*-methylmorpholine, arginine, lysine, ethylenediamine, N-methylpiperidine and choline.

The present invention includes all possible salts of the compounds according to the invention as single salts, or as any mixture of said salts, in any ratio.

Solvates in the context of the invention are described as those forms of the inventive compounds which form a complex in the solid or liquid state by coordination with solvent molecules. The compounds according to the invention may contain polar solvents, in particular water, methanol or ethanol for example, as structural element of the crystal lattice of the compounds. Hydrates are a specific form of the solvates in which the coordination is with water. It is possible for the amount of polar solvents, in particular water, to exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present invention includes all such hydrates or solvates.

Further, the compounds according to the invention can exist as N-oxides, which are defined in that at least one nitrogen of the compounds of the present invention is oxidised in a known manner. The present invention includes all such possible N-oxides.

The present invention additionally also encompasses prodrugs of the inventive compounds. The term "prodrugs" encompasses compounds which for their part may be biologically active or inactive but are converted during their residence time in the body into compounds according to the invention (for example by metabolism or hydrolysis).

Preference is given to compounds of the formula (I) in which

$R^1$ represents hydrogen, fluorine

$R^2$ represents hydrogen, fluorine

$R^3$ represents chloro or trifluoromethyl

$R^4$ represents hydrogen or methyl

$R^5$ represents $C_1$-$C_5$-alkyl

$X_1$ represents nitrogen or carbon

$X_2$ represents nitrogen or carbon

and the salts thereof, the solvates thereof and the solvates of the salts thereof.

Preference is also given to compounds of the formula (I) in which

$R^1$ represents hydrogen, fluorine

$R^2$ represents hydrogen, fluorine

$R^3$ represents chloro or trifluoromethyl

$R^4$ represents hydrogen or methyl

$R^5$ represents methyl, ethyl, n-propyl, i-propyl, 2,2,-dimethyl-propyl, isobutyl

$X_1$ represents nitrogen or carbon

$X_2$ represents nitrogen or carbon

and the salts thereof, the solvates thereof and the solvates of the salts thereof.

Preference is also given to compounds of the formula (I) in which

$R^1$ represents hydrogen

$R^2$ represents hydrogen

$R^3$ represents chloro or trifluoromethyl

$R^4$ represents hydrogen or methyl

$R^5$ represents methyl, ethyl, n-propyl, i-propyl, 2,2,-dimethyl-propyl, isobutyl

$X_1$ represents carbon or C-F

$X_2$ represents carbon

and the salts thereof, the solvates thereof and the solvates of the salts thereof.

Preference is also given to compounds of the formula (I) in which

$R^1$ represents hydrogen

$R^2$ represents hydrogen

$R^3$ represents chloro or trifluoromethyl

$R^4$ represents hydrogen

$R^5$ represents methyl, ethyl, n-propyl, i-propyl, 2,2,-dimethyl-propyl, isobutyl

$X_1$ represents carbon

$X_2$ represents carbon

and the salts thereof, the solvates thereof and the solvates of the salts thereof.

Preference is also given to compounds of the formula (I) in which

$R^1$ represents hydrogen

$R^2$ represents hydrogen

$R^3$ represents chloro

$R^4$ represents hydrogen

$R^5$ represents isobutyl

$X_1$ represents carbon

$X_2$ represents carbon

and the salts thereof, the solvates thereof and the solvates of the salts thereof.

Preference is also given to compound of the formula

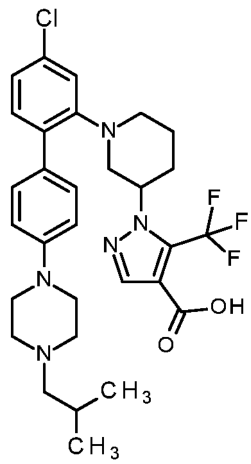

and the salts thereof, solvates thereof or solvates of the salts thereof.

Especially preference is given to compound of formula

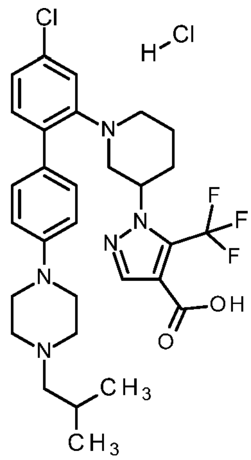

The invention further provides a process for preparing compounds of the formula (I), or salts thereof, solvates thereof or solvates of the salts thereof, wherein

in a first step [B] the compounds of the formula (IV)

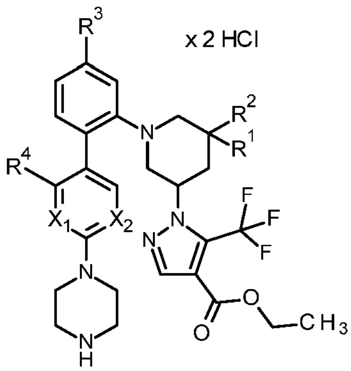

(IV),

in which $R^1$, $R^2$, $R^3$, $R^4$ and $X_1$ and $X_2$ are defined as above, are reacted with compounds of the formula (III)

$R^{5a}$-CHO (III),

in which $R^{5a}$ represents $C_1$-$C_3$-alkyl, preferably isopropyl in the presence of a reducing agent, a suitable solvent and a base to provide compounds of the formula (II)

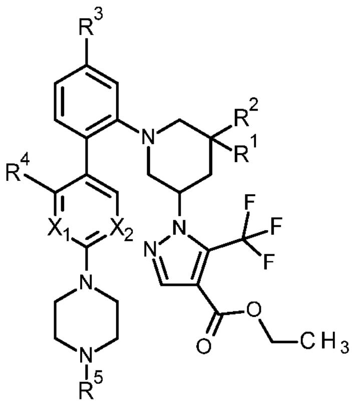

(II)

in which $R^1$, $R^2$, $R^3$, $R^4$, $R^5$ and $X_1$ and $X_2$ are defined as above
and
in a second step [A]
compounds of formula (II) are reacted with a base to provide compounds of the formula (I),

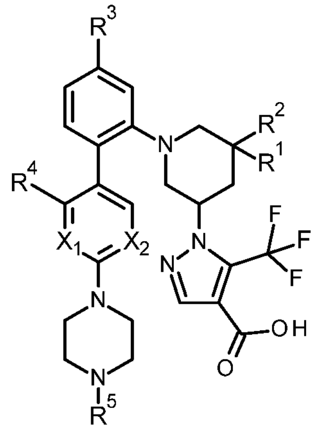

(I)

in which $R^1$, $R^2$, $R^3$, $R^4$ , $R^5$ and $X_1$ and $X_2$ are defined as above
optionally compounds of formula (I) are transferred in a third step [A] *
into the corresponding salts of formula (Ia)

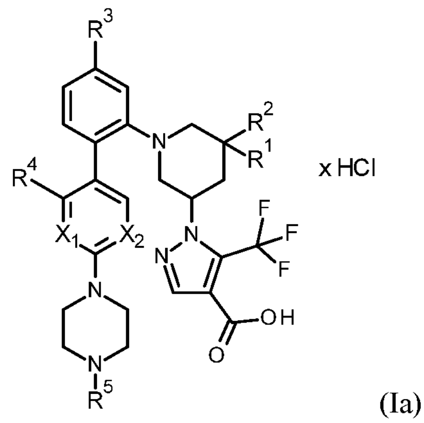

(Ia)

in the presence of a suitable acid in a suitable solvent
in which $R^1$, $R^2$, $R^3$, $R^4$ , $R^5$ and $X_1$ and $X_2$ are defined as above.

Reaction [A]* (salt formation)

The reaction [A]* is generally carried out in inert solvents in the presence of an acid preferably in a temperature range from 0°C to 60°C at atmospheric pressure.

Suitable acids for the salt formation are generally sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride, hydrogen bromide, toluenesulfonic acid, methanesulfonic acid or sulfuric acid.

Suitable inert solvents for the salt formation are, for example, ethers such as diethyl ether, dioxane, tetrahydrofuran, glycol dimethyl ether or diethylene glycol dimethyl ether, or other solvents such as acetone, ethyl acetate, ethanol, n-propanol, isopropanol, acetonitrile, dimethyl sulphoxide, N,N-dimethylformamide, N,N-dimethylacetamide, N,N'-dimethylpropyleneurea (DMPU) or N-methylpyrrolidone (NMP). It is also possible to use mixtures of the solvents mentioned. Preference is given to diethyl ether, dioxane, tetrahydrofuran or mixtures of these solvents.

Reaction [A] (ester hydrolyses)

The hydrolysis of the ester group in compounds of formula II is carried out by customary methods, by treating the esters in inert solvents with acids or bases, where in the latter variant the salts initially formed are converted into the free carboxylic acids by treatment with acid. In the case of the tert-butyl esters, the ester hydrolysis is preferably effected with acids.

Suitable inert solvents for these reactions are water or the organic solvents customary for ester cleavage.

These preferably include alcohols such as methanol, ethanol, *n*-propanol, isopropanol, n-butanol or *tert*-butanol, ethers such as diethyl ether, tetrahydrofuran, 1,4-dioxane or 1,2-dimethoxyethane, or other solvents such as dichloromethane, acetone, methyl ethyl ketone, *N*,*N* dimethylformamide or dimethyl sulphoxide. It is equally possible to use mixtures of these solvents. In the case of a basic ester hydrolysis, preference is given to using mixtures of water with dioxane, tetrahydrofuran, methanol, ethanol and/or dimethylformamide or mixtures of tetrahydrofuran and methanol or ethanol. In the case of the reaction with trifluoroacetic acid, preference is given to using dichloromethane, and in the case of the reaction with hydrogen chloride preference is given to using tetrahydrofuran, diethyl ether, dioxane or water.

Suitable bases are the customary inorganic bases. These especially include alkali metal or alkaline earth metal hydroxides, for example lithium hydroxide, sodium hydroxide, potassium hydroxide or barium hydroxide, or alkali metal or alkaline earth metal carbonates, such as sodium carbonate, potassium carbonate or calcium carbonate. Preference is given to lithium hydroxide, sodium hydroxide or potassium hydroxide.

Suitable acids for the ester hydrolysis are generally sulfuric acid, hydrogen chloride/

hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid in the case of the tert-butyl esters and to hydrochloric acid in the case of the methyl esters.

The ester hydrolysis is generally carried out within a temperature range from -20°C to +120°C, preferably at 0°C to +80°C.

The compounds of the formula (II)

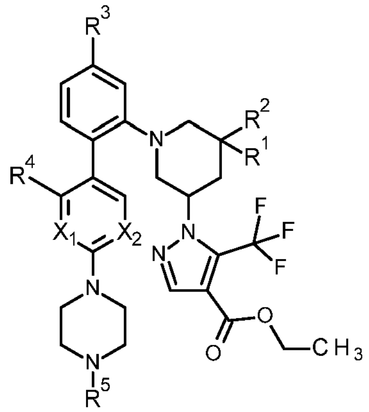

(II)

in which $R^1$, $R^2$, $R^3$, $R^4$, $R^5$ and $X_1$ and $X_2$ are defined as above are novel.

The compounds of the formula (II) can be synthesized from the corresponding starting compounds of formula (IV) by

[B] reacting the compounds of the formula (IV)

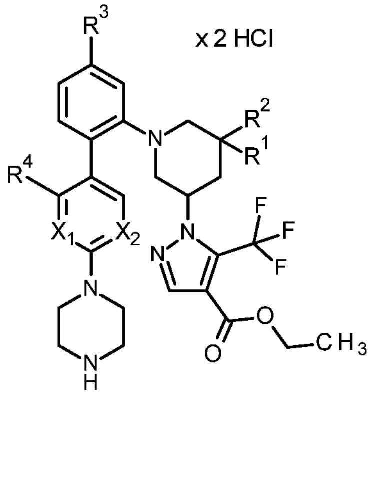

(IV),

in which $R^1$, $R^2$, $R^3$, $R^4$ and $X_1$ and $X_2$ are defined as above,

with compounds of the formula (III)

$R^{5a}$-CHO (III),

in which $R^{5a}$ represents $C_1$-$C_3$-alkyl, preferably isopropyl,

in the presence of a reducing agent, a suitable base and a suitable solvent to provide compounds of the formula (II)

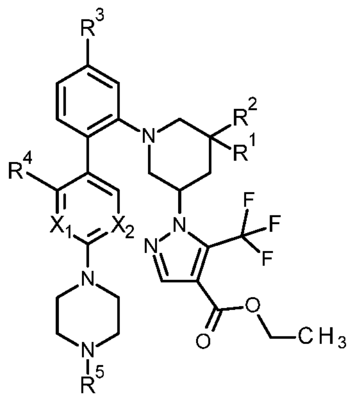

(II)

in which $R^1$, $R^2$ , $R^3$, $R^4$, $R^5$ and $X_1$ and $X_2$ are defined as above.

Reaction [B] (reductive amination)

The reaction of step [B] is generally carried out in inert solvents in the presence of a reducing agent, if appropriate in the presence of a base and or a dehydrating agent, preferably in a temperature range from 0°C to 60°C at atmospheric pressure.

Suitable reducing agents for reductive aminations are alkali metal borohydrides customary for such purposes such as sodium borohydride, sodium cyanoborohydride or sodium triacetoxyborohydride; preference is given to using sodium triacetoxyborohydride.

The addition of an acid, such as acetic acid in particular, and/or of a dehydrating agent, for example molecular sieve or trimethyl orthoformate or triethyl orthoformate, may be advantageous in these reactions.

Bases are, for example organic bases such as trialkylamines, for example triethylamine, N-methylmorpholine, N-methylpiperidine, 4-dimethylaminopyridine or diisopropylethylamin, or pyridine. Bases, such as N,N-diisopropylethylamine and triethylamine in particular, may be advantageous in these reactions.

Suitable solvents for these reactions are especially alcohols such as methanol, ethanol, n-propanol or isopropanol, ethers such as diisopropyl ether, methyl tert-butyl ether, tetrahydrofuran, 1,4-dioxane or 1,2-dimethoxyethane, polar aprotic solvents such as acetonitrile or *N*,*N* dimethylformamide (DMF) or mixtures of such solvents; preference is given to using tetrahydrofuran.

The reactions are generally conducted within a temperature range of 0°C to +60°C.

The aldehydes of formula (III) are commercial available, known or can be synthesized from known starting materials by known processes.

Compounds of the formula (IV)

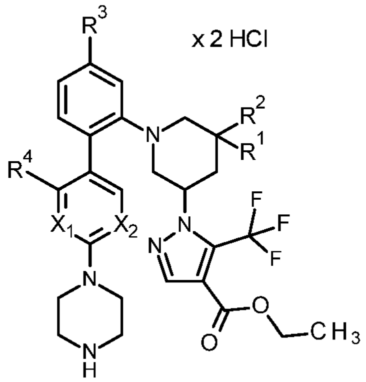

(IV),

in which $R^1$, $R^2$, $R^3$, $R^4$ and $X_1$ and $X_2$ are defined as above are novel.

The compounds of the formula (IV) can be synthesized from the corresponding compounds of formula (V) by

[C] reacting the compounds of the formula (V)

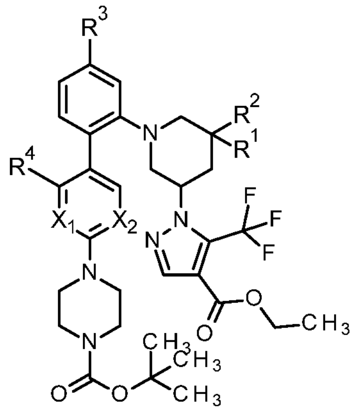

(V),

in which $R^1$, $R^2$, $R^3$, $R^4$ and $X_1$ and $X_2$ are defined as above,

in the presence of a suitable acid and a suitable solvent.

Reaction [C] (Deprotection)

The reaction [C] is generally carried out in inert solvents in the presence of a suitable acid in the presence of an inert solvent, preferably in a temperature range from 0°C to 60°C at atmospheric pressure.

Acids are, for example organic or inorganic acids such as sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid.

Suitable solvents for these reactions are especially alcohols such as methanol, ethanol, n-propanol or isopropanol, ethers such as diisopropyl ether, methyl tert-butyl ether, tetrahydrofuran, 1,4-dioxane or 1,2-dimethoxyethane, polar aprotic solvents such as acetonitrile or *N*,*N* dimethylformamide (DMF) or mixtures of such solvents; preference is given to using tetrahydrofuran.

The reactions are generally conducted within a temperature range of 0°C to +60°C.

Compounds of formula (V)

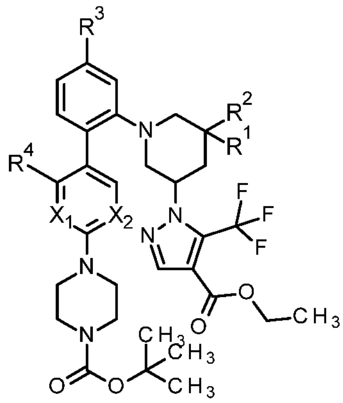

(V),

in which $R^1$, $R^2$, $R^3$, $R^4$ and $X_1$ and $X_2$ are defined as above are novel.

The compounds of the formula (V) can be synthesized from the corresponding compounds of formula (VIII) by

[D] reacting the compounds of the formula (VIII)

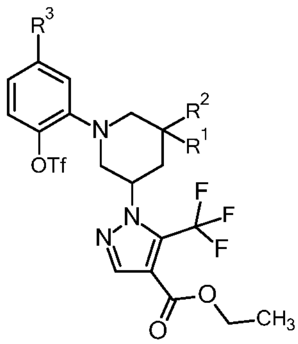

(VIII),

in which $R^1$, $R^2$ and $R^3$ are defined as above,

in the presence of a suitable palladium catalyst, base and a suitable solvent

with compounds of the formula (VI)

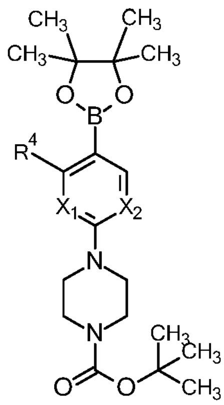

(VI).

Reaction [D] (Suzuki coupling)

The reaction [D] is generally carried out in the presence of a suitable palladium catalyst and a suitable base in inert solvents, preferably at temperature range from room temperature up to reflux of the solvents at atmospheric pressure.

Inert solvents for reaction [D] are for example alcohols like methanol, ethanol, n-propanol, isopropanol, n-butanol or tert.-butanol, ether like diethylether, dioxane, tetrahydrofuran, glycoldimethylether or di-ethylenglycoldimethylether, hydrocarbons like benzene, xylol, toluene, hexane, cyclohexane or petroleum oil, or other solvents like dimethylformamide (DMF), dimethylsulfoxide (DMSO), N,N'-dimethylpropylene urea (DMPU), N-methylpyrrolidone (NMP), pyridine, acetonitrile or also water. It is also possible to utilize mixtures of the aforementioned solvents. Preferred is a mixture of dimethylformamide / water and toluene / ethanol.

Suitable bases for reaction steps are the customary inorganic bases. These especially include alkali metal or alkaline earth metal hydroxides, for example lithium hydroxide, sodium hydroxide, potassium hydroxide or barium hydroxide alkali metal hydrogencarbonates like sodium or potassium - hydrogencarbonate, or alkali metal or alkaline earth metal carbonates such as lithium, sodium, potassium, calcium or cesium carbonate, or alkali hydrogenphosphates like disodium or dipotassium hydrogenphosphate. Preferably used bases are sodium or potassium carbonate.

Examples of suitable palladium catalysts for reaction steps ["Suzuki-coupling"] are e.g. palladium on charcoal, palladium(II)-acetate, tetrakis-(triphenylphosphine)-palladium(0), bis-(triphenylphosphine)-palladium(II)-chloride, bis-(acetonitrile)-palladium(II)-chloride and [1,1'-bis(diphenylphosphino)ferro-cene]dichloropalladium(II)-dichlormethane-complex [cf. e.g. Hassan J. et al., Chem. Rev. 102, 1359-1469 (2002)].

The reaction steps are generally carried out within a temperature range from +20°C to +150°C, preferably at +50°C to +100°C.

The compounds of the formula (VI) are novel, commercial available or available via known processes.

The compounds of the formula (VIII)

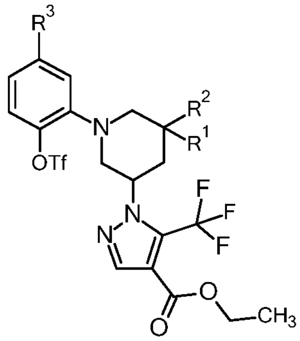

(VIII),

in which $R^1$, $R^2$ and $R^3$ are defined as above are novel.

The compounds of the formula (VIII) can be prepared

[E] by reacting compounds of the formula (IX)

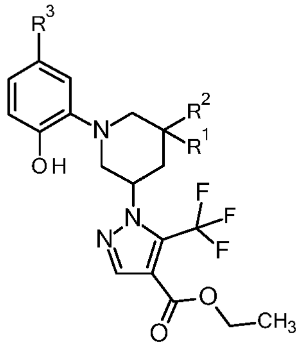

(IX),

in which $R^1$, $R^2$ and $R^3$ are as defined above,

with triflic acid anhydride in the presence of base and an inert solvent.

Reaction [E] (triflatization)

The reaction [E] is generally carried out in inert solvents, preferably in a temperature range from room temperature up to reflux of the solvents at atmospheric pressure.

Bases are, for example, organic bases like alkali amines or pyridines or inorganic bases such as sodium hydroxide, lithium hydroxide or potassium hydroxide, or alkali metal carbonates such as caesium carbonate, sodium carbonate or potassium carbonate, or alkoxides such as potassium tert-butoxide or sodium *tert*-butoxide, or pyridines such as pyridine or 2,6-lutidine, or alkali amines such as triethylamine or N,N-diisopropylethylamine; preference is given to triethylamine.

Inert solvents are, for example, ethers such as diethyl ether, methyl *tert*-butyl ether, 1,2-dimethoxyethane, dioxane or tetrahydrofuran, or other solvents such as dichloromethane, dimethylformamide, dimethylacetamide, acetonitrile or pyridine, or mixtures of solvents; preference is given to dichloromethane.

The compounds of the formula (IX)

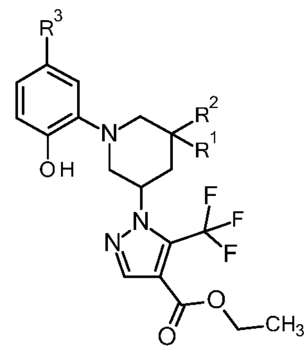

(IX),

in which $R^1$, $R^2$ and $R^3$ are defined as above are novel.

The compounds of the formula (IX) can be prepared

[F] by reacting compounds of the formula (X)

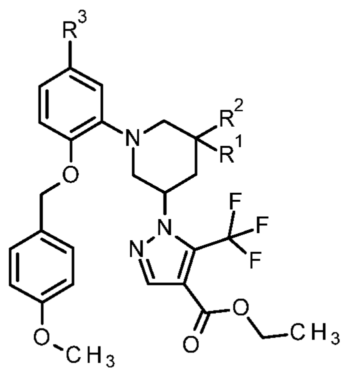

(X),

in which $R^1$, $R^2$ and $R^3$ are as defined above,

with an acid optionally in an inert solvent.

Reaction [F] (acidic deprotection)

The reaction [F] is generally carried out in inert solvents or without solvents, preferably in a temperature range from 0°C up to reflux of the solvents at atmospheric pressure.

Inert solvents are, for example, halogenated hydrocarbons such as dichloromethane, trichloromethane, carbon

tetrachloride or 1,2-dichloroethane, alcohols such as methanol or ethanol, ethers such as diethyl ether, methyl tert-butyl ether, 1,2-dimethoxyethane, dioxane or tetrahydrofuran, or other solvents such as dimethylformamide, dimethoxy ethane, N-methyl-pyrrolidone, dimethylacetamide, acetonitrile, acetone or pyridine, or mixtures of solvents; preference is given to dichloromethane or dioxane.

Suitable acids for the acidic deprotection are generally sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid.

Compounds of the formula (X)

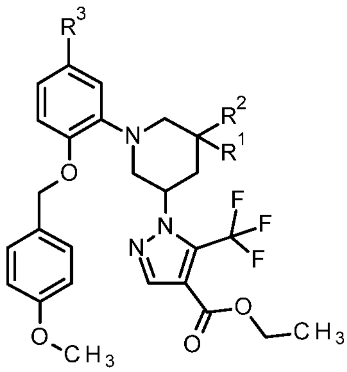

(X),

in which $R^1$, $R^2$ and $R^3$ are as defined above are novel.

The compounds of the formula (X) can be prepared

[G] by reacting compounds of the formula (XII)

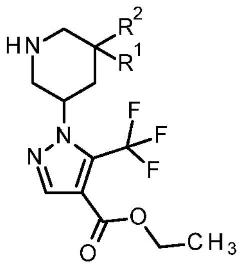

(XII),

in which $R^1$ and $R^2$ are as defined above,

with compounds of the formula (XI)

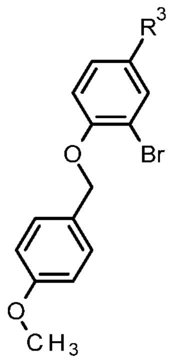

(XI),

in which

$R^3$ is as defined above,

in the presence of a palladium source, a suitable ligand and a base.

Reaction [G] (Buchwald Hartwig coupling)

The reaction [G] is generally carried out in the presence of a palladium source, a suitable ligand and a base in inert solvents, preferably in a temperature range from room temperature up to reflux of the solvents at atmospheric pressure.

The palladium source and a suitable ligand are, for example, palladium on charcoal, palladium(II)-acetate, tris(dibenzylideneacetone)palladium(0), tetrakis-(triphenylphosphine)-palladium(0), bis-(triphenylphosphine)-palladium(II) chloride, bis-(acetonitrile)-palladium(II) chloride, [1,1'-bis(diphenylphosphino)ferrocene]dichloro palladium (II) and corresponding dichloromethan-complex, optionally in conjunction with additional phosphane ligands like for example 2,2'-Bis(diphenylphosphino)-1,1'-binaphthyl (BINAP), (2-dicyclohexylphosphino-2',4',6'-triisopropyl-1,1'-biphenyl)[2-(2'-amino-1,1'-biphenyl)]palladium(II) methanesulfonate (XPhos-Pd-G3, CAS-No: 1445085-55-1), (2-biphenyl)di-tert.-butylphosphine, dicyclohexyl[2',4',6'-tris(1-methylethyl)biphenyl-2-yl]phosphane (XPhos, CAS-No: CAS-No: 564483-18-7), Bis(2-phenylphosphinophenyl)ether (DPEphos), or 4,5-bis(diphenylphosphino)-9,9-dimethylxanthene (Xantphos: CAS-No: 161265-03-8) [cf. e.g. Hassan J. et al., Chem. Rev. 2002, 102, 1359-1469], 2-(dicyclohexylphosphine)-3,6-dimethoxy-2',4',6'-triisopropyl-1,1'-biphenyl (BrettPhos, CAS-No: 1070663-78-3), 2-dicyclohexylphosphino-2',6'-dimethoxybiphenyl (SPhos, CAS-No: 657408-07-6), 2-dicyclohexylphosphino-2',6'-diisopropoxybiphenyl (RuPhos, CAS-No: 787618-22-8), 2-(di-tert-butylphosphino)-3-methoxy-6-methyl-2',4',6'-tri-i-propyl-1,1'-biphenyl (RockPhos) and 2-di-tert-butylphosphino-2',4',6'-triisopropylbiphenyl (tert-ButylXPhos). It is also possible to use corresponding precatalysts such as chloro-[2-(dicyclohexylphosphine)-3,6-dimethoxy-2',4',6'-triisopropyl-1,1'-biphenyl] [2-(2-aminoethyl)-phenyl]palladium(II) (BrettPhos precatalysts) [cf. e.g. S. L. Buchwald et al., Chem. Sci. 2013, 4, 916] optionally be used in conjunction with additional phosphine ligands such as 2-(dicyclohexylphosphine)-3,6-dimethoxy-2',4',6'-triisopropyl-1,1'-biphenyl (BrettPhos).

Preference is given to 2,2'-Bis(diphenylphosphino)-1,1'-binaphthyl (BINAP), tris(dibenzylideneacetone)palladium(0), or in combination with 4,5-bis(diphenylphosphino)-9,9-dimethylxanthen (Xantphos) or dicyclohexyl[2',4',6'-tris(1-methylethyl)biphenyl-2-yl]phosphane (XPhos).

Bases are, for example, suitable inorganic or organic bases like e.g. alkali or earth alkali metal carbonates such as lithium, sodium , potassium, calcium or cesium carbonate, or sodium bicarbonate or potassium bicarbonate, alkali metal hydrogencarbonates such as sodium hydrogencarbonate or potassium hydrogencarbonate, alkali metal or earth alkali hydroxides such as sodium, barium or potassium hydroxide; alkali metal or earth alkali phosphates like potassium phosphate; alkali metal alcoholates like sodium or potassium tert.-butylate and sodium methanolate, alkali metal phenolates like sodium phenolate, potassium acetate, amides like sodium amide, lithium-, sodium- or potassium -bis(trimethylsilyl)amide or lithium diisopropylamide or organic amines like 1,5-diazabicyclo[4.3.0]non-5-ene (DBN), 1,8-diazabicyclo[5.4.0]undec-7-en (DBU). Preference is given to caesium carbonate, sodium carbonate, potassium carbonate or sodium hydrogencarbonate .

Inert solvents are, for example, ethers such as dioxane, diethyl ether, tetrahydrofuran, 2-methyltetrahydrofuran, di-n-butylether, cyclopentylmethylether, glycoldimethylether or diethyleneglycol-dimethylether, alcohols like tert.-butanol or amylalcohols or dimethylformamide, dimethylacetamide, dimethyl sulphoxide, N-methylpyrrolidone, toluene or acetonitrile, or mixtures of the solvents; preference is given to *tert.*-butanol, 1,4-dioxane and toluene.

The compounds of the formula (XI) are known or can be synthesized from the corresponding, commercial

available starting compounds by known processes.

The compounds of the formula (XII) are novel

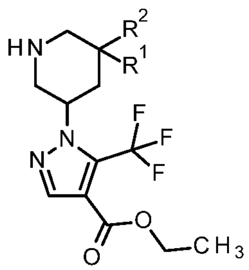

(XII),

in which $R^1$ and $R^2$ are as defined above.

The compounds of the formula (XII) can be prepared

[H] by reacting compounds of the formula (XIII)

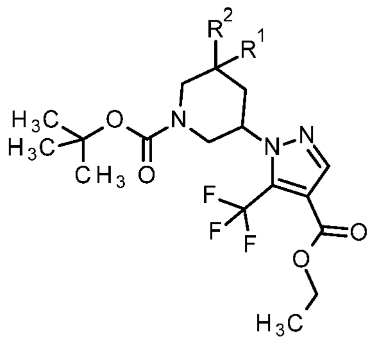

(XIII),

in which $R^1$ and $R^2$ are as defined above,
with an acid in an inert solvent.

Reaction [H] (debocylation)

The reaction [H] is generally carried out in inert solvents in the presence of a suitable acid, preferably in a temperature range from 0°C to 60°C at atmospheric pressure.

Acids are for example organic or inorganic acids such as sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid

Inert solvents are alcohols such as methanol, ethanol or isopropanol, ethers such as diethyl ether, diisopropyl ether, methyl tert-butyl ether, tetrahydrofuran or 1,4-dioxane, dichloromethane, polar aprotic solvents such as acetonitrile or *N,N*-dimethylformamide (DMF) or mixtures of such solvents; preference is given to using 1,4-dioxane.

The compounds of the formula (XIII)

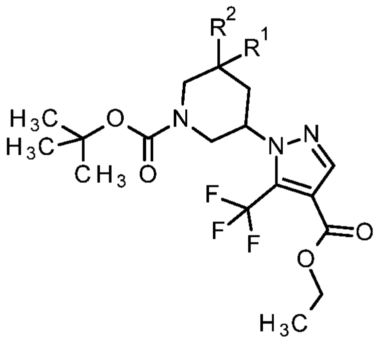

(XIII)

in which

$R^1$ and $R^2$ are as defined above,

are novel.

The compounds of the formula (XIII) can be prepared

[I] by reacting compounds of the formula (XV)

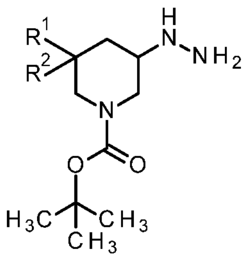

(XV),

in which

$R^1$ and $R^2$ are as defined above,

with compounds of the formula (XIV)

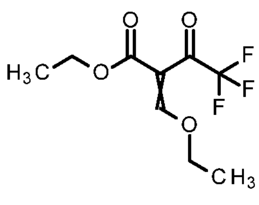

(XIV)

in a solvent.

Reaction [I] (pyrazole formation)

The reaction [I] is generally carried out in a solvent at temperatures from room temperature to reflux.
Suitable solvents are alcohols such as methanol, ethanol or isopropanol, ethers such as diethyl ether, diisopropyl ether, methyl tert-butyl ether, tetrahydrofuran or 1,4-dioxane, dichloromethane, polar aprotic solvents such as acetonitrile or *N,N*-dimethylformamide (DMF) or mixtures of such solvents; preference is given to using ethanol.
The compound of the formula (XIV) are known, commercial available or can be synthesized from the corresponding starting compounds by known processes.

The compounds of the formula (XV)

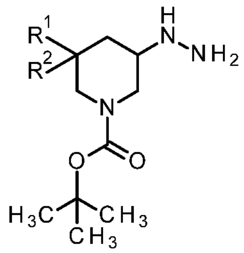

(XV),

in which $R^1$ and $R^2$ are as defined above are novel.

The compounds of the formula (XV) can be prepared

[J] by reacting compounds of the formula (XVI)

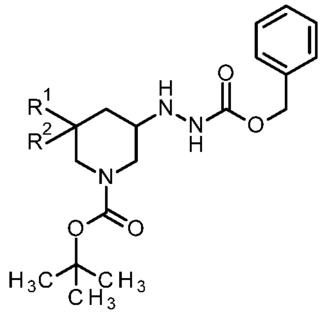

(XVI),

in which $R^1$ and $R^2$ are as defined above

with hydrogen in the presence of palladium on charcoal in a suitable solvent.

<u>Reaction [J] (Z deprotection)</u>

The reaction [J] is generally carried out in the presence of palladium on charcoal in a suitable solvent at from room temperature to reflux, preferable at 1 bar.

Suitable solvents are alcohols such as methanol, ethanol or isopropanol, ethers such as diethyl ether, diisopropyl ether, methyl tert-butyl ether, tetrahydrofuran or 1,4-dioxane, dichloromethane, polar solvents such as acetonitrile, *N*,*N* dimethylformamide (DMF), NMP, acetic acid or water or mixtures of such solvents; preference is given to ethanol/acetic acid.

The compounds of the formula (XVI)

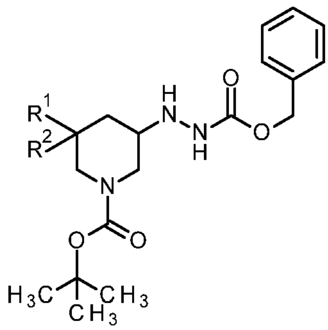

(XVI),

in which $R^1$ and $R^2$ are as defined above are novel.

The compounds of the formula (XVI) can be prepared

[K] by reacting compounds of the formula (XVII)

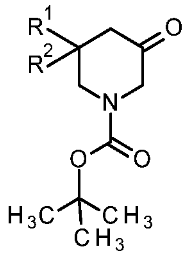

(XVII)

in which

$R^1$ and $R^2$ are as defined above

with a compound of the formula (XVIII)

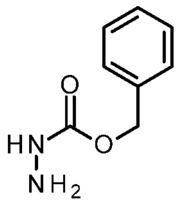

(XVIII)

in the presence of a reducing agent and a suitable solvent.

Reaction [K] (reductive hydrazination)

Reaction [K] is generally carried out in the presence of a reducing agent and a suitable solvent at a temperature range from room temperature up to reflux of the solvents at atmospheric pressure.

Suitable solvents are alcohols such as methanol, ethanol, n-propanol or isopropanol, ethers such as diisopropyl ether, methyl tert-butyl ether, tetrahydrofuran, 1,4-dioxane or 1,2-dimethoxyethane, polar aprotic solvents such as acetonitrile or *N*,*N* dimethylformamide (DMF) or mixtures of such solvents; preference is given to using tetrahydrofuran/methanol.

Suitable reducing agents are alkali metal borohydrides such as sodium borohydride, sodium cyanoborohydride or sodium triacetoxyborohydride; preference is given to using sodium borohydride.

The compound of the formula (XVIII) is known and commercial available or can be synthesized from the corresponding starting compounds by known processes.

The compound of the formula (XVII) is known and commercial available or can be synthesized from the corresponding starting compounds by known processes.

The preparation of the starting compounds and of the compounds of the formula (I) can be illustrated by the synthesis schemes 1 to 3 which follow.

Scheme 1

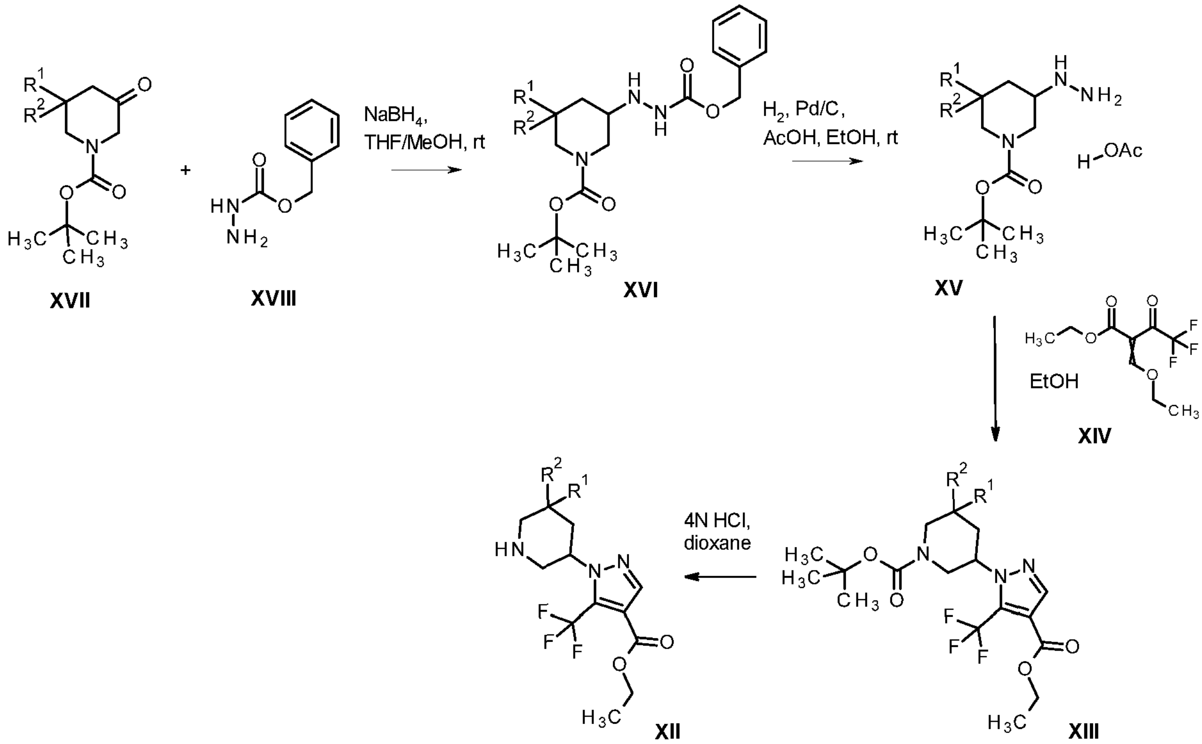


XVII XVIII XVI XV XIV XIII XII

Scheme 2

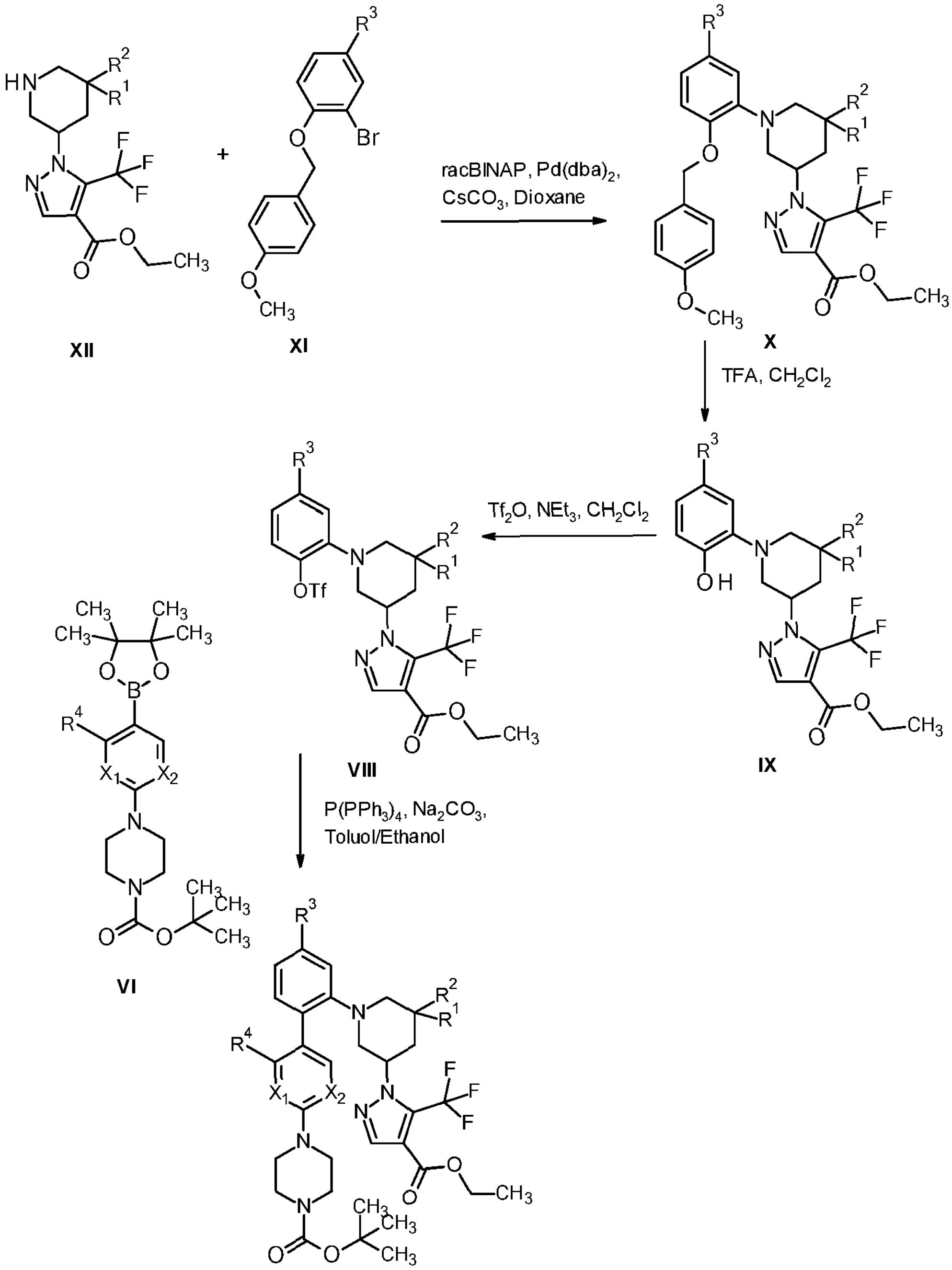


XII XI X IX VIII VI V

Scheme 3

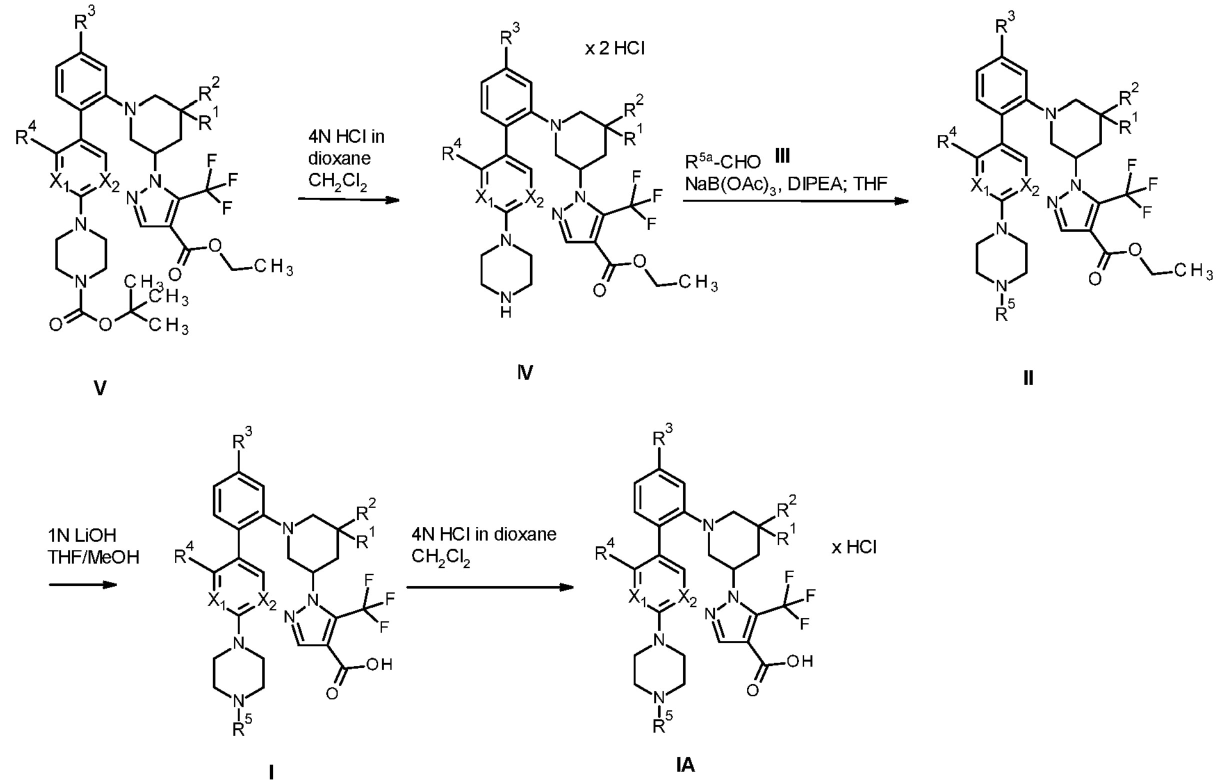


Any references to methods of treatment in the subsequent paragraphs of this description are to be interpreted as references to the compounds, pharmaceutical compositions and medicaments of the present invention for use in method of treatment of the human (or animal) body by therapy (or diagnosis).

The compounds of the invention have valuable pharmacological properties and can be used for prevention and treatment of diseases in humans and animals.

The compounds according to the invention are potent activators of soluble guanylate cyclase. They lead to vasorelaxation, inhibition of platelet aggregation and lowering of blood pressure and increase of coronary and renal blood flow. These effects are mediated via direct haem-independent activation of soluble guanylate cyclase and an increase of intracellular cGMP.

In addition, the compounds according to the invention have advantageous pharmacokinetic properties, in particular with respect to their bioavailability and/or duration of action after intravenous or oral administration.

The compounds according to the present invention show superior pharmacokinetic (PK) properties in comparison to compounds disclosed in the prior art (WO 2012/058132) (see experimental part, tables 3 to 6). For instance example 2 of the present invention shows a lower plasma clearance ($CL_{plasma}$) (up to 10 times) and therefore a much higher exposure in comparison to the prior art compound disclosed as example 174 in WO 2012/058132 in rats as well as in dogs. Example 2 shows also a long half-life and mean residence time (MRT) in all tested species after p.o. (per oral) application. Due to the significantly lower plasma clearance of example 2 and the resulting very high exposure ($AUC_{norm}$, exposure, area under curve normated) with good bioavailability after p.o. application in all tested species, we see a clear superiority of pharmacokinetic (PK) properties versus example 174 dislosed in WO 2012/058132.

The compounds according to the invention have an unforeseeable useful pharmacological activity spectrum and good pharmacokinetic behavior, in particular a sufficient exposure of such a compound in the blood above the minimal effective concentration within a given dosing interval after oral administration. Such a profile results in an improved peak-to-trough ratio (quotient of maximum to minimum concentration) within a given dosing interval, which has the advantage that the compound can be administered less frequently and at a significantly lower dose to achieve an effect. They are compounds that activate soluble guanylate cyclase.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

In the context of the present invention, the terms "prevention", "prophylaxis" and "preclusion" are used synonymously and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

In addition, the compounds according to the invention have further advantageous properties, in particular with respect to their pulmoselective action (in contrast to a systemic action), their lung retention time and/or their duration of action following intrapulmonary administration.

The compounds according to the invention are particularly suitable for the treatment and/or prevention of cardiovascular and cardiac diseases, cardio-renal and kidney diseases, cardiopulmonary and lung diseases, neurodegenerative diseases, thromboembolic diseases, fibrotic disorders and / or wound healing disorders.

The compounds according to the invention are particularly suitable for the treatment and/or prevention of cardiovascular and cardiac diseases, preferably heart failure with reduced and preserved ejection fraction (HFrEF, HFmrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU).

Accordingly, the compounds according to the invention can be used in medicaments for the treatment and/or prevention of cardiovascular, cardiopulmonary and cardiorenal disorders such as, for example high blood pressure (hypertension), heart failure, coronary heart disease, stable and unstable angina pectoris, pulmonary arterial hypertension (PAH) and secondary forms of pulmonary hypertension (PH), chronic thromboembolic pulmonary hypertension (CTEPH), renal, renovascular and treatment resistant hypertension, disorders of peripheral and cardiac vessels, arrhythmias, atrial and ventricular arrhythmias and impaired conduction such as, for example, grade I-III atrioventricular blocks, supraventricular tachy-arrhythmia, atrial fibrillation, atrial flutter, ventricular fibrillation, ventricular flutter, ventricular tachy-arrhythmia, Torsade de pointes tachycardia, atrial and ventricular extrasystoles, AV-junctional extrasystoles, sick sinus syndrome, syncopes, AV nodes reentry tachycardia, Wolff-Parkinson-White syndrome, acute coronary syndrome (ACS), autoimmune heart disorders (pericarditis, endocarditis, valvolitis, aortitis, cardiomyopathies), boxer cardiomyopathy, aneurysms, shock such as cardiogenic shock, septic shock and anaphylactic shock, furthermore for the treatment and/or prevention of thromboembolic disorders and ischaemias such as myocardial ischaemia, myocardial infarction, stroke, cardial hypertrophy, transistory and ischaemic attacks, preeclampsia, inflammatory cardiovascular disorders, spasms of the coronary - arteries and the peripheral arteries, formation of oedemas such as, for example, pulmonary oedema, brain oedema, renal oedema or heart failure-induced oedema, impaired peripheral perfusion, reperfusion damage, arterial and venous thromboses, microalbuminuria, heart failure, endothelial dysfunction, micro- and macrovascular damage (vasculitis), and also for preventing restenoses for example after thrombolysis therapies, percutaneous transluminal angioplasties (PTA), percutaneous transluminal coronary angioplasties (PTCA), heart transplants and bypass operations.

In the context of the present invention, the term "pulmonary hypertension" encompasses both primary and secondary subforms thereof, as defined below by the Dana Point classification according to their respective aetiology [see D. Montana and G. Simonneau, in: A.J. Peacock et al. (Eds.), Pulmonary Circulation. Diseases and their treatment, 3rd edition, Hodder Amold Publ., 2011, pp. 197-206; M.M. Hoeper et al., J. Am. Coll. Cardiol. 2009, 54 (1), S85-S96]. These include in particular in group 1 pulmonary arterial hypertension (PAH), which, among others, embraces the idiopathic and the familial forms (IPAH and FPAH, respectively). Furthermore, PAH also embraces persistent pulmonary hypertension of the newborn and the associated pulmonary arterial hypertension (APAH) associated with collagenoses, congenital systemic pulmonary shunt lesions, portal hypertension, HIV infections, the intake of certain drugs and medicaments (for example of appetite supressants), with disorders having a significant venous/capillary component such as pulmonary venoocclusive disorder and pulmonary capillary haemangiomatosis, or with other disorders such as disorders of the thyroid, glycogen storage diseases, Gaucher disease, hereditary teleangiectasia, haemoglobinopathies, myeloproliferative disorders and splenectomy. Group 2 of the Dana Point classification comprises PH patients having a causative left heart disorder, such as ventricular, atrial or valvular disorders. Group 3 comprises forms of pulmonary hypertension associated with a lung disorder, for example with chronic obstructive lung disease (COPD), interstitial lung disease (ILD), pulmonary fibrosis (IPF), and/or hypoxaemia (e.g. sleep apnoe syndrome, alveolar hypoventilation, chronic high-altitude sickness, hereditary deformities). Group 4 includes PH patients having chronic thrombotic and/or embolic disorders, for example in the case of thromboembolic obstruction of proximal and distal pulmonary arteries (CTEPH) or non-thrombotic embolisms (e.g. as a result of tumour disorders, parasites, foreign bodies). Less common forms of pulmonary hypertension, such as in patients suffering from sarcoidosis, histiocytosis X or lymphangiomatosis, are summarized in group 5.

In the context of the present invention, the term "heart failure" encompasses both acute and chronic forms of heart failure, and also more specific or related types of disease, such as acute decompensated heart failure, right heart failure, left heart failure, global heart failure, also diastolic heart failure and systolic heart failure, heart failure with reduced ejection fraction (HFrEF), heart failure with preserved ejection fraction (HFpEF), heart failure with mid-range efjection fraction (HFmEF), ischemic cardiomyopathy, dilated cardiomyopathy, hypertrophic cardiomyopathy, idiopathic cardiomyopathy, congenital heart defects and cardiomyopathies, heart valve defects, heart failure associated with heart valve defects, mitral valve stenosis, mitral valve insufficiency, aortic valve stenosis, aortic valve insufficiency, tricuspid valve stenosis, tricuspid valve insufficiency, pulmonary valve stenosis, pulmonary valve insufficiency, combined heart valve defects, myocardial inflammation (myocarditis), chronic myocarditis, acute myocarditis, viral myocarditis, diabetic heart failure, alcoholic cardiomyopathy, cardiac storage disorders.

In addition, the compounds according to the invention can also be used for treatment and/or prevention of arteriosclerosis, disturbed lipid metabolism, hypolipoproteinaemias, dyslipidaemias, hypertriglyceridaemias, hyperlipidaemias, combined hyperlipidaemias, hypercholesterolaemias, abetalipoproteinemia, sitosterolemia, xanthomatosis, Tangier disease, adiposity, obesity, and also of metabolic syndrome.

Furthermore, the compounds according to the invention can be used for treatment and/or prevention of primary and secondary Raynaud's phenomenon, of microcirculation disorders, claudication, hearing disorders, tinnitus, peripheral and autonomic neuropathies, diabetic microangiopathies, diabetic retinopathy, diabetic ulcers at the extremities, gangrene, CREST syndrome, erythematosis, onychomycosis and rheumatic disorders.

Futhermore, the compounds according to the invention can be used for the treatment of sickle cell disease (SCD), sickle cell anemia, and also other SCD-related disease symptoms (for example end organ damage affecting lung brain, kidney or heart) but also vasocclusive events or pain crisis, achalasia, hemolyis-induced vasculopathies for treating malaria, thalassemia, hemolytic uremic syndrome, paroxysmal nocturnal hemoglobinuria, drug-induced hemolytic anemias or rhabdomyolysis. In addition, since similar above-mentioned pathophysiological mechanisms are effective when blood transfusions (for example by storage etc. with an elevated concentration of free Hb) are administered to patients having a transfusion indication, this compounds could be used for patients receiving a blood transfusion. Finally, in the future the combination of an sGC activator with a synthetic Hb-based oxygen carrier may mitigate the side effects hitherto observed [Weiskopf, Anaesthesia & Analgesia, 110:3; 659-661, 2010] which are caused by reduced availability of NO, thus allowing further clinical applications.

The compounds according to the invention can additionally also be used for preventing ischaemic and/or reperfusion-related damage to organs or tissues and also as additives for perfusion and preservation solutions of organs, organ parts, tissues or tissue parts of human or animal origin, in particular for surgical interventions or in the field of transplantation medicine.

Furthermore, the compounds according to the invention are suitable for treatment and/or prophylaxis of renal disorders, especially of renal insufficiency and kidney failure. In the context of the present invention, the terms renal insufficiency and kidney failure comprise both acute and chronic manifestations (chronic kidney disease; CKD) thereof, as well as underlying or related kidney diseases such as renal hypoperfusion, intradialytic hypotension, obstructive uropathy, glomerulopathies, glomerulonephritis, acute glomerulonephritis, glomerulosclerosis, tubulointerstitial diseases, nephropathic diseases such as primary and congenital kidney disease, nephritis, immunological kidney diseases such as kidney graft rejection and immunocomplex-induced kidney diseases, nephropathy induced by toxic substances, nephropathy induced by contrast agents, diabetic and non-diabetic nephropathy, diabetic kidney diseas (DKD), pyelonephritis, renal cysts and polycystic kidney disease, nephrosclerosis, hypertensive nephrosclerosis and nephrotic syndrome, which can be characterized diagnostically for example by abnormally reduced creatinine and/or water excretion, abnormally raised blood concentrations of urea, nitrogen, potassium and/or creatinine, altered activity of renal enzymes such as, for example, glutamyl synthetase, altered urine osmolarity or urine volume, increased microalbuminuria, macroalbuminuria, lesions on glomerulae and arterioles, tubular dilation, hyperphosphataemia and/or need for dialysis. The present invention also encompasses the use of the compounds according to the invention for treatment and/or prophylaxis of sequelae of renal insufficiency, for example hypertension, pulmonary oedema, heart failure, uremia, anemia, electrolyte disturbances (for example hypercalemia, hyponatremia) and disturbances in bone and carbohydrate metabolism.

In addition, the compounds according to the invention are suitable for treatment and/or prevention of urological disorders, for example benign prostate syndrome (BPS), benign prostate hyperplasia (BPH), benign prostate enlargement (BPE), bladder outlet obstruction (BOO), lower urinary tract syndrome (LUTS), prostatitis, neurogenic overactive bladder (OAB), incontinence, for example mixed, urge, stress or overflow incontinence (MUI, UUI, SUI, OUI), pelvic pain, interstitial cystitis (IC) and also erectile dysfunction and female sexual dysfunction.

The compounds according to the invention are also suitable for treatment and/or prevention of asthmatic disorders, chronic-obstructive pulmonary diseases (COPD), acute respiratory distress syndrome (ARDS) and acute lung injury (ALI), alpha-1 antitrypsin deficiency (AATD), pulmonary fibrosis, pulmonary emphysema (for example pulmonary emphysema induced by cigarette smoke) and cystic fibrosis (CF).

The compounds described in the present invention are also active compounds for control of central nervous

system disorders characterized by disturbances of the NO/cGMP system. They are suitable in particular for improving perception, concentration, learning or memory after cognitive impairments like those occurring in particular in association with situations/diseases/syndromes such as mild cognitive impairment, age-associated learning and memory impairments, age-associated memory losses, vascular dementia, craniocerebral trauma, stroke, dementia occurring after strokes (post stroke dementia), post-traumatic craniocerebral trauma, general concentration impairments, concentration impairments in children with learning and memory problems, Alzheimer's disease, Lewy body dementia, dementia with degeneration of the frontal lobes including Pick's syndrome, Parkinson's disease, progressive nuclear palsy, dementia with corticobasal degeneration, amyolateral sclerosis (ALS), Huntington's disease, demyelination, multiple sclerosis, thalamic degeneration, Creutzfeld-Jacob dementia, HIV dementia, schizophrenia with dementia or Korsakoff's psychosis. They are also suitable for the treatment and/or prevention of central nervous system disorders such as states of anxiety, tension and depression, CNS-related sexual dysfunctions and sleep disturbances, and for controlling pathological disturbances of the intake of food, stimulants and addictive substances.

Furthermore, the compounds according to the invention are also suitable for regulation of cerebral blood flow and are thus effective agents for control of migraine. They are also suitable for the prophylaxis and control of sequelae of cerebral infarct (Apoplexia cerebri) such as stroke, cerebral ischaemias and craniocerebral trauma. The compounds according to the invention can likewise be used to control states of pain.

Moreover, the compounds according to the invention have antiinflammatory action and can therefore be used as antiinflammatories for treatment and/or prevention of sepsis (SIRS), multiple organ failure (MODS, MOF), inflammatory disorders of the kidney, chronic bowel inflammations (IBD, Crohn's Disease, UC), pancreatitis, peritonitis, rheumatoid disorders, inflammatory skin disorders and inflammatory eye disorders.

Furthermore, the compounds according to the invention are suitable for the treatment and/or prevention of fibrotic disorders of the internal organs, for example of the lung, of the heart, of the kidneys, of the bone marrow and especially of the liver, and also of dermatological fibroses and fibrotic disorders of the eye. In the context of the present inventions, the term "fibrotic disorders" encompasses especially disorders such as hepatic fibrosis, hepatic cirrhosis, non-alcoholic steato-hepatosis (NASH), pulmonary fibrosis, endomyocardial fibrosis, nephropathy, glomerulonephritis, interstitial renal fibrosis, fibrotic damage resulting from diabetes, myelofibrosis and similar fibrotic disorders, scleroderma, systemic sclerosis, morphea, keloids, hypertrophic scarring, naevi, diabetic retinopathy, proliferative vitreoretinopathy and disorders of the connective tissue (for example sarcoidosis). The compounds according to the invention can likewise be used for promoting wound healing including the healing of digital ulcer and diabeteic foot ulcer, for controlling postoperative scarring, for example resulting from glaucoma operations, and cosmetically for ageing and keratinized skin.

By virtue of their activity profile, the compounds according to the invention are particularly suitable for the treatment and/or prevention of cardiovascular and cardiac diseases and cardiopulmonary disorders such as primary and secondary forms of pulmonary hypertension, heart failure, angina pectoris and hypertension, and also for the treatment and/or prevention of thromboembolic disorders, ischaemias, vascular disorders, impaired microcirculation, renal insufficiency, fibrotic disorders and arteriosclerosis.

The present invention furthermore provides the use of the compounds according to the invention for the treatment and/or prevention of disorders, in particular the disorders mentioned above.

The present invention furthermore provides the use of the compounds according to the invention for preparing a medicament for the treatment and/or prevention of disorders, in particular the disorders mentioned above.

The present invention furthermore provides a medicament comprising at least one of the compounds according to the invention for the treatment and/or prevention of disorders, in particular the disorders mentioned above.

The present invention furthermore provides the use of the compounds according to the invention in a method for the treatment and/or prevention of disorders, in particular the disorders mentioned above.

The present invention furthermore provides a method for the treatment and/or prevention of disorders, in particular the disorders mentioned above, using an effective amount of at least one of the compounds according to the invention.

They are therefore suitable for use as medicaments for the treatment and/or prophylaxis of diseases in humans and animals.

The present invention further provides for the use of the compounds according to the invention for the treatment and/or prophylaxis of disorders, in particular cardiovascular disorders, preferably thrombotic or thromboembolic disorders and/or thrombotic or thromboembolic complications such as acute coronary syndrome or myocardial infarction or ischemic stroke or peripheral arterial occlusive disease , and/or diabetes, and/or urogenital disorders, in particular those associated with.

For the purpose of the present invention, the "thrombotic or thromboembolic disorders" include disorders which occur preferably in the arterial vasculature and which can be treated with the compounds according to the invention, in particular disorders leading to peripheral arterial occlusive disorders and in the coronary arteries of the heart, such as acute coronary syndrome (ACS), myocardial infarction with ST segment elevation (STEMI) and without ST segment elevation (non-STEMI), stable angina pectoris, unstable angina pectoris, reocclusions and restenoses after coronary

interventions such as angioplasty, stent implantation or aortocoronary bypass, but also thrombotic or thromboembolic disorders in cerebrovascular arteries, such as transitory ischaemic attacks (TIA), ischemic strokes including cardioembolic strokes, such as strokes due to atrial fibrillation, non-cardioembolic strokes, such as lacunar stroke, strokes due to large or small artery diseases, or strokes due to undetermined cause, cryptogenic strokes, embolic strokes, embolic strokes of undetermined source, or events of thrombotic and/or thromboembolic origin leading to stroke or TIA.

Moreover, the compounds according to the invention are suitable in particular for the treatment and/or prophylaxis of disorders where, the pro-inflammatory component plays an essential role, including vasculitides like Kawasaki disease, Takayasu arteritis and Thrombangiitis obliterans (Buerger's disease) as well as inflammatory disorders like myocarditis.

Furthermore, the compounds according to the invention are suitable for the treatment and/or prophylaxis of disorders of the urogenital tract like overactive bladder, interstitial cystitis and bladder pain syndrome.

Moreover, the compounds according to the invention are suitable for the treatment and/or prophylaxis of diabetes mellitus including its end-organ manifestations like diabetic retinopathy and diabetic nephropathy.

Furthermore, the compounds according to the invention are suitable in particular for the treatment and/or prophylaxis of neurological disorders like neuropathic pain, neurodegenerative disorders and dementias such as vascular dementia or Alzheimer's disease and Parkinson's disease.

Moreover, the compounds according to the invention are suitable in particular for the treatment and/or prophylaxis of pulmonologic disorders like chronic cough, asthma and COPD.

The present invention further provides for the use of the compounds according to the invention for the treatment and/or prophylaxis of disorders, especially the disorders mentioned above.

The present invention further provides for the use of the compounds according to the invention for production of a medicament for the treatment and/or prophylaxis of disorders, especially the disorders mentioned above.

The present invention further provides a method for the treatment and/or prophylaxis of disorders, especially the disorders mentioned above, using a therapeutically effective amount of a compound according to the invention.

The present invention further provides the compounds according to the invention for use in a method for the treatment and/or prophylaxis of disorders, especially the disorders mentioned above, using a therapeutically effective amount of a compound according to the invention.

Particularly the present invention provides the compounds according to the invention for use in a method for the treatment and/or prophylaxis of thrombotic or thromboembolic, in particular atherothrombotic disorders using a therapeutically effective amount of a compound according to the invention.

The present invention further provides medicaments comprising a compound according to the invention and one or more further active compounds.

In addition, the compounds according to the invention can also be used for preventing coagulation ex vivo, for example for the protection of organs to be transplanted against organ damage caused by formation of clots and for protecting the organ recipient against thromboemboli from the transplanted organ, for preserving blood and plasma products, for cleaning/pretreating catheters and other medical auxiliaries and instruments, for coating synthetic surfaces of medical auxiliaries and instruments used in vivo or ex vivo or for biological samples which may comprise factor XIa or plasma kallikrein.

The present invention furthermore provides a method for preventing the coagulation of blood in vitro, in particular in banked blood or biological samples which may comprise factor XIa or plasma kallikrein or both enzymes, which method is characterized in that an anticoagulatory effective amount of the compound according to the invention is added.

The compounds of the invention can act systemically and/or locally. For this purpose, they can be administered in a suitable manner, for example by the oral, parenteral, pulmonal, nasal, sublingual, lingual, buccal, rectal, dermal, transdermal, conjunctival or otic route, or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Suitable for extraocular (topic) administration are administration forms which operate in accordance with the prior art, which release the active compound rapidly and/or in a modified or controlled manner and which contain the active compound in crystalline and/or amorphized and/or dissolved form such as, for example, eye drops, sprays and lotions (e.g. solutions, suspensions, vesicular/colloidal systems, emulsions, aerosols), powders for eye drops, sprays and lotions (e.g. ground active compound, mixtures, lyophilisates, precipitated active compound), semisolid eye preparations (e.g. hydrogels, in-situ hydrogels, creams and ointments), eye inserts (solid and semisolid preparations, e.g. bioadhesives, films/wafers, tablets, contact lenses).

Intraocular administration includes, for example, intravitreal, subretinal, subscleral, intrachoroidal, subconjunctival, retrobulbar and subtenon administration. Suitable for intraocular administration are administration forms which operate in accordance with the prior art, which release the active compound rapidly and/or in a modified or controlled manner and which contain the active compound in crystalline and/or amorphized and/or dissolved form such as, for example, preparations for injection and concentrates for preparations for injection (e.g. solutions, suspensions, vesicular/colloidal systems, emulsions), powders for preparations for injection (e.g. ground active compound, mixtures, lyophilisates, precipitated active compound), gels for preparations for injection (semisolid preparations, e.g. hydrogels, in-situ hydrogels) and implants (solid preparations, e.g. biodegradable and nonbiodegradable implants, implantable pumps).

Preference is given to oral administration.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,

- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc,

highly-disperse silicas (such as, for example, Aerosil®)),

- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),

- capsule materials (for example gelatine, hydroxypropylmethylcellulose),

- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),

- plasticizers (for example polyethylene glycols, propylene glycol, glycerol, triacetine, triacetyl citrate, dibutyl phthalate),

- penetration enhancers,

- stabilisers (for example antioxidants such as, for example, ascorbic acid, ascorbyl palmitate, sodium ascorbate, butylhydroxyanisole, butylhydroxytoluene, propyl gallate),

- preservatives (for example parabens, sorbic acid, thiomersal, benzalkonium chloride, chlorhexidine acetate, sodium benzoate),

- colourants (for example inorganic pigments such as, for example, iron oxides, titanium dioxide),

- flavourings, sweeteners, flavour- and/or odour-masking agents.

The present invention furthermore relates to a pharmaceutical composition which comprises at least one compound according to the invention, conventionally together with one or more pharmaceutically suitable excipient(s), and to their use according to the present invention.

An embodiment of the invention are pharmaceutical compositions comprising at least one compound of formula (I) according to the invention, preferably together with at least one inert, non-toxic, pharmaceutically suitable auxiliary, and the use of these pharmaceutical compositions for the above cited purposes.

In accordance with another aspect, the present invention covers pharmaceutical combinations, in particular medicaments, comprising at least one compound of general formula (I) of the present invention and at least one or more further active ingredients, in particular for the treatment and/or prophylaxis of cardiovascular disorders, preferably thrombotic or thromboembolic disorders, and diabetes, and also urogenital and ophthalmic disorders.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

The inventive compounds can be employed alone or, if required, in combination with other active ingredients. The present invention further provides medicaments comprising at least one of the inventive compounds and one or more further active ingredients, especially for treatment and/or prophylaxis of the aforementioned disorders. Preferred examples of suitable active ingredient combinations include:

- organic nitrates and NO donors, for example sodium nitroprusside, nitroglycerin, isosorbide mononitrate, isosorbide dinitrate, molsidomine or SIN-1, and inhaled NO;

- compounds which inhibit the breakdown of cyclic guanosine monophosphate (cGMP), for example inhibitors of phosphodiesterases (PDE) 1, 2, 5 and/or 9, especially PDE 5 inhibitors such as sildenafil, vardenafil, tadalafil, udenafil, desantafil, avanafil, mirodenafil, lodenafil or PF-00489791;

- compounds which inhibit the breakdown of cyclic adenosine monophosphate (cAMP), for example inhibitors of phosphodiesterases (PDE) 3 and 4, especially cilostatzole, milrinone, roflumilast, apremilast, or crisaborole;

- hypotensive active ingredients, by way of example and with preference from the group of the calcium antagonists, angiotensin AII antagonists, ACE inhibitors, NEP-inhibitors, vasopeptidase-inhibitors, endothelin antagonists, renin inhibitors, alpha-receptor blockers, beta-receptor blockers, mineralocorticoid receptor antagonists, rho-kinase-inhibitors and the diuretics;

- antiarrhythmic agents, by way of example and with preference from the group of sodium channel blocker, beta-receptor blocker, potassium channel blocker, calcium antagonists, If-channel blocker, digitalis, parasympatholytics (vagoliytics), sympathomimetics and other antiarrhythmics as adenosin, adenosine receptor agonists as well as vemakalant;

- positive-inotrop agents, by way of example cardiac glycoside (Dogoxin), beta-adrenergic and dopaminergic agonists, such as isoprenalin, adrenalin, noradrenalin, dopamin or dobutamin;

- vasopressin-receptor-antagonists, by way of example and with preference from the group of conivaptan, tolvaptan, lixivaptan, mozavaptan, satavaptan, pecavaptan, SR-121463, RWJ 676070 or BAY 86-8050, as well as the compounds described in WO 2010/105770, WO2011/104322 and WO 2016/071212;

- active ingredients which alter lipid metabolism, for example and with preference from the group of the thyroid receptor agonists, cholesterol synthesis inhibitors such as, by way of example and preferably, HMG-CoA reductase inhibitors or squalene synthesis inhibitors, of ACAT inhibitors, CETP inhibitors, MTP inhibitors, PPAR-alpha, PPAR-gamma and/or PPAR-delta agonists, cholesterol absorption inhibitors, lipase inhibitors, polymeric bile acid adsorbents, bile acid reabsorption inhibitors and lipoprotein(a) antagonists.

- bronchodilatory agents, for example and with preference from the group of the beta-adrenergic rezeptor-agonists, such as, by way of example and preferably, albuterol, isoproterenol, metaproterenol, terbutalin, formoterol or salmeterol, or from the group of the anticholinergics, such as, by way of example and preferably, ipratropiumbromid;

- anti-inflammatory agents, for example and with preference from the group of the glucocorticoids, such as, by way of example and preferably, prednison, prednisolon, methylprednisolon, triamcinolon, dexamethason, beclomethason, betamethason, flunisolid, budesonid or fluticason as well as the non-steroidal anti-inflammatory agents (NSAIDs), by way of example and preferably, acetyl salicylic acid (aspirin), ibuprofen and naproxen, 5-amino salicylic acid-derivates, leukotriene-antagonists, TNF-alpha-inhibitors and chemokin-receptor antagonists, such as CCR1, 2 and/or 5 inhibitors;

- agents modulating the immune system, for example immunoglobulins;

- agents that inhibit the signal transductions cascade, for example and with preference from the group of the kinase inhibitors, by way of example and preferably, from the group of the tyrosine kinase- and/or serine/threonine kinase inhibitors;

- agents, that inhibit the degradation and modification of the extracellular matrix, for example and with preference from the group of the inhibitors of the matrix-metalloproteases (MMPs), by way of example and preferably, inhibitors of chymasee, stromelysine, collagenases, gelatinases and aggrecanases (with preference from the group of MMP-1, MMP-3, MMP-8, MMP-9, MMP-10, MMP-11 and MMP-13) as well as of the metallo-elastase (MMP-12) and neutrophil-elastase (HNE), as for example sivelestat or DX-890;

- agents, that block the bindung of serotonin to its receptor, for example and with preference antagonists of the 5-HT2b-receptor;

- organic nitrates and NO-donators, for example and with preference sodium nitroprussid, nitroglycerine, isosorbid mononitrate, isosorbid dinitrate, molsidomine or SIN-1, as well as inhaled NO;
- NO-independent, but heme-dependent stimulators of the soluble guanylate cyclase, for example and with preference the compounds described in WO 00/06568, WO 00/06569, WO 02/42301, WO 03/095451, WO 2011/147809, WO 2012/004258, WO 2012/028647 and WO 2012/059549;
- NO-independent and heme-independent activators of the soluble guanylate cyclase, for example and with preference the compounds described in WO 01/19355, WO 01/19776, WO 01/19778, WO 01/19780, WO 02/070462 and WO 02/070510 beschriebenen Verbindungen;
- agents, that stimulates the synthesis of cGMP, like for example sGC modulators, for example and with preference riociguat, cinaciguat, vericiguat or runcaciguat;
- prostacyclin-analogs, for example and with preference iloprost, beraprost, treprostinil or epoprostenol;
- agents, that inhibit soluble epoxidhydrolase (sEH), for example and with preference N,N'-Dicyclohexyl urea, 12-(3-Adamantan-1-yl-ureido)-dodecanic acid or 1-Adamantan-1-yl-3-{5-[2-(2-ethoxyethoxy)ethoxy]pentyl} -urea;
- agents that interact with glucose metabolism, for example and with preference insuline, biguanide, thiazolidinedione, sulfonyl urea, acarbose, DPP4 inhibitors, GLP-1 analogs or SGLT-2 inhibitors, for example empagliflozin, dapagliflozin, canagliflozin, sotagliflozin;
- natriuretic peptides, for example and with preference atrial natriuretic peptide (ANP), natriuretic peptide type B (BNP, Nesiritid) natriuretic peptide type C (CNP) or urodilatin;
- activators of the cardiac myosin, for example and with preference omecamtiv mecarbil (CK-1827452);
- calcium-sensitizers, for example and with preference levosimendan;
- agents that affect the energy metabolism of the heart, for example and with preference etomoxir, dichloroacetat, ranolazine or trimetazidine, full or partial adenosine A1 receptor agonists such as GS-9667 (formerly known as CVT-3619), capadenoson, neladenoson and neladenoson bialanate;
- agents that affect the heart rate, for example and with preference ivabradin;
- cyclooxygenase inhibitors such as, for example, bromfenac and nepafenac;
- inhibitors of the kallikrein-kinin system such as, for example, safotibant and ecallantide;
- inhibitors of the sphingosine 1-phosphate signal paths such as, for example, sonepcizumab;
- inhibitors of the complement-C5a receptor such as, for example, eculizumab;
- plasminogen activators (thrombolytics/fibrinolytics) and compounds which promote thrombolysis/fibrinolysis such as inhibitors of the plasminogen activator inhibitor (PAI inhibitors) or inhibitors of the thrombin-activated fibrinolysis inhibitor (TAFI inhibitors) such as, for example, tissue plasminogen activator (t-PA, for example Actilyse®), streptokinase, reteplase and urokinase or plasminogen-modulating substances causing increased formation of plasmin;
- anticoagulatory substances (anticoagulants) such as, for example, heparin (UFH), low-molecular-weight heparins (LMW), for example tinzaparin, certoparin, pamaparin, nadroparin, ardeparin, enoxaparin, reviparin, dalteparin, danaparoid, semuloparin (AVE 5026), adomiparin (M118) and EP-42675/ORG42675;
- direct thrombin inhibitors (DTI) such as, for example, Pradaxa (dabigatran), atecegatran (AZD-0837), DP-4088, SSR-182289A, argatroban, bivalirudin and tanogitran (BIBT-986 and prodrug BIBT-1011) and hirudin;
- direct factor Xa inhibitors such as, for example, rivaroxaban, apixaban, edoxaban (DU-176b), betrixaban (PRT-54021), R-1663, darexaban (YM-150), otamixaban (FXV-673/RPR-130673), letaxaban (TAK-442), razaxaban

(DPC-906), DX-9065a, LY-517717, tanogitran (BIBT-986, prodrug: BIBT-1011), idraparinux and fondaparinux;

- inhibitors of coagulation factor XI and XIa such as, for example, FXI ASO-LICA, fesomersen, BAY 121-3790, MAA868, BMS986177, EP-7041 and AB-022;
- substances which inhibit the aggregation of platelets (platelet aggregation inhibitors, thrombocyte aggregation inhibitors), such as, for example, acetylsalicylic acid (such as, for example, aspirin), P2Y12 antagonists such as, for example, ticlopidine (Ticlid), clopidogrel (Plavix), prasugrel, ticagrelor, cangrelor and elinogrel, and PAR-1 antagonists such as, for example, vorapaxar, and PAR-4 antagonists;
- platelet adhesion inhibitors such as GPVI and/or GPIb antagonists such as, for example, Revacept or caplacizumab;
- fibrinogen receptor antagonists (glycoprotein-IIb/IIIa antagonists) such as, for example, abciximab, eptifibatide, tirofiban, lamifiban, lefradafiban and fradafiban;
- recombinant human activated protein C such as, for example, Xigris or recombinant thrombomodulin.

Antithrombotic agents are preferably understood to mean compounds from the group of the platelet aggregation inhibitors, the anticoagulants or the profibrinolytic substances.

In a preferred embodiment of the invention, the inventive compounds are administered in combination with a platelet aggregation inhibitor, by way of example and with preference aspirin, clopidogrel, prasugrel, ticagrelor, ticlopidin or dipyridamole.

In a preferred embodiment of the invention, the inventive compounds are administered in combination with a thrombin inhibitor, by way of example and with preference ximelagatran, dabigatran, melagatran, bivalirudin or clexane.

In a preferred embodiment of the invention, the inventive compounds are administered in combination with a GPIIb/IIIa antagonist such as, by way of example and with preference, tirofiban or abciximab.

In a preferred embodiment of the invention, the inventive compounds are administered in combination with a factor Xa inhibitor, by way of example and with preference rivaroxaban (BAY 59-7939), DU-176b, apixaban, betrixaban, otamixaban, fidexaban, razaxaban, letaxaban, eribaxaban, fondaparinux, idraparinux, PMD-3112, darexaban (YM-150), KFA-1982, EMD-503982, MCM-17, MLN-1021, DX 9065a, DPC 906, JTV 803, SSR-126512 or SSR-128428.

In a preferred embodiment of the invention, the inventive compounds are administered in combination with a factor XI or factor XIa inhibitor, by way of example and with preference FXI ASO-LICA, fesomersen, BAY 121-3790, MAA868, BMS986177, EP-7041 or AB-022.

In a preferred embodiment of the invention, the inventive compounds are administered in combination with heparin or with a low molecular weight (LMW) heparin derivative.

In a preferred embodiment of the invention, the inventive compounds are administered in combination with a vitamin K antagonist, by way of example and with preference coumarin.

Hypotensive agents are preferably understood to mean compounds from the group of the calcium antagonists, angiotensin AII antagonists, ACE inhibitors, endothelin antagonists, renin inhibitors, alpha-receptor blockers, beta-receptor blockers, mineralocorticoid receptor antagonists, rho-kinase inhibitors and the diuretics.

In a preferred embodiment of the invention, the inventive compounds are administered in combination with a calcium antagonist, by way of example and with preference nifedipine, amlodipine, verapamil or diltiazem.

In a preferred embodiment of the invention, the inventive compounds are administered in combination with an alpha-1-receptor blocker, by way of example and with preference prazosin.

In a preferred embodiment of the invention, the inventive compounds are administered in combination with a beta-receptor blocker, by way of example and with preference propranolol, atenolol, timolol, pindolol, alprenolol, oxprenolol, penbutolol, bupranolol, metipranolol, nadolol, mepindolol, carazalol, sotalol, metoprolol, betaxolol, celiprolol, bisoprolol, carteolol, esmolol, labetalol, carvedilol, adaprolol, landiolol, nebivolol, epanolol or bucindolol.

In a preferred embodiment of the invention, the inventive compounds are administered in combination with an angiotensin AII antagonist, by way of example and with preference losartan, candesartan, valsartan, telmisartan or embusartan or a dual angiotensin AII antagonist/neprilysin-inhibitor, by way of example and with preference LCZ696 (valsartan/sacubitril).

In a preferred embodiment of the invention, the inventive compounds are administered in combination with an ACE inhibitor, by way of example and with preference enalapril, captopril, lisinopril, ramipril, delapril, fosinopril, quinopril, perindopril or trandopril.

In a preferred embodiment of the invention, the inventive compounds are administered in combination with an endothelin antagonist, by way of example and with preference bosentan, darusentan, ambrisentan or sitaxsentan.

In a preferred embodiment of the invention, the inventive compounds are administered in combination with a

renin inhibitor, by way of example and with preference aliskiren, SPP-600 or SPP-800.

In a preferred embodiment of the invention, the inventive compounds are administered in combination with a mineralocorticoid receptor antagonist, by way of example and with preference spironolactone, AZD9977, finerenone or eplerenone.

In a preferred embodiment of the invention, the inventive compounds are administered in combination with a loop diuretic, for example furosemide, torasemide, bumetanide and piretanide, with potassium-sparing diuretics, for example amiloride and triamterene, with aldosterone antagonists, for example spironolactone, potassium canrenoate and eplerenone, and also thiazide diuretics, for example hydrochlorothiazide, chlorthalidone, xipamide and indapamide.

Lipid metabolism modifiers are preferably understood to mean compounds from the group of the CETP inhibitors, thyroid receptor agonists, cholesterol synthesis inhibitors such as HMG-CoA reductase inhibitors or squalene synthesis inhibitors, the ACAT inhibitors, MTP inhibitors, PPAR-alpha, PPAR-gamma and/or PPAR-delta agonists, cholesterol absorption inhibitors, polymeric bile acid adsorbents, bile acid reabsorption inhibitors, lipase inhibitors and the lipoprotein(a) antagonists.

In a preferred embodiment of the invention, the inventive compounds are administered in combination with a CETP inhibitor, by way of example and with preference dalcetrapib,anacetrapib, torcetrapib (CP-529 414), JJT-705 or CETP vaccine (Avant).

In a preferred embodiment of the invention, the inventive compounds are administered in combination with a thyroid receptor agonist, by way of example and with preference D-thyroxine, 3,5,3'-triiodothyronine (T3), CGS 23425 or axitirome (CGS 26214).

In a preferred embodiment of the invention, the inventive compounds are administered in combination with an HMG-CoA reductase inhibitor from the class of statins, by way of example and with preference lovastatin, simvastatin, pravastatin, fluvastatin, atorvastatin, rosuvastatin or pitavastatin.

In a preferred embodiment of the invention, the inventive compounds are administered in combination with a squalene synthesis inhibitor, by way of example and with preference BMS-188494 or TAK-475.

In a preferred embodiment of the invention, the inventive compounds are administered in combination with an ACAT inhibitor, by way of example and with preference avasimibe, melinamide, pactimibe, eflucimibe or SMP-797.

In a preferred embodiment of the invention, the inventive compounds are administered in combination with an MTP inhibitor, by way of example and with preference implitapide, BMS-201038, R-103757 or JTT-130.

In a preferred embodiment of the invention, the inventive compounds are administered in combination with a PPAR-gamma agonist, by way of example and with preference pioglitazone or rosiglitazone.

In a preferred embodiment of the invention, the inventive compounds are administered in combination with a PPAR-delta agonist, by way of example and with preference GW 501516 or BAY 68-5042.

In a preferred embodiment of the invention, the inventive compounds are administered in combination with a cholesterol absorption inhibitor, by way of example and with preference ezetimibe, tiqueside or pamaqueside.

In a preferred embodiment of the invention, the inventive compounds are administered in combination with a lipase inhibitor, a preferred example being orlistat.

In a preferred embodiment of the invention, the inventive compounds are administered in combination with a polymeric bile acid adsorbent, by way of example and with preference cholestyramine, colestipol, colesolvam, CholestaGel or colestimide.

In a preferred embodiment of the invention, the inventive compounds are administered in combination with a bile acid reabsorption inhibitor, by way of example and with preference ASBT (= IBAT) inhibitors, for example AZD-7806, S-8921, AK-105, BARI-1741, SC-435 or SC-635.

In a preferred embodiment of the invention, the inventive compounds are administered in combination with a lipoprotein(a) antagonist, by way of example and with preference, gemcabene calcium (CI-1027) or nicotinic acid.

In a preferred embodiment of the invention, the inventive compounds are administered in combination with a lipoprotein(a) antagonist, by way of example and with preference, gemcabene calcium (CI-1027) or nicotinic acid.

In a preferred embodiment of the invention, the inventive compounds are administered in combination with sGC modulators, by way of example and with preference, riociguat, cinaciguat or vericiguat.

In a preferred embodiment of the invention, the inventive compounds are administered in combination with an agent affecting the glucose metabolism, by way of example and with preference, insuline, a sulfonyl urea, acarbose, DPP4 inhibitors, GLP-1 analogs or SGLT-1 inhibitors empagliflozin, dapagliflozin, kanagliflozin, sotagliflozin.

In a preferred embodiment of the invention, the compounds according to the invention are administered in combination with a TGFbeta antagonist, by way of example and with preference pirfenidone or fresolimumab.

In a preferred embodiment of the invention, the compounds according to the invention are administered in combination with a CCR2 antagonist, by way of example and with preference CCX-140.

In a preferred embodiment of the invention, the compounds according to the invention are administered in combination with a TNFalpha antagonist, by way of example and with preference adalimumab.

In a preferred embodiment of the invention, the compounds according to the invention are administered in

combination with a galectin-3 inhibitor, by way of example and with preference GCS-100.

In a preferred embodiment of the invention, the compounds according to the invention are administered in combination with a Nrf-2 inhibitor, by way of example and with preference bardoxolone

In a preferred embodiment of the invention, the compounds according to the invention are administered in combination with a BMP-7 agonist, by way of example and with preference THR-184.

In a preferred embodiment of the invention, the compounds according to the invention are administered in combination with a NOX1/4 inhibitor, by way of example and with preference GKT-137831.

In a preferred embodiment of the invention, the compounds according to the invention are administered in combination with a medicament which affects the vitamin D metabolism, by way of example and with preference calcitriol, alfacalcidol, doxercalciferol, maxacalcitol, paricalcitol, cholecalciferol or paracalcitol.

In a preferred embodiment of the invention, the compounds according to the invention are administered in combination with a cytostatic agent, by way of example and with preference cyclophosphamide.

In a preferred embodiment of the invention, the compounds according to the invention are administered in combination with an immunosuppressive agent, by way of example and with preference ciclosporin.

In a preferred embodiment of the invention, the compounds according to the invention are administered in combination with a phosphate binder, by way of example and with preference colestilan, sevelamer hydrochloride and sevelamer carbonate, Lanthanum and lanthanum carbonate.

In a preferred embodiment of the invention, the compounds according to the invention are administered in combination with renal proximal tubule sodium-phosphate co-transporter, by way of example and with preference, niacin or nicotinamide.

In a preferred embodiment of the invention, the compounds according to the invention are administered in combination with a calcimimetic for therapy of hyperparathyroidism.

In a preferred embodiment of the invention, the compounds according to the invention are administered in combination with agents for iron deficit therapy, by way of example and with preference iron products.

In a preferred embodiment of the invention, the compounds according to the invention are administered in combination with agents for the therapy of hyperurikaemia, by way of example and with preference allopurinol or rasburicase.

In a preferred embodiment of the invention, the compounds according to the invention are administered in combination with glycoprotein hormone for the therapy of anaemia, by way of example and with preference erythropoietin, daprodustat, molidustat, roxadustat, vadadustat, desidustat.

In a preferred embodiment of the invention, the compounds according to the invention are administered in combination with biologics for immune therapy, by way of example and with preference abatacept, rituximab, eculizumab or belimumab.

In a preferred embodiment of the invention, the compounds according to the invention are administered in combination with vasopressin antagonists (group of the vaptanes) for the treatment of heart failure, by way of example and with preference tolvaptan, conivaptan, lixivaptan, mozavaptan, satavaptan, pecavaptan or relcovaptan.

In a preferred embodiment of the invention, the compounds according to the invention are administered in combination with Jak inhibitors, by way of example and with preference ruxolitinib, tofacitinib, baricitinib, CYT387, GSK2586184, lestaurtinib, pacritinib (SB1518) or TG101348.

In a preferred embodiment of the invention, the compounds according to the invention are administered in combination with prostacyclin analogs for therapy of microthrombi.

In a preferred embodiment of the invention, the compounds according to the invention are administered in combination with an alkali therapy, by way of example and with preference sodium bicarbonate.

In a preferred embodiment of the invention, the compounds according to the invention are administered in combination with an mTOR inhibitor, by way of example and with preference everolimus or rapamycin.

In a preferred embodiment of the invention, the compounds according to the invention are administered in combination with an NHE3 inhibitor, by way of example and with preference AZD1722 or tenapanor.

In a preferred embodiment of the invention, the compounds according to the invention are administered in combination with an eNOS modulator, by way of example and with preference sapropterin.

In a preferred embodiment of the invention, the compounds according to the invention are administered in combination with a CTGF inhibitor, by way of example and with preference FG-3019.

The present invention further provides medicaments which comprise at least one compound according to the invention, typically together with one or more inert, nontoxic, pharmaceutically suitable auxiliaries, and the use thereof for the aforementioned purposes.

The compounds according to the invention may act systemically and/or locally. For this purpose, they can be administered in a suitable manner, for example by the oral, parenteral, pulmonal, nasal, sublingual, lingual, buccal, rectal, dermal, transdermal, conjunctival, otic route, or as an implant or stent.

The compounds according to the invention can be administered in administration forms suitable for these

administration routes.

Suitable administration forms for oral administration are those which work according to the prior art, which release the compounds according to the invention rapidly and/or in a modified manner and which contain the compounds according to the invention in crystalline and/or amorphized and/or dissolved form, for example tablets (uncoated or coated tablets, for example with gastric juice-resistant or retarded-dissolution or insoluble coatings which control the release of the compound according to the invention), tablets or films/wafers which disintegrate rapidly in the oral cavity, films/lyophilizates or capsules (for example hard or soft gelatin capsules), sugar-coated tablets, granules, pellets, powders, emulsions, suspensions, aerosols or solutions.

Parenteral administration can bypass an absorption step (e.g. intravenously, intraarterially, intracardially, intraspinally or intralumbally) or include an absorption (e.g. intramuscularly, subcutaneously, intracutaneously, percutaneously or intraperitoneally). Administration forms suitable for parenteral administration include preparations for injection and infusion in the form of solutions, suspensions, emulsions, lyophilizates or sterile powders.

For the other administration routes, suitable examples are inhalable medicament forms (including powder inhalers, nebulizers), nasal drops, solutions or sprays, tablets, films/wafers or capsules for lingual, sublingual or buccal administration, suppositories, ear or eye preparations, vaginal capsules, aqueous suspensions (lotions, shaking mixtures), lipophilic suspensions, ointments, creams, transdermal therapeutic systems (e.g. patches), milk, pastes, foams, sprinkling powders, implants or stents.

Oral or parenteral administration is preferred, especially oral and intravenous administration.

The compounds according to the invention can be converted to the administration forms mentioned. This can be done in a manner known per se, by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), dyes (e.g. inorganic pigments, for example iron oxides) and flavour and/or odour correctants.

In general, it has been found to be advantageous in the case of parenteral administration to administer amounts of about 0.001 to 1 mg/kg, preferably about 0.01 to 0.5 mg/kg, of body weight to achieve effective results. In the case of oral administration, the dosage is about 0.01 to 100 mg/kg, preferably about 0.01 to 20 mg/kg and most preferably 0.1 to 10 mg/kg of body weight.

It may nevertheless be necessary where appropriate to deviate from the stated amounts, specifically as a function of the body weight, route of administration, individual response to the active compound, nature of the preparation and time or interval over which administration takes place. For instance, in some cases, less than the aforementioned minimum amount may be sufficient, while in other cases the upper limit mentioned must be exceeded. In the case of administration of relatively large amounts, it may be advisable to divide these into several individual doses over the course of the day.

The total amount of the active ingredient to be administered will generally range from about 0.001 mg/kg to about 200 mg/kg body weight per day, and preferably from about 0.01 mg/kg to about 50 mg/kg body weight per day, and more preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

Nevertheless, it may optionally be necessary to deviate from the stated amounts, namely depending on body weight, route of administration, individual response to the active substance, type of preparation and time point or interval when application takes place. Thus, in some cases it may be sufficient to use less than the aforementioned minimum amount, whereas in other cases the stated upper limit must be exceeded. When applying larger amounts, it may be

advisable to distribute these in several individual doses throughout the day.

According to a further embodiment, the compounds of formula (I) according to the invention are administered orally once or twice or three times a day. According to a further embodiment, the compounds of formula (I) according to the invention are administered orally once or twice a day. According to a further embodiment, the compounds of formula (I) according to the invention are administered orally once a day. For the oral administration, a rapid release or a modified release dosage form may be used.

Unless stated otherwise, the percentages in the tests and examples which follow are percentages by weight; parts are parts by weight. Solvent ratios, dilution ratios and concentration data for the liquid/liquid solutions are based in each case on volume. "w/v" means "weight/volume". For example, "10% w/v" means: 100 ml of solution or suspension comprise 10 g of substance.

## EXPERIMENTAL SECTION

## EXPERIMENTAL SECTION - GENERAL PART

NMR peak forms are stated as they appear in the spectra, possible higher order effects have not been considered.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary per se to the skilled person.

**Table 1: Abbreviations**

The following table lists the abbreviations used herein.

| Abbreviation | Meaning |
|---|---|
| $BH_3 \cdot THF$ | Borane-tetrahydrofuran |
| BINAP | 2,2'-Bis(diphenylphosphino)-1,1'-binaphthyl |
| br | broad ($^1$H-NMR signal) |
| CI | chemical ionisation |
| d | doublet ($^1$H-NMR signal) |
| d | day(s) |
| DAD | diode array detector |
| dd | double-doublet |
| DMF | N,N-dimethylformamide |
| DMSO | dimethylsulfoxide |
| ESI | electrospray (ES) ionisation |
| EtOAc | Ethyl acetate |
| h | hour(s) |
| HATU | 1-[Bis(dimethylamino)methylene]-1H-1,2,3-triazolo[4,5-b]pyridinium 3-oxide hexafluorophosphate, |
| HPLC | CAS 148893-10-1 high performance liquid chromatography |
| LC-MS | liquid chromatography mass spectrometry |
| m | multiplet ($^1$H-NMR signal) |
| M | molar |
| min | minute (s) |
| MS | mass spectrometry |
| MTBE | methyl-tert-butylether |
| $NaBH_4$ | Sodium borohydride, sodium tetrahydroborate |
| $NaHCO_3$ | Sodium hydrogen carbonate |
| $Na_2SO_4$ | Sodium sulphate |
| NMR | nuclear magnetic resonance spectroscopy: chemical shifts ($\delta$) are given in ppm. The chemical shifts were corrected by setting the DMSO signal to 2.50 ppm unless otherwise stated. |
| PDA | Photo Diode Array |

(continued)

| Abbreviation | Meaning |
|---|---|
| $Pd_2dba_3$ | Tris(dibenzylideneacetone)dipalladium (0), CAS 51364-51-3 |
| $Pd(PPh_3)_4$ | Tetrakis(triphenylphosphane)palladium(0), CAS 14221-01-3 |
| quant. | quantitative |
| rac | racemic |
| $R_t$, Rt | retention time (as measured either with HPLC or UPLC) in minutes |
| RuPhos Pd G3 | (2-Dicyclohexylphosphino-2',6'-diisopropoxy-1,1'-biphenyl)[2-(2'-amino-1,1'-biphenyl)]palladium (II) methanesulfonate, CAS 1445085-77-7 |
| s | singlet ($^1$H-NMR signal) |
| SFC | Supercritical Fluid Chromatography |
| SQD | Single-Quadrupole-Detector |
| t | triplet ($^1$H-NMR signal) |
| td | triple-doublet ($^1$H-NMR signal) |
| TFA | trifluoroacetic acid |
| THF | tetrahydrofuran |
| UPLC | ultra performance liquid chromatography |
| X-Phos | 2-Dicyclohexylphosphino-2',4',6'-triisopropylbiphenyl, CAS 564483-18-7 |

The various aspects of the invention described in this application are illustrated by the following examples which are not meant to limit the invention in any way.

The example testing experiments described herein serve to illustrate the present invention and the invention is not limited to the examples given.

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage auto purifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

In the case of the synthesis intermediates and working examples of the invention described hereinafter, any compound specified in the form of a salt of the corresponding base or acid is generally a salt of unknown exact stoichiometric composition, as obtained by the respective preparation and/or purification process. Unless specified in more detail, additions to names and structural formulae, such as "hydrochloride", "trifluoroacetate", "sodium salt" or "x HCl", "x $CF_3COOH$", "x $Na^+$" should not therefore be understood in a stoichiometric sense in the case of such salts, but have merely descriptive character with regard to the salt-forming components present therein.

This applies correspondingly if synthesis intermediates or working examples or salts thereof were obtained in the form of solvates, for example hydrates, of unknown stoichiometric composition (if they are of a defined type) by the preparation and/or purification processes described.

NMR peak forms are stated as they appear in the spectra, possible higher order effects have not been consid-

ered.

The $^1$H-NMR data of selected compounds are listed in the form of $^1$H-NMR peaklists. For each signal peak the $\delta$ value in ppm is given, followed by the signal intensity, reported in round brackets. The $\delta$ value-signal intensity pairs from different peaks are separated by commas. Therefore, a peaklist is described by the general form: $\delta_1$ (intensityi), $\delta_2$ (intensity$_2$), ... , $\delta_i$ (intensity$_i$), ... , $\delta_n$ (intensity.).

The intensity of a sharp signal correlates with the height (in cm) of the signal in a printed NMR spectrum. When compared with other signals, this data can be correlated to the deal ratios of the signal intensities. In the case of broad signals, more than one peak, or the center of the signal along with their relative intensity, compared to the most intense signal displayed in the spectrum, are shown. A $^1$H-NMR peaklist is similar to a classical $^1$H-NMR readout, and thus usually contains all the peaks listed in a classical NMR interpretation. Moreover, similar to classical $^1$H-NMR printouts, peaklists can show solvent signals, signals derived from stereoisomers of target compounds (also the subject of the invention), and/or peaks of impurities. The peaks of stereoisomers, and/or peaks of impurities are typically displayed with a lower intensity compared to the peaks of the target compounds (e.g., with a purity of >90%). Such stereoisomers and/or impurities may be typical for the particular manufacturing process, and therefore their peaks may help to identify the reproduction of our manufacturing process on the basis of "by-product fingerprints". An expert who calculates the peaks of the target compounds by known methods (MestReC, ACD simulation, or by use of empirically evaluated expectation values), can isolate the peaks of target compounds as required, optionally using additional intensity filters. Such an operation would be similar to peak-picking in classical $^1$H-NMR interpretation. A detailed description of the reporting of NMR data in the form of peaklists can be found in the publication "Citation of NMR Peaklist Data within Patent Applications" (cf. Research Disclosure Database Number 605005, 2014, 01 Aug 2014, or http://www.researchdisclosure.com/searching-disclosures). In the peak picking routine, as described in the Research Disclosure Database Number 605005, the parameter "MinimumHeight" can be adjusted between 1% and 4%. Depending on the chemical structure and/or depending on the concentration of the measured compound it may be reasonable to set the parameter "MinimumHeight" <1%.

In NMR spectra of mixtures of stereoisomers, numbers mentioned with "/" indicate that the stereoisomers show separate signals for the respective hydrogen atom, i.e. ".... / ..... (2s, 1H)" means that one hydrogen atom is represented by 2 singlets, each singlet from one or more different stereoisomer(s).

IUPAC names of the following intermediates and example compounds were generated using the ACD/Name software (batch version 14.00; Advanced Chemistry Development, Inc.) or the naming tool implemented in the BIOVIA Draw software (version 4.2 SP1; Dassault Systèmes SE).

**HPLC and LC-MS methods:**

**Method 1 (LC-MS)**

MS instrument type: SHIMADZU LCMS-2020, Column: Kinetex EVO C18 30*2.1mm, 5um, mobile phase A: 0.0375% TFA in water (v/v), B: 0.01875% TFA in Acetonitrile (v/v), gradient: 0.0 min 0% B→0.8 min 95% B→1.2 min 95% B→1.21 min 5% B→1.55 min 5% B, flow rate: 1.5 mL/min, oven temperature: 50 °C; UV detection: 220 nm & 254 nm.

**Method 2 (LC-MS)**

HPLC instrument type: SHIMADZU LCMS-2020, Column: Kinetex EVO C18 50*4.6mm, 5um, mobile phase A: 0.0375% TFA in water (v/v), B: 0.01875% TFA in Acetonitrile (v/v), gradient: 0.0 min 10% B→2.4 min 80% B→3.7 min 80% B→3.71 min 10% B→4.0 min 10% B, flow rate: 1.5 mL/min, oven temperature: 50 °C; UV detection: 220 nm & 215 nm & 254 nm.

**Method 3 (LC-MS)**

Instrument: Waters ACQUITY SQD UPLC System; Column: Waters Acquity UPLC HSS T3 1.8 μm 50 x 1 mm; Eluent A: 1 1 water + 0.25 ml formic acid , Eluent B: 1 1 Acetonitrile + 0.25 ml formic acid; Gradient: 0.0 min 90% A → 1.2 min 5% A → 2.0 min 5% A; oven: 50°C; flow rate: 0.40 ml/min; UV-Detection: 210 nm.

**Method 4 (LC-MS)**

Instrument MS: Thermo Scientific FT-MS; Instrument type UHPLC+: Thermo Scientific UltiMate 3000; Column: Waters, HSST3, 2.1 x 75 mm, C18 1.8 μm; Eluent A: 11 water + 0.01% formic acid; Eluent B: 11 Acetonitrile + 0.01% formic acid; Gradient: 0.0 min 10% B → 2.5 min 95% B → 3.5 min 95% B; oven: 50°C; flow rate: 0.90 ml/min; UV-Detection: 210 nm/ Optimum Integration Path 210-300 nm.

**Method 5 (LC-MS)**

Instrument: Waters ACQUITY SQD UPLC System; Column: Waters Acquity UPLC HSS T3 1.8 μm 50 x 1 mm; Eluent A: 1 1 water + 0.25 ml formic acid, Eluent B: 1 1 Acetonitrile + 0.25 ml formic acid; Gradient: 0.0 min 95% A → 6.0 min 5% A → 7.5 min 5% A; oven: 50°C; flow rate: 0.35 ml/min; UV-Detection: 210 nm.

**Method 6 (LC-MS)**

Instrument: Agilent MS Quad 6150;HPLC: Agilent 1290; Column: Waters Acquity UPLC HSS T3 1.8 μm 50 x 2.1 mm; Eluent A: 11 water + 0.25 ml formic acid, Eluent B: 1 1 Acetonitrile + 0.25 ml formic acid; Gradient: 0.0 min 90% A ---7 0.3 min 90% A → 1.7 min 5% A ---7 3.0 min 5% A oven: 50°C; flow rate: 1,20 ml/min; UV-Detection: 205 - 305 nm.

**Method 7 (LC-MS)**

System MS: Waters TOF instrument; System UPLC: Waters Acquity I-CLASS; Column: Waters Acquity UPLC HSS T3 1.8 μm 50 x 1 mm; Eluent A: 1 1 Water + 0.100 ml 99%ige Formic acid, Eluent B: 1 1 Acetonitrile + 0.100 ml 99%ige Formic acid; Gradient: 0.0 min 90%A →1.2 min 5%A ---7 2.0 min 5% A Oven: 50°C; Flow: 0.40 ml/min; UV-Detection: 210 nm.

**Method 8 (LC-MS)**

System MS: Waters TOF instrument; System UPLC: Waters Acquity I-CLASS; Column: Waters, HSST3, 2.1 x 50 mm, C18 1.8 μm; Eluent A: 11 Water + 0.01% Formic acid; Eluent B: 1 1 Acetonitrile + 0.01% Formic acid; Gradient: 0.0 min 2% B → 0.5 min 2% B → 7.5 min 95% B → 10.0 min 95% B; Oven: 50°C; Flow: 1.00 ml/min; UV-Detection: 210 nm

**Method 9 (preparative HPLC)**

Instrument: Waters Prep LC/MS System, column: Phenomenex Kinetex C18 5μm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 55 ml/min, eluent B 15 ml/min; 2 to 10 min : eluent A from 55 ml/min to 31 ml/min, eluent B from 15 ml/min to 39 ml/min; 10 to 12 min eluent A 0 ml/min and eluent B 70 ml/min; eluent C and eluent D have a constant flow of 5 ml/min each over the whole running time.

**Method 10 (preparative HPLC)**

Instrument: Waters Prep LC/MS System, column: XBridge C18 5μm 100x30 mm, UV-detection 200-400 nm, room temperature, At-Column Injection (complete injection), eluent A : water, eluent B : acetonitrile, eluent C : 2 % ammonia in water, eluent D : acetonitrile/water (80 vol.% / 20 vol.%); flow: 80 ml/min, gradient profil: 0 to 2 min : eluent A 55 ml/min, eluent B 15 ml/min; 2 to 10 min : eluent A from 55 ml/min to 31 ml/min, eluent B from 15 ml/min to 39 ml/min; 10 to 12 min eluent A 0 ml/min and eluent B 70 ml/min; eluent C and eluent D have a constant flow of 5 ml/min each over the whole running time.

**Method 11 (preparative HPLC)**

Instrument: Waters Prep LC/MS System, column: Phenomenex Kinetex C18 5μm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 47 ml/min, eluent B 23 ml/min; 2 to 10 min : eluent A from 47 ml/min to 23 ml/min, eluent B from 23 ml/min to 47 ml/min; 10 to 12 min eluent A 0 ml/min and eluent B 70 ml/min; eluent C and eluent D have a constant flow of 5 ml/min each over the whole running time.

**Method 12 (preparative HPLC)**

Instrument: Waters Prep LC/MS System, column: Phenomenex Kinetex C18 5μm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 23 ml/min, eluent B 47 ml/min; 2 to 10 min : eluent A from 23 ml/min to 0 ml/min, eluent B from 47 ml/min

to 70 ml/min; 10 to 12 min eluent A 0 ml/min and eluent B 70 ml/min; eluent C and eluent D have a constant flow of 5 ml/min each over the whole running time.

**Method 13 (Preparative HPLC)**

Instrument: Waters Prep LC/MS System, Säule: Phenomenex Kinetex C18 5μm 100x30 mm, UV-detection 200-400 nm, room temperature, At-Column Injection (complete injection), eluent A : Water, eluent B : acetonitrile, eluent C : 2 % formic acid in Water, eluent D : acetonitrile/Water (80 vol.% / 20 vol.%) flow: 80 ml/min , gradient profil: eluent A 0 bis 2 min 70 ml/min, eluent B 0 bis 2min 0 ml/min, eluent A 2 bis 10 min von 70 ml/min to 0 ml/min and eluent B von 0 ml/min to 70 ml/min, 10 bis 12 min 0 ml/min eluent A und 70 ml/min eluent B; eluent C and eluent D have a constant flow of 5 ml/min over the whole running time.

Microwave: The microwave reactor used was an Initiator$^+$ microwave system with robot sixty from Biotage®.

When compounds according to the invention are purified by preparative HPLC by the above-described methods in which the eluents contain additives, for example trifluoroacetic acid, formic acid or ammonia, the compounds according to the invention may be obtained in salt form, for example as trifluoroacetate, formate or ammonium salt, if the compounds according to the invention contain a sufficiently basic or acidic functionality. Such a salt can be converted to the corresponding free base or acid by various methods known to the person skilled in the art.

In the case of the synthesis intermediates and working examples of the invention described hereinafter, any compound specified in the form of a salt of the corresponding base or acid is generally a salt of unknown exact stoichiometric composition, as obtained by the respective preparation and/or purification process. Unless specified in more detail, additions to names and structural formulae, such as "hydrochloride", "trifluoroacetate", "sodium salt" or "x HCl", "x $CF_3COOH$", "x $Na^+$" should not therefore be understood in a stoichiometric sense in the case of such salts, but have merely descriptive character with regard to the salt-forming components present therein.

This applies correspondingly if synthesis intermediates or working examples or salts thereof were obtained in the form of solvates, for example hydrates, of unknown stoichiometric composition (if they are of a defined type) by the preparation and/or purification processes described.

Enantiomer 1 is an enantiomer which eluted first out of the column when the preparative separation was done under separation conditions (see for example 4A).

Enantiomer 2 is an enantiomer which eluted second out of the column.

An exception is for Example 4A, where Enantiomer 1 eluated second out of the column and Enatiomer 2 eluated first out of the column.

Diastereomeric mixture 1 defines a compound where its starting material is defined as Enantiomer 1 and is reacted with a building block containaing at least one chiral center and where the configuration is not defined

Diastereomeric mixture 2 defines a compound where its starting material is defined as Enantiomer 2 and is reacted with a building block containaing at least one chiral center and where the configuration is not defined

Diastereomer 1 and Diastereomer 2 defines the two compounds resulting from the chiral separation of the *diastereomeric mixture 1* described above.

Diastereomer 3 and Diastereomer 4 defines the two compounds resulting from the chiral separation of the *diastereomeric mixture 2* described above.

Stereoisomer 1 defines a compound where its starting material is defined as Enantiomer 1 and is reacted with a building block containaing at least one chiral center and where the configuration is defined

Stereoisomer 2 defines a compound where its starting material is defined as Enantiomer 2 and is reacted with a building block containaing at least one chiral center and where the configuration is defined

**EXPERIMENTAL SECTION - STARTING MATERIALS AND INTERMEDIATES**

**Example 1A**

Tert-butyl 3-{2-[(benzyloxy)carbonyl]hydrazino}piperidine-1-carboxylate (Racemate)

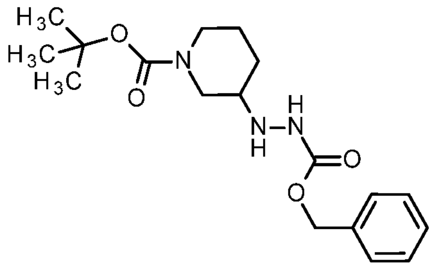

A solution of tert-butyl 3-oxopiperidine-1-carboxylate [CAS No. 989-36-7] (300 g, 1.51 mol) in THF (1.50 L) and methanol (300 mL) was treated with benzyl hydrazinecarboxylate [CAS No. 5331-43-1] (250 g, 1.51 mol) and stirred 1 hour at 25°C. Afterwards sodium borohydride (114 g, 3.01 mol) was added in portions to the reaction mixture and the resulting mixture was stirred 2 hours at 25°C. The reaction mixture was cooled to 10°C and adjusted to pH=-6 by dropwise addition of a saturated solution of of ammonium chloride. The aqueous phase was extracted with ethyl acetate (300 mL x 2) and the combined organic layers evaporated. The residue was dissolved in MTBE (300 mL) and petroleum ether (300 mL) was added to the solution. The resulting suspension was filtered off and the precipitate washed with petroleum ether (100 mL) affording 400 g (76.0 % yield) of the title compound as a white solid.

LC-MS: (Method 1) Rt = 0.832 min, MS (M-100+1 = 250.4).

## Example 2A

Tert-butyl 3-hydrazinopiperidine-1-carboxylate acetic acid (Racemate)

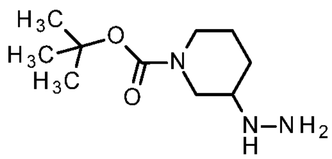

x $CH_3COOH$

To a solution of tert-butyl 3-{2-[(benzyloxy)carbonyl]hydrazino}piperidine-1-carboxylate (prepared in analogy to Example 1A, 1.20 kg, 3.43 mol) in ethanol (11.0 L) and acetic acid (415 g, 6.91 mol, 395 mL) was added Pd/C (120 g, 20% purity). The resulting suspension was stirred 12 hours at 25 °C under hydrogen atmosphere (15 Psi). The mixture was filtered and the precipitate was washed with ethanol (11.0 L) to give a solution of the title compound in ethanol (945 g) as a black liquid, which was used in the next step without further purification.

$^1$H-NMR (400 MHz, $CDCl_3$) $\delta$ [ppm]: 7.52 (s, 5H), 3.59 (d, $J$ = 6.0 Hz, 12H), 3.30 - 3.24 (m, 2H), 2.75 - 2.71 (m, 2H), 1.38 - 1.34 (m, 1H), 1.20 - 1.18 (m, 1H), 1.10 (s, 9H)

LC-MS: (Method 1) Rt = 0.263 min, MS (M-56+1 = 160.2)

## Example 3A

Tert-butyl 3-[4-(ethoxycarbonyl)-5-(trifluoromethyl)- 1H-pyrazol-1-yl]piperidine-1-carboxylate (Racemate)

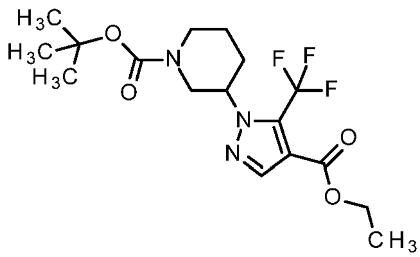

Tert-butyl 3-hydrazinopiperidine-1-carboxylate acetic acid (Example 2A, 945 g, 3.43 mol) in ethanol (20 L) was treated with ethyl 2-(ethoxymethylene)-4,4,4-trifluoro-3-oxobutanoate (907 g, 3.78 mol). The resulting mixture was stirred 16 hours at 25°C, diluted with a saturated solution of sodium hydrogencarbonate (2.0 L) and concentrated to ~ 5.0 L. The resulting mixture was diluted with water (5.0 L) and extracted with ethyl acetate (5.0 L). The organic phase was washed with a saturated solution of sodium chloride (5.0 L) and evaporated. The residue was purified by flash chromatography (silica gel, petroleum ether/ethyl acetate, 10:1) affording 548 g (41% yield) of the title compound.
$^1$H-NMR (400 MHz, $CDCl_3$) δ [ppm]: 7.90 (s, 1H), 4.33 - 3.09 (m, 5H), 3.26 - 3.12 (m, 1H), 2.89 - 2.61 (m, 1H), 2.35 - 2.05 (m, 2H), 1.98 - 1.78 (m, 1H), 1.71 - 1.51 (m, 1H), 1.50 - 1.37 (m, 9H), 1.32 (m, 3H)

## Example 4A

Ethyl 1-(piperidin-3-yl)-5-(trifluoromethyl)-1H-pyrazole-4-carboxylate (Racemate)

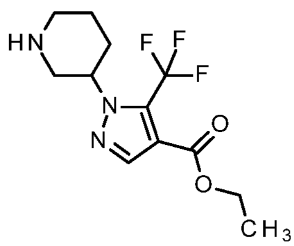

Tert-butyl 3-[4-(ethoxycarbonyl)-5-(trifluoromethyl)-1H-pyrazol-1-yl]piperidine-1-carboxylate (Example 3A, 548 g, 1.40 mol) was was treated with a solution of hydrogen chloride in dioxane (4 M, 2.38 L), stirred 2 hours at 25°C and evaporated. The residue was retaken in 1.0 L water and extracted with MTBE (500 mL x 1). The aqueous phase was separated and adjusted to pH to 8 - 9 with a saturated solution of sodium hydrogencarbonate. The aqueous phase was extracted with dichloromethane (1.0 L x 2), and the combined organic layers were washed with a saturated solution of sodium chloride (1 L), dried over sodium sulfate and evaporated affording 325 g (80% yield) of the title compound.
LC-MS: (Method 1) Rt = 0.955 min, MS (M +1) = 299.2.
The two enantiomers were separated by SFC [325 g, column: Phenomenex-Cellulose-2 (250mm*50mm, 10μm); eluent: $CO_2$/(methanol + 0.1% aqueous ammonia); 75:25, 4.5 min; 1400 min] affording 103.0 g of enantiomer 1 (Example 5A) and 110.1 g of enantiomer 2 (Example 6A).

## Example 5A

Ethyl 1-(piperidin-3-yl)-5-(trifluoromethyl)-1H-pyrazole-4-carboxylate (Enantiomer 1)

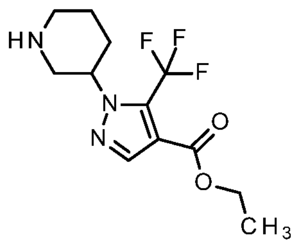

For separation conditions see Example 4A.

Analytical SFC: $R_t$ = 1.345 min, e.e. = 99% [Column Cellulose 2-3: 50 x 4.6 mm; eluent: $CO_2$/[methanol + 0.5% diethyl amine]: 95:5 to 60:40 flow rate: 3.0 ml/min; temperature: 35°C; UV detection: 220 nm, back pressure 100 bar].

LCMS (Method 2), $R_t$ = 0.906 min, MS (M +1) = 292.1.

$^1$H-NMR (400 MHz, $CDCl_3$) $\delta$ [ppm]: 7.89 (s, 1H), 4.50 - 4.47 (m, 1H), 4.31 - 4.25 (m, 2H), 3.24 - 3.05 (m, 4H), 2.70 - 2.67 (m, 1H), 2.10 - 2.02 (m, 2H), 1.92 - 1.79 (m, 1H), 1.74 - 1.56 (m, 1H), 1.31 (t, *J* = 7.2 Hz, 3H).

**Example 6A**

Ethyl 1-(piperidin-3-yl)-5-(trifluoromethyl)-1H-pyrazole-4-carboxylate (Enantiomer 2)

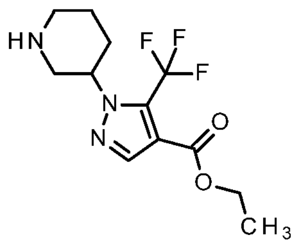

For separation conditions see Example 4A.

Analytical SFC: $R_t$ = 1.071 min, e.e. = 99% [Column Cellulose 2-3: 50 x 4.6 mm; eluent: $CO_2$/[methanol + 0.5% diethyl amine]: 95:5 to 60:40 flow rate: 3.0 ml/min; temperature: 35°C; UV detection: 220 nm, back pressure 100 bar].

LCMS (Method 2), $R_t$ = 0.906 min, MS (M +1) = 292.1.

$^1$H-NMR (400 MHz, $CDCl_3$) $\delta$ [ppm]: 7.91 (s, 1H), 4.58 - 4.41 (m, 1H), 4.35 - 4.23 (m, 2H), 3.70 - 3.56 (m, 1H), 3.31 - 3.12 (m, 2H), 3.11 - 3.02 (m, 1H), 2.75 - 2.62 (m, 1H), 2.15 - 2.02 (m, 2H), 1.92 - 1.79 (m, 1H), 1.74 - 1.56 (m, 1H), 1.33 (t, *J* = 7.2 Hz, 3H).

**Example 7A**

2-Bromo-4-chloro-1-[(4-methoxyphenyl)methoxy]benzene

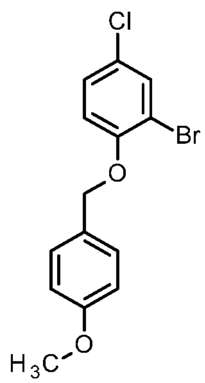

A solution of 2-bromo-4-chlorophenol [CAS No. 695-96-5] (10.0 g, 48.2 mmol) in acetone (75 ml) was treated with potassium carbonate (13.3 g, 96.4 mmol) and potassium iodide (12.0 g, 72.3 mmol) and 1-(chloromethyl)-4-methoxybenzene (7.55 g, 48.2 mmol). The resulting mixture was stirred 19 hours at 70°C. The reaction mixture was diluted with water and extracted twice with ethyl acetate. The combined organic layers were dried over sodium sulphate and evaporated. The residue was purified by flash chromatography (silica gel, cyclohexane/ethyl acetate gradient) affording 13.8 g (86 % yield) of the title compound.
LC-MS (Method 4): $R_t$ = 2.48 min; MS (ESIneg): m/z = 324 $[M-H]^-$

$^1$H-NMR (600 MHz, DMSO-d6) δ [ppm]: 3.349 (10.98), 5.124 (16.00), 6.949 (0.87), 6.954 (8.36), 6.957 (2.68), 6.965 (2.83), 6.968 (8.92), 6.973 (1.00), 7.218 (5.23), 7.233 (6.21), 7.380 (0.90), 7.384 (7.80), 7.399 (7.44), 7.402 (4.47), 7.406 (3.89), 7.417 (3.04), 7.421 (3.07), 7.697 (6.51), 7.702 (6.34).

**Example 8A**

Ethyl 1-[1-{5-chloro-2-[(4-methoxyphenyl)methoxy]phenyl}piperidin-3-yl]-5-(trifluoromethyl)- 1H-pyrazole-4-carboxylate (Enantiomer 1)

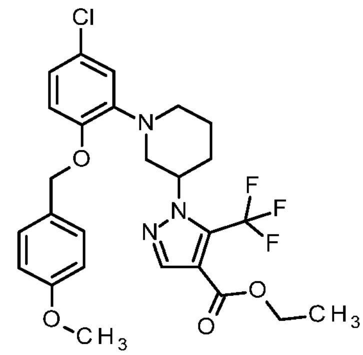

Under argon, a solutionfethyl 1-[piperidin-3-yl]-5-(trifluoromethyl)-1H-pyrazole-4-carboxylate (prepared in analogy to Example 5A, Enantiomer 1, 75.0 g, 257 mmol) and 2-bromo-4-chloro-1-[(4-methoxyphenyl)methoxy]benzene (prepared in analogy to Example 7A, 84.4 g, 257 mmol) in 1,4-dioxane (1.11) was treated with $Pd_2dba_3$ (23.6 g, 25.7 mmol), rac-BINAP (32.1 g, 51.5 mmol) and cesium carbonate (252 g, 772 mmol). The resulting mixture was stirred 3 days at 100°C and cooled to room temperature. The reaction mixture was diluted with an aqueous solution of sodium chloride (10 %) and ethyl acetate, filtered over celite and rinsed with ethyl acetate. The aqueous phase of the filtrate was separated and extracted with ethyl acetate. The combined organic layers were washed with an aqueous solution of sodium chloride (10 %), dried over sodium sulfate and evaporated. The residue was purified by flash chromatography (silica gel, dichloromethane/petrol ether gradient) affording 119 g (71 % yield) of the title compound.
LC-MS (Method 4): $R_t$ = 2.81 min; MS (ESIpos): m/z = 538 $[M+H]^+$

**Example 9A**

Ethyl 1-[1-(5-chloro-2-hydroxyphenyl)piperidin-3-yl]-5-(trifluoromethyl)-1H-pyrazole-4-carboxylate (Enantiomer 1)

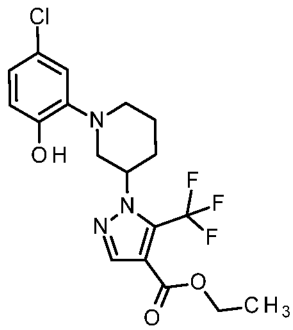

A solution of ethyl 1-[1-{5-chloro-2-[(4-methoxyphenyl)methoxy]phenyl}piperidin-3-yl]-5-(triffuoromethyl)-1H-pyrazole-4-carboxylate (Example 8A, Enantiomer 1, 119 g, 221 mmol) in dichloromethane (1.8 1) was treated with trifluoroacetic acid (170 ml, 2.2 mol) and the resulting mixture was stirred 3 days at room temperature. Thre reaction mixture was quenched carefully with an aqueous solution of sodium hydrogenocarbonate (10 %) until pH = 8. The phases were separated. The organic layer was evaporated and the residue was purified by flash chromatography (silica gel, dichloromethane / petrol ether gradient) affording 85 g (90 % purity, 92 % yield) of the title compound.
LC-MS (Method 4): $R_t$ = 2.47 min; MS (ESIpos): m/z = 418 $[M+H]^+$

## Example 10A

Ethyl 1-[1-{5-chloro-2-[(trifluoromethanesulfonyl)oxy]phenyl}piperidin-3-yl]-5-(trifluoromethyl)-1H-pyrazole-4-carboxylate (Enantiomer 1)

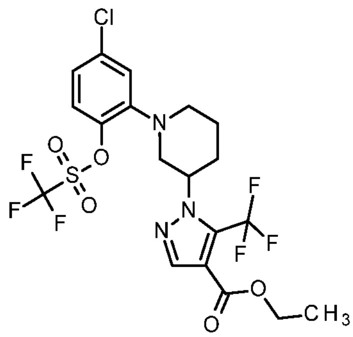

Under argon, a solution of ethyl 1-[1-(5-chloro-2-hydroxyphenyl)piperidin-3-yl]-5-(trifluoromethyl)-1H-pyrazole-4-carboxylate (Example 9A, Enantiomer 1, 85.0 g, 90 % purity, 184 mmol) in dichloromethane (520 ml) was cooled to -50°C and treated with triethylamine (77 ml, 550 mmol). Trifluoromethanesulfonic anhydride (43 ml, 260 mmol) was added dropwise to the reaction mixture and the resulting solution was stirred 1 hour at -50°C. The reaction mixture was diluted with dichloromethane (520 ml) and ice-cooled water (590 ml). The aqueous layer was extracted with dichloromethane (520 ml). The combined organice layers were washed once with ice-cooled water (590 ml), dried over sodium sulfate and evaporated. The residue was purified by flash chromatography (silica gel, dichloromethane/petrol ether gradient) affording 94 g (93 % yield) of the title compound.
LC-MS (Method 4): $R_t$ = 2.79 min; MS (ESIpos): m/z = 550 $[M+H]^+$
$^1$H-NMR (400 MHz, DMSO-d6) δ [ppm]: 1.259 (7.63), 1.271 (16.00), 1.282 (7.94), 1.771 (0.45), 1.779 (0.80), 1.786 (0.61), 1.793 (0.61), 1.800 (0.94), 1.807 (0.61), 1.821 (0.45), 1.932 (1.22), 1.955 (0.96), 2.099 (0.77), 2.106 (0.73), 2.120 (1.04), 2.126 (1.33), 2.138 (1.91), 2.820 (0.73), 2.825 (0.87), 2.841 (1.56), 2.845 (1.61), 2.861 (0.93), 2.865 (0.82), 3.140 (1.18), 3.159 (1.09), 3.186 (1.39), 3.204 (2.87), 3.222 (1.78), 3.318 (1.51), 3.324 (1.60), 3.336 (1.08), 3.342 (1.04), 4.247 (2.31), 4.259 (7.26), 4.270 (7.27), 4.282 (2.41), 4.669 (0.70), 4.679 (0.84), 4.686 (1.34), 4.694 (0.96), 4.704 (0.72), 4.711 (0.42), 7.286 (2.29), 7.290 (2.44), 7.300 (2.89), 7.304 (3.11), 7.415 (5.01), 7.430 (4.13), 7.457 (5.11), 7.461 (5.05), 8.123 (6.61).

**Example 11A**

Tert-butyl 4-(4'-chloro-2'-{3-[4-(ethoxycarbonyl)-5-(trifluoromethyl)-1H-pyrazol-1-yl]piperidin-1-y1}[1,1'-biphenyl]-4-yl)piperazine-1-carboxylate (Enantiomer 1)

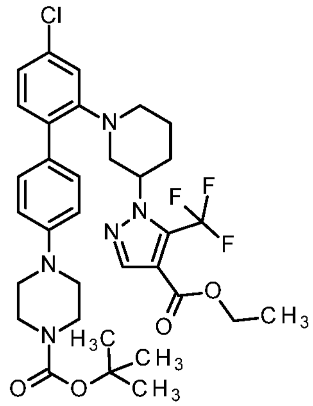

Under argon, a solution of ethyl 1-[1-{5-chloro-2-[(trifluoromethanesulfonyl)oxy]phenyl}piperidin-3-yl]-5-(trifluoromethyl)-1H-pyrazole-4-carboxylate (Example 10A, Enantiomer 1, 92.1 g, 167 mmol) and tert-butyl 4-[4-(4,4,5,5-tetramethyl-1,3,2-dioxaborolan-2-yl)phenyl]piperazine-1-carboxylate (78.0 g, 201 mmol) in toluene (840 ml) and ethanol (840 ml) was treatd with an aqueous solution of sodium carbonate (250 ml, 2.0 M, 500 mmol) and $Pd(PPh_3)_4$ (9.68 g, 8.37 mmol) and the resulting mixture was stirred overnight at 100°C. The reaction mixture was cooled to room temperature, filtered over celite, rinsed with ethyl acetate and evaporated. The residue was purified by flash chromatography (silica gel, petrol ether/ ethyl acetate gradient) affording 94 g (85 % yield) of the title compound.

LC-MS (Method 4): $R_t$ = 3.19 min; MS (ESIpos): m/z = 662 $[M+H]^+$ $^1$H-NMR (400 MHz, DMSO-d6) δ [ppm]: -0.008 (0.66), 0.008 (0.84), 1.038 (0.55), 1.088 (0.76), 1.232 (1.60), 1.250 (3.49), 1.268 (1.69), 1.419 (0.77), 1.431 (16.00), 1.989 (0.77), 2.957 (0.43), 3.127 (0.91), 3.140 (1.32), 3.152 (1.09), 3.457 (0.99), 3.470 (1.25), 3.481 (0.88), 4.211 (0.45), 4.228 (1.43), 4.246 (1.38), 4.264 (0.43), 6.985 (1.10), 7.007 (1.20), 7.068 (0.79), 7.073 (1.06), 7.089 (0.49), 7.109 (0.80), 7.114 (0.69), 7.146 (1.34), 7.166 (0.65), 7.433 (1.33), 7.455 (1.19), 8.062 (1.56).

**Example 12A**

Ethyl 1-{1-[4-chloro-4'-(piperazin-1-yl)[1,1'-biphenyl]-2-yl]piperidin-3-yl}-5 -(trifluoromethyl)- 1H-pyrazole-4-carboxylate hydrochloride (Enantiomer 1)

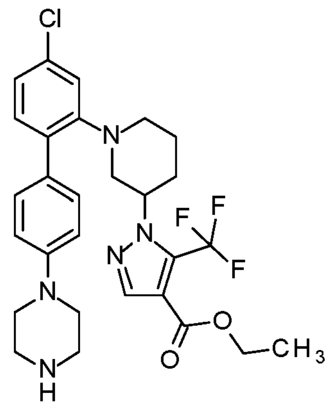

x HCl

A solution of tert-butyl 4-(4'-chloro-2'-{3-[4-(ethoxycarbonyl)-5-(trifluoromethyl)-1H-pyrazol-1-yl]piperidin-1-yl}[1,1'-biphenyl]-4-yl)piperazine-1-carboxylate (Example 11A, Enantiomer 1, 93.0 g, 140 mmol) in dichloromethane (290 ml) was treated with a solution of hydrogen chloride in dioxane (350 ml, 4.0 M, 1.4 mol) and stirred 3 hours at room temperature. The reaction mixture was evaporated and the residue co-evaporated with MTBE affording 95 g (quant.) of the title compound which was used in the next step without further purification.
LC-MS (Method 4): $R_t$ = 1.97 min; MS (ESIpos): m/z = 562 $[M+H]^+$

**Example 13A**

Ethyl 1-[1-{4-chloro-4'-[4-(2-methylpropyl)piperazin-1-yl][1,1'-biphenyl]-2-yl}piperidin-3-yl]-5-(trifluoromethyl)-1H-pyrazole-4-carboxylate (Enantiomer 1)

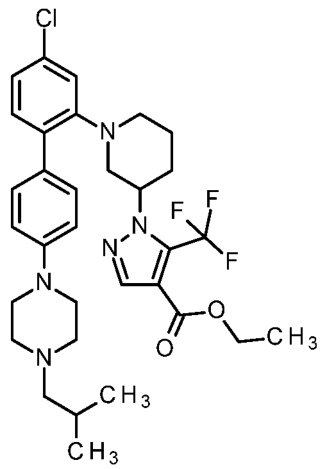

A solution of ethyl 1-{1-[4-chloro-4'-(piperazin-1-yl)[1,1'-biphenyl]-2-yl]piperidin-3-yl}-5-(trifluoromethyl)-1H-pyrazole-4-carboxylate hydrochloride (Example 12A, Enantiomer 1, 95.0 g, 150 mmol) in THF (1.8 1) was treated with N,N-diisopropylethylamine (100 ml, 600 mmol) and 2-methylpropanal [CAS No. 78-84-2] (53.9 g, 748 mmol) and stirred 1 hour at room temperature. Sodium triacetoxyborohydride (127 g, 598 mmol]) was added and the resulting mixture was stirred 18 hours at room temperature. The reaction mixture was diluted with an aqueous solution of sodium hydrogen carbonate (10%) and extracted three times with ethyl acetate. The combined organic layers were washed with a saturated solution of sodium chloride, dried over sodium sulphate and evaporated. The residue was purified by flash chromatography (silica gel, petrol ether/ethyl acetate gradient) affording 78 g (84 % yield) of the title compound.
LC-MS (Method 4): $R_t$ = 2.03 min; MS (ESIpos): m/z = 618 $[M+H]^+$

$^1$H-NMR (600 MHz, DMSO-d6) $\delta$ [ppm]: 0.827 (0.53), 0.839 (0.55), 0.867 (0.66), 0.871 (0.81), 0.883 (15.73), 0.894 (16.00), 1.041 (0.92), 1.090 (1.35), 1.241 (4.62), 1.252 (9.48), 1.264 (4.77), 1.554 (0.58), 1.575 (0.64), 1.753 (0.80), 1.775 (0.81), 1.786 (0.63), 1.798 (0.90), 1.809 (1.08), 1.820 (0.88), 1.831 (0.48), 1.889 (0.64), 1.895 (0.58), 1.909 (0.66), 1.916 (0.63), 1.988 (0.94), 1.998 (0.79), 2.015 (0.59), 2.085 (4.57), 2.097 (4.16), 2.467 (3.65), 2.476 (5.06), 2.483 (4.05), 2.595 (0.61), 2.612 (1.08), 2.615 (1.10), 2.631 (0.59), 2.937 (0.86), 2.955 (1.65), 2.972 (0.98), 3.073 (0.84), 3.093 (0.80), 3.156 (3.65), 3.164 (4.72), 3.172 (3.66), 3.212 (0.97), 3.227 (0.83), 4.221 (1.39), 4.233 (4.19), 4.245 (4.15), 4.256 (1.41), 4.362 (0.49), 4.379 (0.85), 4.397 (0.49), 6.949 (3.70), 6.963 (3.93), 7.060 (2.66), 7.063 (3.31), 7.082 (1.52), 7.085 (1.15), 7.095 (2.12), 7.099 (1.92), 7.141 (3.61), 7.154 (2.29), 7.411 (4.15), 7.426 (3.95), 8.049 (4.51).

**EXPERIMENTAL SECTION - EXAMPLE COMPOUNDS**

**Example 1**

1-[1-{4-Chloro-4'-[4-(2-methylpropyl)piperazin-1-yl][1,1'-biphenyl]-2-yl}piperidin-3-yl]-5-(trifluoromethyl)-1H-pyrazole-4-carboxylic acid (Enantiomer 1)

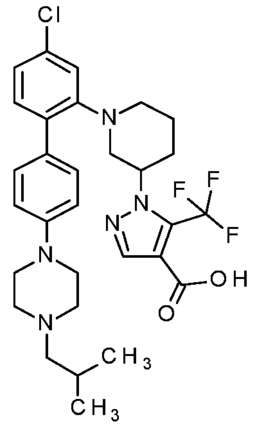

An aqueous solution of lithium hydroxide (1.2 1, 1.0 M, 1.2 mol) was added to a solution of ethyl 1-[1-{4-chloro-4'-[4-(2-methylpropyl)piperazin-1-yl][1,1'-biphenyl]-2-yl}piperidin-3-yl]-5-(trifluoromethyl)-1H-pyrazole-4-carboxylate (Example 13A, Enantiomer 1, 77.0 g, 125 mmol) in a THF/methanol mixture (9:1) (1.5 1). The resulting mixture was stirred overnight at room temperature and acidified to pH~2 with an aqueous solution of hydrogen chloride (2N). The reaction mixture was diluted with dichloromethane. The organic layer was washed with water and evaporated affording 74 g (quant.) of the title compound which was used in the next step without further purification.

LC-MS (Method 4): $R_t$ = 1.74 min; MS (ESIpos): m/z = 590 $[M+H]^+$

$^1$H-NMR (600 MHz, DMSO-d6) $\delta$ [ppm]: 0.830 (0.48), 0.841 (0.49), 1.009 (16.00), 1.020 (16.00), 1.045 (0.89), 1.094 (1.29), 1.187 (0.45), 1.363 (0.58), 1.528 (0.64), 1.549 (0.68), 1.750 (2.54), 1.755 (2.82), 1.760 (5.81), 1.766 (2.92), 1.771 (2.27), 1.919 (0.75), 1.926 (0.61), 1.940 (0.72), 1.946 (0.67), 2.003 (0.92), 2.019 (0.59), 2.105 (0.70), 2.117 (0.84), 2.128 (0.67), 2.579 (0.64), 2.863 (1.29), 2.981 (1.01), 2.998 (1.83), 3.016 (1.09), 3.051 (1.01), 3.069 (0.93), 3.216 (1.08), 3.238 (1.70), 3.256 (1.35), 3.573 (0.51), 3.594 (2.25), 3.604 (4.74), 3.615 (1.96), 4.383 (0.57), 4.394 (0.64), 4.400 (0.96), 4.407 (0.64), 4.418 (0.51), 7.033 (4.04), 7.048 (4.15), 7.082 (3.12), 7.085 (3.80), 7.099 (1.74), 7.102 (1.19), 7.113 (2.42), 7.116 (2.08), 7.155 (4.12), 7.168 (2.53), 7.473 (4.69), 7.488 (4.22), 8.020 (5.33).

## Example 2

1-[1-{4-chloro-4'-[4-(2-methylpropyl)piperazin-1-yl][1,1'-biphenyl]-2-yl}piperidin-3-yl]-5-(trifluoromethyl)-1H-pyrazole-4-carboxylic acid hydrochloride (Enantiomer 1)

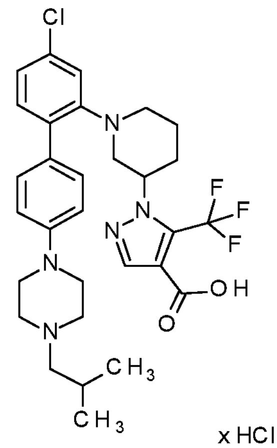

x HCl

## Method A

A solution of 1-[1-{4-chloro-4'-[4-(2-methylpropyl)piperazin-1-yl][1,1'-biphenyl]-2-yl}piperidin-3-yl]-5-(trifluoromethyl)-1H-pyrazole-4-carboxylic acid (Example 1, Enantiomer 1, 78.0 g, 132 mmol) in diethyl ether (1.51) was treated with a solution of hydrogen chloride in diethyl ether (150 ml, 150 mmol). The resulting mixture was stirred overnight at

room temperature and evaporated affording 82 g (quant.) of the title compound.
LC-MS (Method 4): $R_t$ = 1.77 min; MS (ESIpos): m/z = 590 [M-HCl+H]$^+$

$^1$H-NMR (400 MHz, DMSO-d6) δ [ppm]: -0.839 (0.40), 1.013 (0.67), 1.029 (15.68), 1.039 (16.00), 1.057 (0.67), 1.081 (1.77), 1.092 (3.56), 1.104 (1.68), 1.360 (0.54), 1.520 (0.54), 1.540 (0.60), 1.741 (0.73), 1.750 (0.52), 1.761 (0.83), 1.921 (0.56), 1.927 (0.52), 1.941 (0.61), 1.947 (0.58), 2.004 (0.77), 2.020 (0.52), 2.147 (0.46), 2.158 (0.88), 2.169 (1.10), 2.180 (0.89), 2.192 (0.47), 2.578 (0.57), 2.984 (0.91), 2.995 (1.90), 3.004 (3.37), 3.016 (1.95), 3.044 (0.88), 3.062 (0.77), 3.119 (0.77), 3.125 (0.78), 3.135 (1.02), 3.145 (0.85), 3.151 (0.86), 3.244 (0.80), 3.258 (0.71), 3.361 (1.04), 3.368 (0.87), 3.380 (3.28), 3.391 (1.89), 3.403 (1.41), 3.570 (1.41), 3.589 (1.33), 3.603 (0.52), 3.785 (0.79), 3.814 (0.99), 3.838 (0.75), 4.383 (0.46), 4.394 (0.53), 4.400 (0.79), 4.407 (0.55), 4.418 (0.46), 7.055 (3.42), 7.070 (3.66), 7.085 (2.44), 7.089 (3.02), 7.105 (1.54), 7.108 (1.13), 7.118 (2.11), 7.121 (1.92), 7.157 (3.63), 7.171 (2.19), 7.485 (3.98), 7.500 (3.68), 8.023 (4.23), 10.650 (0.49).

**Method B**

Ethyl 1-[1-{4-chloro-4'-[4-(2-methylpropyl)piperazin-1-yl][1,1'-biphenyl]-2-yl}piperidin-3-yl]-5-(trifluoromethyl)-1H-pyrazole-4-carboxylate (prepared in analogy to Example 13A, Enantiomer 1, 149 mg, 241 μmol) was dissolved in THF/ethanol (6.3/0.63 ml). 1 M aqueous lithium hydroxide solution (2.4 ml, 2.4 mmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. Then the residue was dissolved in acetonitrile, mixed with 0.1 M hydrochloric acid in dioxane, carefully evaporated at 30°C (thrice) and then lyophilized. 130 mg of the target compound (85% of theory) were obtained.
LC-MS (Method 4): $R_t$ = 1.81 min; MS (ESIpos): m/z = 590 [M-HCl+H]$^+$

$^1$H-NMR (600 MHz, DMSO-d6) δ [ppm]: 1.014 (15.69), 1.025 (16.00), 1.522 (0.64), 1.543 (0.70), 1.747 (0.89), 1.769 (0.75), 1.916 (0.67), 1.935 (0.74), 2.003 (0.95), 2.020 (0.62), 2.133 (0.48), 2.144 (0.96), 2.155 (1.17), 2.166 (0.94), 2.177 (0.49), 2.588 (0.65), 2.605 (1.20), 2.624 (0.66), 2.968 (0.93), 2.986 (1.83), 3.006 (2.63), 3.017 (3.24), 3.027 (1.95), 3.052 (1.01), 3.070 (0.94), 3.115 (0.97), 3.133 (1.36), 3.148 (1.12), 3.230 (1.08), 3.251 (1.98), 3.273 (2.05), 3.292 (1.01), 3.578 (1.97), 3.597 (1.82), 3.800 (1.79), 3.823 (2.21), 3.841 (2.82), 4.367 (0.59), 4.385 (1.02), 4.403 (0.56), 7.051 (3.88), 7.065 (4.03), 7.092 (3.43), 7.110 (1.41), 7.123 (2.32), 7.155 (3.64), 7.169 (1.99), 7.486 (4.35), 7.501 (3.97), 8.028 (4.91), 10.135 (0.55).

$[\alpha]_D^{20}$ = -68.23°, c = 0.49 g/100 cm$^3$, trichloromethane.

**Comparative Example 174 (WO2012/058132)**

1-{1-[4-Chloro-4'-(4-cyclopropylmethylpiperazin-1-yl)[biphenyl]-2-yl]pyridin-3-yl}-5-(trifluoromethyl)-1H-pyrazole-4-carboxylic acid

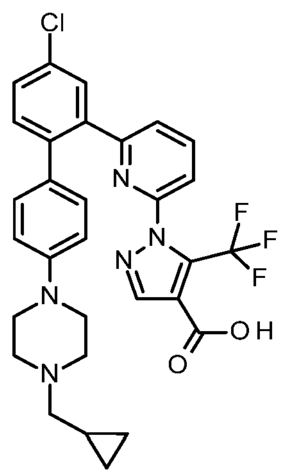

The compound was synthesized according to the procedures disclosed in WO 2012/058132 (experimental part, pages 58 to 84).

## B. Assessment of pharmacological efficacy and pharmacokinetic profile

The following abbreviations are used:

| | |
|---|---|
| ATP | adenosine triphosphate |
| Brij35 | polyoxyethylene(23) lauryl ether |
| BSA | bovine serum albumin: |
| DTT | dithiothreitol |
| TEA | triethanolamine |

## Biological investigations

The example testing experiments described herein serve to illustrate the present invention and the invention is not limited to the examples given.
The following assays can be used to illustrate the commercial utility of the compounds according to the present invention.
Examples were tested in selected biological assays one or more times. When tested more than once, data are reported as either average values or as median values, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values calculated utilizing data sets obtained from testing of one or more synthetic batch.
The *in vitro* activity of the compounds of the present invention can be demonstrated in the following assays.
The pharmacological action of the compounds of the invention can be demonstrated in the following assays:

### B-1. Effect on a recombinant guanylate cyclase reporter cell line

The cellular activity of the compounds according to the invention was determined using a recombinant guanylate cyclase reporter cell line, as described in F. Wunder et al., Anal. Biochem. 339, 104-112 (2005).
Representative MEC values (MEC = minimum effective concentration) and EC50 values (half maximal effective concentration) for the compounds of the invention are shown in the table below (in some cases as mean values from individual determinations):

Table 2:

| Example | MEC [nM] | $EC_{50}$ [nM] |
|---|---|---|
| 1 | 6.5 | 40 |
| 2 | 2.2 | 11.0 |

### B-2. Vasorelaxant effect *in vitro*

Rabbits were killed in deep anaesthesia and exsanguinated. The aorta was removed, freed from adhering tissue and divided into rings of width 1.5 mm, which were placed individually under prestress into 5 ml organ baths with carbogen-sparged Krebs-Henseleit solution at 37°C having the following composition (each in mM): sodium chloride: 119; potassium chloride: 4.8; calcium chloride dihydrate: 1; magnesium sulfate heptahydrate: 1.4; potassium dihydrogenphosphate: 1.2; sodium bicarbonate: 25; glucose: 10. To generate a contraction, phenylephrine was added to the bath cumulatively in increasing concentration. After several control cycles, the substance to be studied was added in

increasing dosage each time in every further run, and the magnitude of the contraction was compared with the magnitude of the contraction attained in the last preceding run. This was used to calculate the concentration needed to reduce the magnitude of the control value by 50% ($IC_{50}$ value). The standard administration volume was 5 μl; the DMSO content in the bath solution corresponds to 0.1%.

**B-3. Blood pressure measurement on anaesthetized rats**

Male Wistar rats having a body weight of 300-350 g were anaesthetized with thiopental (100 mg/kg i.p.). After tracheotomy, a catheter was introduced into the femoral artery to measure the blood pressure. The substances to be tested were administered as solutions, either orally by means of a gavage or intravenously via the femoral vein (Stasch et al. Br. J. Pharmacol. 2002; 135: 344-355).

**B-4. Radiotelemetry measurement of blood pressure in conscious, spontaneously hypertensive rats**

A commercially available telemetry system from DATA SCIENCES INTERNATIONAL DSI, USA, was employed for the blood pressure measurement on conscious rats described below.
The system consists of 3 main components:

implantable transmitters (Physiotel® telemetry transmitter)

receivers (Physiotel® receiver) which were linked via a multiplexer (DSI Data Exchange Matrix 2.0) to a data acquisition computer.

The telemetry system makes it possible to continuously record blood pressure, heart rate and body motion of conscious animals in their usual habitat.

Animal material

The studies were conducted on adult female spontaneously hypertensive rats (SHR Okamoto) with a body weight of > 200 g. SHR/NCrl from the Okamoto Kyoto School of Medicine, 1963, were a cross of male Wistar Kyoto rats having greatly elevated blood pressure and female rats having slightly elevated blood pressure, and were handed over at F13 to the U.S. National Institutes of Health.
After transmitter implantation, the experimental animals were housed singly in type 3 Makrolon cages. They had free access to standard feed and water.
The day/night rhythm in the experimental laboratory was changed by the room lighting at 6:00 am and at 7:00 pm.

Transmitter implantation

The HD S 10 telemetry transmitters used were surgically implanted under aseptic conditions in the experimental animals at least 14 days before the first experimental use. The animals instrumented in this way can be used repeatedly after the wound has healed and the implant has settled.
For the implantation, the fasted animals were anesthetized with isoflurane (Rimadyl analgesia) and shaved and disinfected over a large area of their abdomens. After the abdominal cavity had been opened along the linea alba, the liquid-filled measuring catheter of the system was inserted into the descending aorta in the cranial direction above the bifurcation and fixed with tissue glue (VetBonD TM, 3M). The transmitter housing was fixed intraperitoneally to the abdominal wall muscle, and the wound was closed layer by layer.
An antibiotic (Ursocyclin 10% pro inj., Serumwerk, s.c.) was administered postoperatively for prophylaxis of infection.

Substances and solutions

Unless stated otherwise, the substances to be studied were administered orally by gavage to a group of animals in each case (n = 6). In accordance with an administration volume of 2ml/kg of body weight, the test substances were dissolved in suitable solvent mixtures or suspended in 0.5% tylose.
A solvent-treated group of animals was used as control.

Experimental procedure

The telemetry measuring unit present was configured for 24 animals. Each experiment was recorded under an experiment number (Vyear month day).

Each of the instrumented rats living in the system was assigned a separate receiving antenna (RPC-1 Receiver, DSI).

The implanted transmitters can be activated externally by means of an incorporated magnetic switch. They were switched to transmission in the run-up to the experiment. The signals emitted can be detected online by a data acquisition system (Physio Tel HD, DSI) and processed accordingly. The data were stored in each case in a file created for this purpose and bearing the experiment number.

In the standard procedure, the following were measured for 10-second periods in each case:

systolic blood pressure (SBP)

diastolic blood pressure (DBP)

mean arterial pressure (MAP)

heart rate (HR)

activity (TEMP).

The acquisition of measurements was repeated under computer control at 5-minute intervals. The source data obtained as absolute values were corrected in the diagram with the currently measured barometric pressure (Ambient Pressure Reference Monitor; APR-1) and stored as individual data. Further technical details were given in the extensive documentation from the manufacturer company (DSI).

Unless indicated otherwise, the test substances were administered at 9:00 am on the day of the experiment. Following the administration, the parameters described above were measured over 24 hours.

Evaluation

After the end of the experiment, the acquired individual data were sorted using the analysis software (Ponemah V 6.x). The blank value was assumed here to be the time 2 hours before administration, and so the selected data set encompasses the period from 7:00 am on the day of the experiment to 9:00 am on the following day.

The data were smoothed over a predefinable period by determination of the average (30-minute average) and transferred as an excel file to a storage medium. The measured values presorted and compressed in this way were transferred to Excel templates and tabulated. For each day of the experiment, the data obtained were stored in a dedicated file bearing the number of the experiment. Results and test protocols were stored in files in paper form sorted by numbers.

Literature:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

**B-5. Determination of pharmacokinetic parameters following intravenous and oral administration**

The pharmacokinetic parameters of the compounds according to the invention were determined in male Wistar rats and and/or in female beagles and/or in cynomolgus monkeys and/or in male CD-1 mice. Intravenous administration in the case of mice and rats was carried out by means of a species-specific plasma/DMSO formulation, and in the case of dogs and monkeys by means of a water/PEG400/ethanol formulation. In all species, oral administration of the dissolved substance was performed via gavage, based on a water/PEG400/ethanol formulation.

An internal standard (which may also be a chemically unrelated substance) was added to the samples of the compounds of the invention, calibration samples and qualifiers, and there follows protein precipitation by means of acetonitrile in excess. Addition of a buffer solution matched to the LC conditions, and subsequent vortexing, was followed by centrifugation at 1000 g. The supernatant was analysed by LC-MS/MS using C18 reversed-phase columns and

variable mobile phase mixtures. The substances were quantified via the peak heights or areas from extracted ion chromatograms of specific selected ion monitoring experiments.

The plasma concentration/time plots determined were used to calculate the pharmacokinetic parameters such as AUC, $C_{max}$, $t_{1/2}$ (terminal half-life), F (bioavailability), MRT (mean residence time) and CL (clearance), by means of a validated pharmacokinetic calculation program.

Since the substance quantification was performed in plasma, it was necessary to determine the blood/plasma distribution of the substance in order to be able to adjust the pharmacokinetic parameters correspondingly. For this purpose, a defined amount of substance was incubated in K3 EDTA whole blood of the species in question in a rocking roller mixer for 20 min. After centrifugation at 1000 g, the plasma concentration was measured (by means of LC-MS/MS; see above) and determined by calculating the ratio of the $C_{blood}/C_{plasma}$ value.

Table 3 shows data of representative compounds of the present invention following intravenous administration in rats:

Table 3:

| Example | $AUC_{norm}$ [kg·h/L] | $CL_{plasma}$ [L/h/kg] | $t_{1/2}$ [h] | MRT [h] |
|---|---|---|---|---|
| 2 | 5.48 | 0.18 | 4.10 | 5.83 |
| 174 (WO2012/ 058132) | 0.77 | 1.30 | 2.33 | 2.78 |

Table 4 shows data of representative compounds of the present invention following oral administration in rats:

Table 4:

| Example | $AUC_{norm}$ [kg·h/L] | $t_{1/2}$ [h] | MRT [h] | F [%] |
|---|---|---|---|---|
| 2 | 4.50 | 4.96 | 8.51 | 82.1 |
| 174 (WO2012/ 058132) | 0.63 | 3.60 | 8.41 | 81.8 |

Table 5 shows data of representative compounds of the present invention following intravenous administration in dogs:

Table 5:

| Example | $AUC_{norm}$ [kg·h/L] | $CL_{plasma}$ [L/h/kg] | $t_{1/2}$ [h] | MRT [h] |
|---|---|---|---|---|
| 2 | 37.4 | 0.03 | 7.86 | 10.3 |
| 174 (WO2012/ 058132) | 5.00 | 0.20 | 10.8 | 7.23 |

Table 6 shows data of representative compounds of the present invention following oral administration (p.o.) in dogs:

Table 6:

| Example | $AUC_{norm}$ [kg·h/L] | $t_{1/2}$ [h] | MRT [h] | F [%] |
|---|---|---|---|---|
| 2 | 31.7 | 9.28 | 13.7 | 84.8 |
| 174 (WO2012/ 058132) | 2.08 | 7.05 | 6.10 | 41.6 |

The compounds according to the present invention show superior pharmacokinetic (PK) properties in comparison to compounds disclosed in the prior art (WO 2012/058132) (see experimental part, tables 3 to 6). For instance example 2 of the present invention shows a lower plasma clearance ($CL_{plasma}$) (up to 10 times) and therefore a much higher exposure in comparison to the prior art compound disclosed as example 174 in WO 2012/058132 in rats as well as in dogs. Example 2 shows also a long half-life and mean residence time (MRT) in all tested species after p.o. (per oral) application. Due to the significantly lower plasma clearance of example 2 and the resulting very high exposure ($AUC_{norm}$, exposure, area under curve normated) with good bioavailability after p.o. application in all tested species, we see a clear superiority of pharmacokinetic (PK) properties versus example 174 dislosed in WO 2012/058132.

### B-6. Metabolic study

To determine the metabolic profile of the inventive compounds, they were incubated with recombinant human cytochrome P450 (CYP) enzymes, liver microsomes or primary fresh hepatocytes from various animal species (e.g. rats, dogs), and also of human origin, in order to obtain and to compare information about a very substantially complete hepatic phase I and phase II metabolism, and about the enzymes involved in the metabolism.

The compounds of the invention were incubated with a concentration of about 0.1-10 μM. To this end, stock solutions of the compounds of the invention having a concentration of 0.01-1 mM in acetonitrile were prepared, and then pipetted with a 1:100 dilution into the incubation mixture. The liver microsomes and recombinant enzymes were incubated at 37°C in 50 mM potassium phosphate buffer pH 7.4 with and without NADPH-generating system consisting of 1 mM $NADP^+$, 10 mM glucose-6-phosphate and 1 unit glucose-6-phosphate dehydrogenase. Primary hepatocytes were incubated in suspension in Williams E medium, likewise at 37°C. After an incubation time of 0-4 h, the incubation mixtures were stopped with acetonitrile (final concentration about 30%) and the protein was centrifuged off at about 15 000 x g. The samples thus stopped were either analyzed directly or stored at -20°C until analysis.

The analysis was carried out by high-performance liquid chromatography with ultraviolet and mass spectrometry detection (HPLC-UV-MS/MS). To this end, the supernatants of the incubation samples were chromatographed with suitable C18 reversed-phase columns and variable mobile phase mixtures of acetonitrile and 10 mM aqueous ammonium formate solution or 0.05% formic acid. The UV chromatograms in conjunction with mass spectrometry data served for identification, structural elucidation and quantitative estimation of the metabolites, and for quantitative metabolic reduction of the compound of the invention in the incubation mixtures.

### B-7. Caco-2 permeability test

The permeability of a test substance was determined with the aid of the Caco-2 cell line, an established *in vitro* model for permeability prediction at the gastrointestinal barrier (Artursson, P. and Karlsson, J. (1991). Correlation between oral drug absorption in humans and apparent drug permeability coefficients in human intestinal epithelial (Caco-2) cells. Biochem. Biophys.175 (3), 880-885). The Caco-2 cells (ACC No. 169, DSMZ, Deutsche Sammlung von Mikroorganismen und Zellkulturen, Braunschweig, Germany) were sown in 24-well plates having an insert and cultivated for 14 to 16 days. For the permeability studies, the test substance was dissolved in DMSO and diluted to the final test concentration with transport buffer (Hanks Buffered Salt Solution, Gibco/Invitrogen, with 19.9 mM glucose and 9.8 mM HEPES). In order to determine the apical to basolateral permeability ($P_{app}$A-B) of the test substance, the solution comprising the test substance was applied to the apical side of the Caco-2 cell monolayer, and transport buffer to the basolateral side. In order to determine the basolateral to apical permeability ($P_{app}$B-A) of the test substance, the solution comprising the test substance was applied to the basolateral side of the Caco-2 cell monolayer, and transport buffer to the apical side. At the start of the experiment, samples were taken from the respective donor compartment in order to ensure the mass balance. After an incubation time of two hours at 37°C, samples were taken from the two compartments. The samples were analyzed by means of LC-MS/MS and the apparent permeability coefficients ($P_{app}$) were calculated. For each cell monolayer, the permeability of Lucifer Yellow was determined to ensure cell layer integrity. In each test run, the permeability of atenolol (marker for low permeability) and sulfasalazine (marker for active excretion) was also determined as quality control.

### B-8. Solubility determination of substances in buffer pH 6.5

2-4 mg of the test compound were dissolved in DMSO to reach a concentration of 50 g/L (solution A, 515 μg/l). To 10 μl of this solution 960 μl PBS buffer pH 6.5 were added; the mixture was shaken for 24h at rt in a 96 well plate. An aliquot was centrifuged at 42000 rpm for 30 min. The supernatant was diluted with ACN/water (8:2) 1:10 and 1:1000 resp. This diluted samples were analyzed by LC-MSMS.

*Calibration:* 10 μl of solution A were diluted with 823 μl DMSO (final concentration: 600 μg/ml), which was further diluted with ACN/water 8:2 by a factor of 100 (solution B).

The calibration curve was obtained from solution B by further diluting with ACN/water 8:2 with target concentrations of 1.2 -12 - 60 - 600 ng/ml and injecting these four solutions for MS measurement.

MS method optimization:

Solution B was utilized for MS method optimization.

*PBS-Puffer:* 6.18 g sodium chloride and 3.96 g sodium dihydrogen phosphate were dissolved in 1L aqua dist., the pH was adjusted to 6.5 with 1N sodium hydroxide.

*LC-MSMS optimization:*

The following configurations were used for optimization
AB Sciex TRIPLE QUAD 4500, Agilent 1260 Infinity (G1312B), degasser (G4225A), column oven (G1316C or G1316A), CTC Analytics PAL injection system HTS-xt or HTC-xt.
Eluent A: 0.5 ml formic acid (50%ig)/ L water, Eluent B: 0.5 ml formic acid (50%ig) / L acetonitrile

| time [min] | flow [μl/min] | %B |
|---|---|---|
| 0.00 | 200 | 70 |
| 0.08 | 200 | 70 |
| 0.09 | 25 | 70 |
| 0.60 | 25 | 70 |
| 0.65 | 200 | 70 |
| 1.10 | 200 | 70 |

| | |
|---|---|
| Autosampler: | without auto inject ahead setting |
| column: | stainless steel capillary |
| oven temperature: | 22°C |
| flow rate: | flow gradient |
| injected volume: | 2 μl |

Water Quattro Micro MS, Agilent 1100 (G1312A), degasser (G1322A), column oven (G1316A), CTC Analytics PAL injection system HTS, eluents as above time [min] flow [μl/min] % B

| | | |
|---|---|---|
| 0.00 | 250 | 70 |
| 1.50 | 250 | 70 |

| | |
|---|---|
| Autosampler: | with auto inject ahead setting |
| column: | stainless steel capillary |
| oven temperature: | 22°C |
| flow rate: | flow gradient |
| injected volume: | 5 μl |
| MS method: | Flow Injection Analysis (FIA) for optimization ("MS-OPTI"); Ionization mode ABSciex-MS: ESI-pos/neg, Waters-MS: ESI-pos |

*HPLC method for MSMS quantification:*

Eluent A, B as above

ABSciex-MS

| time [min] | % A | % B |
|---|---|---|
| 0 | 90 | 10 |
| 0.5 | 5 | 95 |
| 0.84 | 5 | 95 |
| 0.85 | 90 | 10 |
| 1.22 | 90 | 10 |

| | |
|---|---|
| Autosampler: | without auto inject ahead setting |
| column: | Waters OASIS HLB, 2,1 x 20 mm, 25 μ |
| column temperature: | 30°C |
| flow rate: | 2.5 ml |
| injected volume: | 2 μl |
| Splitter (before MS) | 1:20 |

Waters-MS

| time [min] | % A | % B |
|---|---|---|
| 0 | 90 | 10 |
| 0.5 | 5 | 95 |
| 0.84 | 5 | 95 |
| 0.85 | 90 | 10 |
| 1.5 | 90 | 10 |

| | |
|---|---|
| Autosampler: | with auto inject ahead setting |
| column: | Waters OASIS HLB, 2,1 x 20 mm, 25 μ |
| column temperature: | 30°C |
| flow rate: | 2.5 ml |
| injected volume: | 5 μl |
| Splitter (before MS) | 1:20 |
| MS method: | Multiple Reaction Monitoring (MRM) |

**B-9. Determination of solubility from solid**

For each solvent, an Eppendorf plastic vial was charged with 0.5 - 1 mg of the test compound (exact weight), 2-3 glass pearls (diameter 3 mm) and 1.0 ml of the respective solvent. The vial was closed and shaken at RT for 24 h (1400 rpm; Thermomixer, Eppendorf). Thereafter, 230 μl each of the solution/suspension was transferred into one or more centrifuge vials (Beckman Coulter) and were centrifuged at 42000 rpm for 30 min (Beckman Coulter Optima L90). At least 100 μl of the supernatant were withdrawn and further diluted with DMSO in two dilution strength: 1:5 and 1:50 (the latter obtained from the 1:5 dilution step by subsequent DMSO addition). This liquid handling was done either manually or with the help of a pipetting robot (Lissy, Zinsser Analytic).

For HPLC quantification, calibration solutions of the test compound in DMSO were prepared. Starting from an initial concentration of 600 μg/ml, three calibration solutions were prepared: 100 μg/ml, 20 μg/ml and 2.5 μg/ml (manually or via Lissy).

Both calibration solutions and the supernatant were analyzed by HPLC/UV-detection at an appropriate wave length. The solubility was determined using the linear calibration curve.

HPLC systems:

Hewlett Packard / Agilent HPLC systems, G1311A+G1316A+G1315B as well as G1312A+G1316A+G1315A

injector system: CTC-Analytik HTC PAL

or with a Agilent UPLC System (G7117C, G7116B, G7167B and G7120)

oven temperature: 30°C, detection: 210 and/or 254 nm, injected volume: 20μl

eluent A: 0.1% TFA in water, eluent B: 0.1% TFA in acetonitrile

column: ZORBAX Extend-C18, 3.0 x 50mm, 3.5μm

Gradient:

| time [min] | A [%] | B [%] | Flow rate: [ml/min] |
|---|---|---|---|
| 0.0 | 98 | 2 | 1.5 |
| 0.2 | 98 | 2 | 1.5 |
| 3.3 | 10 | 90 | 1.5 |
| 4.0 | 10 | 90 | 1.5 |
| 4.1 | 98 | 2 | 2.5 |
| 4.7 | 98 | 2 | 2.5 |
| 5.0 | 98 | 2 | 1.5 |

## C. Working examples of pharmaceutical compositions

The compounds of the invention can be converted to pharmaceutical preparations as follows:

### Tablet:

*Comnosition:*

100 mg of the compound according to the invention, 50 mg of lactose (monohydrate), 50 mg of corn starch (native), 10 mg of polyvinylpyrrolidone (PVP 25) (from BASF, Ludwigshafen, Germany) and 2 mg of magnesium stearate.
Tablet weight 212 mg. Diameter 8 mm, radius of curvature 12 mm.

*Production:*

The mixture of compound of the invention, lactose and starch is granulated with a 5% solution (w/w) of the PVP in water. The granules are dried and then mixed with the magnesium stearate for 5 minutes. This mixture is compressed using a conventional tableting press (see above for format of the tablet). The guide value used for the pressing is a pressing force of 15 kN.

### Suspension for oral administration:

*Composition:*

1000 mg of the compound of the invention, 1000 mg of ethanol (96%), 400 mg of Rhodigel® (xanthan gum from FMC, Pennsylvania, USA) and 99 g of water.
10 ml of oral suspension correspond to a single dose of 100 mg of the compound of the invention.

*Production:*

The Rhodigel is suspended in ethanol; the compound of the invention is added to the suspension. The water is added while stirring. The mixture is stirred for about 6 h until the swelling of the Rhodigel is complete.

### Solution for oral administration:

*Composition:*

500 mg of the compound of the invention, 2.5 g of polysorbate and 97 g of polyethylene glycol 400. 20 g of oral solution correspond to a single dose of 100 mg of the compound of the invention.

*Production:*

The compound of the invention is suspended in the mixture of polyethylene glycol and polysorbate with stirring. The stirring process is continued until the compound according to the invention has completely dissolved.

**i.v. solution:**

The compound according to the invention is dissolved in a concentration below the saturation solubility in a physiologically tolerated solvent (e.g. isotonic saline, 5% glucose solution and/or 30% PEG 400 solution). The solution is sterilized by filtration and used to fill sterile and pyrogen-free injection containers.

## Claims

**1.** Compound of the formula (I)

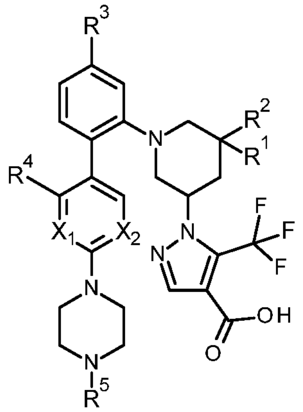

(I),

in which

$R^1$ represents hydrogen or halogen,
$R^2$ represents hydrogen or halogen,
$R^3$ represents chloro or trifluoromethyl,
$R^4$ represents hydrogen or $C_1$-$C_4$-alkyl
$R^5$ represents $C_1$-$C_6$-alkyl
$X_1$ represents nitrogen or carbon
$X_2$ represents nitrogen or carbon

or one of the salts thereof, solvates thereof or solvates of the salts thereof.

**2.** Compound according to Claim 1, **characterized in that**

$R^1$ represents hydrogen, fluorine
$R^2$ represents hydrogen, fluorine
$R^3$ represents chloro or trifluoromethyl
$R^4$ represents hydrogen or methyl
$R^5$ represents isobutyl
$X_1$ represents nitrogen or carbon
$X_2$ represents nitrogen or carbon

or one of the salts thereof, solvates thereof or solvates of the salts thereof.

**3.** Compound according to Claim 1 or 2, of the formula

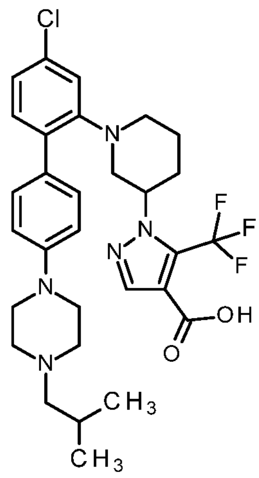

or one of the salts thereof, solvates thereof or solvates of the salts thereof.

**4.** A process for preparing a compound of the formula (I) or one of the salts thereof, solvates thereof or solvates of the salts thereof according to one of Claims 1 to 3, **characterized in that**

in a first step [B] the compounds of the formula (IV)

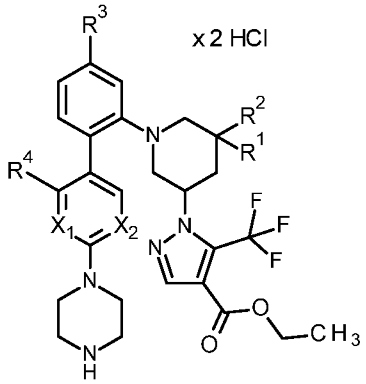

(IV),

in which $R^1$, $R^2$, $R^3$, $R^4$ and $X_1$ and $X_2$ are defined as above,
are reacted with compounds of the formula (III)

$R^{5a}$-CHO (III),

in which $R^{5a}$ represents $C_1$-$C_3$-alkyl, preferably isopropyl,
in the presence of a reducing agent, a suitable solvent and a base to provide compounds of the formula (II)

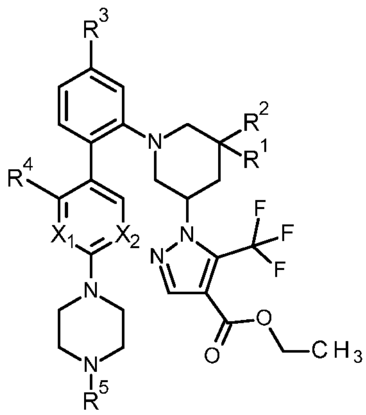

(II)

in which $R^1$, $R^2$, $R^3$, $R^4$, $R^5$ and $X_1$ and $X_2$ are defined as above
and
in a second step [A]
compounds of formula (II) are reacted with a base to provide compounds of the formula (I),

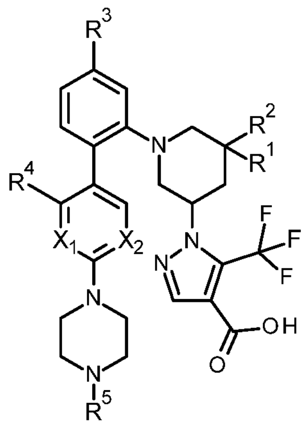

(I)

in which $R^1$, $R^2$, $R^3$, $R^4$ , $R^5$ and $X_1$ and $X_2$ are defined as above
optionally compounds of formula (I) are transferred in a third step [A]*
into the corresponding salts of formula (Ia)

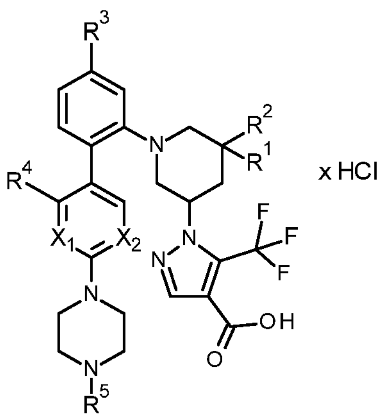

(Ia)

in the presence of a suitable acid in a suitable solvent.

**5.** A compound according to any of Claims 1 to 3 for use in the treatment and/or prophylaxis of diseases.

6. A compound according to any of Claims 1 to 3 for use in the treatment and/or prophylaxis of heart failure (HFrEF, HFmrEF and HFpEF), hypertension (HTN), chronic and diabetic kidney disease (CKD, DKD), pulmonary hypertension (PH), systemic sclerosis (SSc), sickle cell disease (SCD), neurodegenerative diseases and dementias, and diabetic foot ulcer (DFU).

7. Use of a compound according to any of Claims 1 to 3 for producing a medicament for use in the treatment and/or prophylaxis of diseases.

8. Use of a compound according to any of Claims 1 to 3 for producing a medicament for use in the treatment and/or prophylaxis of heart failure (HFrEF, HFmrEF and HFpEF), hypertension (HTN), chronic and diabetic kidney disease (CKD, DKD), pulmonary hypertension (PH), systemic sclerosis (SSc), sickle cell disease (SCD), neurodegenerative diseases and dementias, and diabetic foot ulcer (DFU).

9. Medicament comprising a compound according to any of Claims 1 to 3 in combination with an inert, nontoxic, pharmaceutically suitable excipient.

10. Medicament according to Claim 9 for use in the treatment and/or prophylaxis of heart failure (HFrEF, HFmrEF and HFpEF), hypertension (HTN), chronic and diabetic kidney disease (CKD, DKD), pulmonary hypertension (PH), systemic sclerosis (SSc), sickle cell disease (SCD), neurodegenerative diseaseses and dementias, and diabetic foot ulcer (DFU).

**Patentansprüche**

1. Verbindung der Formel (I)

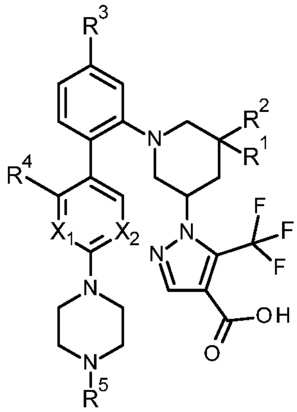
(I),

in der

$R^1$ für Wasserstoff oder Halogen steht,
$R^2$ für Wasserstoff oder Halogen steht,
$R^3$ für Chlor oder Trifluormethyl steht,
$R^4$ für Wasserstoff oder $C_1$-$C_4$-Alkyl steht,
$R^5$ für $C_1$-$C_6$-Alkyl steht,
$X_1$ für Stickstoff oder Kohlenstoff steht,
$X_2$ für Stickstoff oder Kohlenstoff steht,

oder eines ihrer Salze, ihrer Solvate oder der Solvate ihrer Salze.

2. Verbindung nach Anspruch 1, **dadurch gekennzeichnet, dass**

$R^1$ für Wasserstoff oder Fluor steht,
$R^2$ für Wasserstoff oder Fluor steht,
$R^3$ für Chlor oder Trifluormethyl steht,

$R^4$ für Wasserstoff oder Methyl steht,
$R^5$ für Isobutyl steht,
$X_1$ für Stickstoff oder Kohlenstoff steht,
$X_2$ für Stickstoff oder Kohlenstoff steht,

oder eines ihrer Salze, ihrer Solvate oder der Solvate ihrer Salze.

**3.** Verbindung nach Anspruch 1 oder 2 der Formel

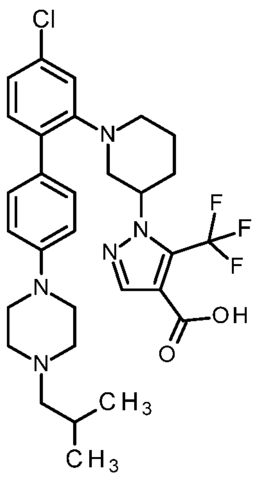

oder eines ihrer Salze, ihrer Solvate oder der Solvate ihrer Salze.

**4.** Verfahren zur Herstellung einer Verbindung der Formel (I) oder eines ihrer Salze, ihrer Solvate oder der Solvate ihrer Salze nach einem der Ansprüche 1 bis 3, **dadurch gekennzeichnet, dass**

in einem ersten Schritt [B] die Verbindungen der Formel (IV)

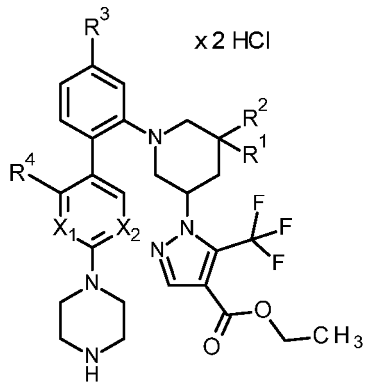

(IV),

in der $R^1$, $R^2$, $R^3$, $R^4$ und $X_1$ und $X_2$ wie oben definiert sind,
mit Verbindungen der Formel (III)

$R^{5a}$-CHO (III),

in der $R^{5a}$ für $C_1$-$C_3$-Alkyl, vorzugsweise Isopropyl, steht,
in Gegenwart eines Reduktionsmittels, eines geeigneten Lösungsmittels und einer Base zu Verbindungen der Formel (II)

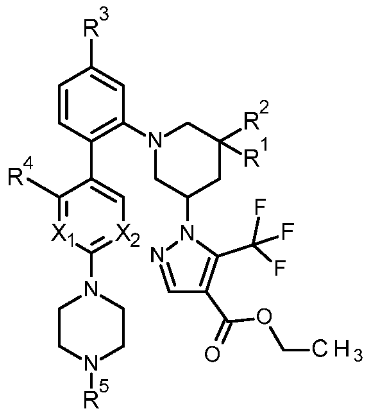

(II)

in der $R^1$, $R^2$, $R^3$, $R^4$, $R^5$ und $X_1$ und $X_2$ wie oben definiert sind, umgesetzt werden und
in einem zweiten Schritt [A]
Verbindungen der Formel (II) mit einer Base zu Verbindungen der Formel (I),

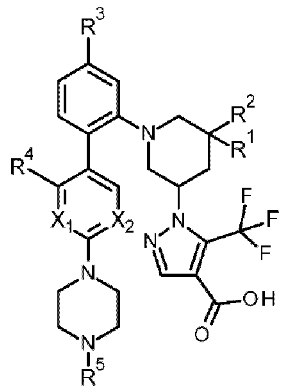

(I)

in der $R^1$, $R^2$, $R^3$, $R^4$, $R^5$ und $X_1$ und $X_2$ wie oben definiert sind, umgesetzt werden
gegebenenfalls Verbindungen der Formel (I) in einem dritten Schritt [A]*
in die entsprechenden Salze der Formel (Ia) überführt werden

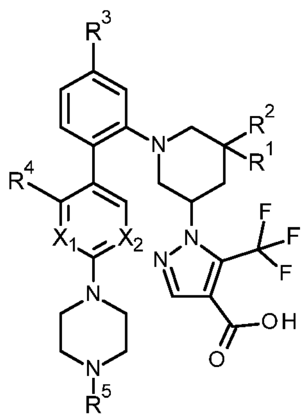

x HCl

(Ia)

in Gegenwart einer geeigneten Säure in einem geeigneten Lösungsmittel.

**5.** Verbindung nach einem der Ansprüche 1 bis 3 zur Verwendung bei der Behandlung und/oder Prophylaxe von Krankheiten.

6. Verbindung nach einem der Ansprüche 1 bis 3 zur Verwendung bei der Behandlung und/oder Prophylaxe von Herzinsuffizienz (HFrEF, HFmrEF und HFpEF), Hypertonie (HTN), chronischer und diabetischer Nierenkrankheit (CKD, DKD), pulmonaler Hypertonie (PH), systemischer Sklerose (SSc), Sichelzellkrankheit (SCD), neurodegenerativen Krankheiten und Demenzen und diabetischem Fußulcus (DFU).

7. Verwendung einer Verbindung nach einem der Ansprüche 1 bis 3 zur Herstellung eines Medikaments zur Verwendung bei der Behandlung und/oder Prophylaxe von Krankheiten.

8. Verwendung einer Verbindung nach einem der Ansprüche 1 bis 3 zur Herstellung eines Medikaments zur Verwendung bei der Behandlung und/oder Prophylaxe von Herzinsuffizienz (HFrEF, HFmrEF und HFpEF), Hypertonie (HTN), chronischer und diabetischer Nierenkrankheit (CKD, DKD), pulmonaler Hypertonie (PH), systemischer Sklerose (SSc), Sichelzellkrankheit (SCD), neurodegenerativen Krankheiten und Demenzen und diabetischem Fußulcus (DFU).

9. Medikament, umfassend eine Verbindung nach einem der Ansprüche 1 bis 3 in Kombination mit einem inerten, nichttoxischen, pharmazeutisch geeigneten Hilfsstoff.

10. Medikament nach Anspruch 9 zur Verwendung bei der Behandlung und/oder Prophylaxe von Herzinsuffizienz (HFrEF, HFmrEF und HFpEF), Hypertonie (HTN), chronischer und diabetischer Nierenkrankheit (CKD, DKD), pulmonaler Hypertonie (PH), systemischer Sklerose (SSc), Sichelzellkrankheit (SCD), neurodegenerativen Krankheiten und Demenzen und diabetischem Fußulcus (DFU).

**Revendications**

1. Composé de la formule (I)

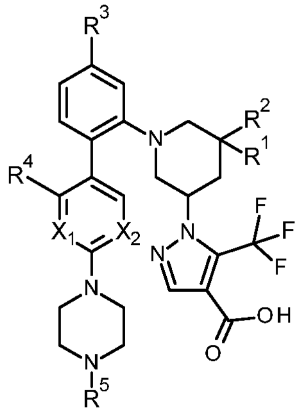

(I),

$R^1$ représentant hydrogène ou halogène,
$R^2$ représentant hydrogène ou halogène,
$R^3$ représentant chloro ou trifluorométhyle,
$R^4$ représentant hydrogène ou $C_1$-$C_4$-alkyle
$R^5$ représentant $C_1$-$C_6$-alkyle
$X_1$ représentant azote ou carbone
$X_2$ représentant azote ou carbone

ou un des sels correspondants, des solvates correspondants ou des solvates des sels correspondants.

2. Composé selon la revendication 1, **caractérisé en ce que**

$R^1$ représente hydrogène, fluor
$R^2$ représente hydrogène, fluor
$R^3$ représente chloro ou trifluorométhyle
$R^4$ représente hydrogène ou méthyle

$R^5$ représente isobutyle
$X_1$ représente azote ou carbone
$X_2$ représentant azote ou carbone

ou un dès sels correspondants, dès soivaues correspondants ou des solvates des sels correspondants.

**3.** Composé selon la revendication 1 ou 2, de la formule

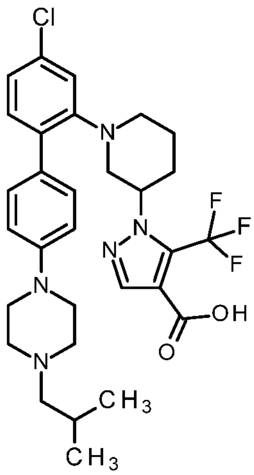

ou un des sels correspondants, des solvates correspondants ou des solvates des sels correspondants.

**4.** Procédé pour la préparation d'un composé de la formule (I) ou un des sels correspondants, des solvates correspondants ou des solvates des sels correspondants selon l'une des revendications 1 à 3, **caractérisé en ce que**

dans une première étape [B] les composés de la formule (IV)

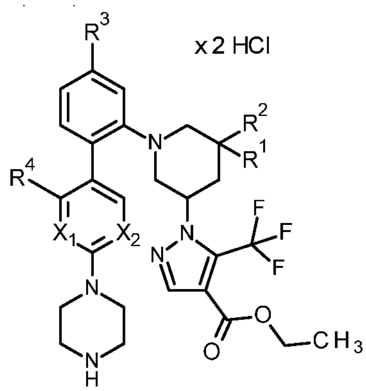

(IV),

dans laquelle $R^1$, $R^2$, $R^3$, $R^4$ et $X_1$ et $X_2$ sont définis comme ci-dessus,
sont mis à réagir avec des composés de la formule (III) $R^{5a}$-CHO (III),
dans laquelle $R^{5a}$ représente $C_1$-$C_3$-alkyle, préférablement isopropyle,
en la présence d'un agent réducteur, d'un solvant approprié et d'une base pour fournir des composés de la formule (II)

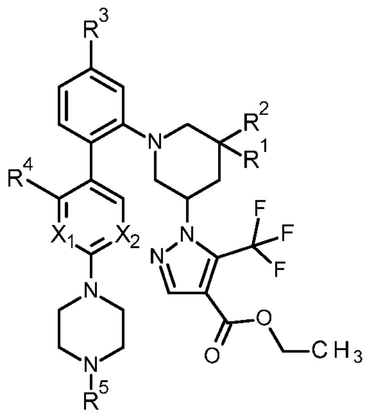

(II)

dans laquelle $R^1$, $R^2$, $R^3$, $R^4$, $R^5$ et $X_1$ et $X_2$ sont définis comme ci-dessus
et
dans une deuxième étape [A]
des composés de formule (II) sont mis à réagir avec une base pour fournir des composés de la formule (I),

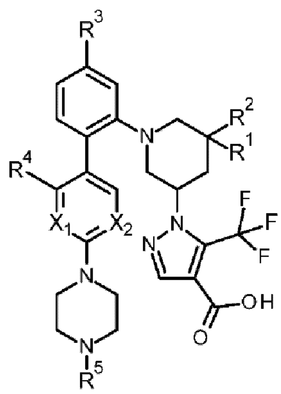

(I)

dans laquelle $R^1$, $R^2$, $R^3$, $R^4$, $R^5$ et $X_1$ et $X_2$ sont définis comme ci-dessus
éventuellement des composés de formule (I) sont transférés dans une troisième étape [A]*
en les sels correspondants de formule (Ia)

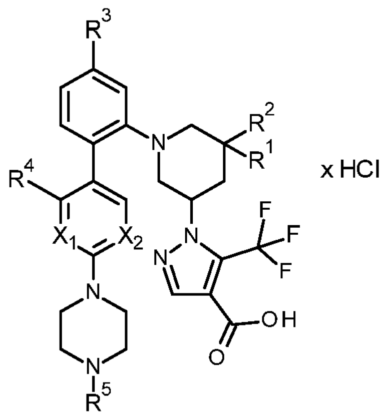

(Ia)

en la présence d'un acide approprié dans un solvant approprié.

**5.** Composé selon l'une quelconque des revendications 1 à 3 pour une utilisation dans le traitement et/ou la prophylaxie de maladies.

**6.** Composé selon l'une quelconque des revendications 1 à 3 pour une utilisation dans le traitement et/ou la prophylaxie de l'insuffisance cardiaque (ICFEr, ICFEm et ICFEp), de l'hypertension (HTA), de la maladie rénale chronique et diabétique (MRC, MRD), de l'hypertension pulmonaire (HP), de la sclérodermie systémique (SSc), de la drépanocytose (SCD), des maladies neurodégénératives et des démences, et de l'ulcère du pied diabétique (UPD).

**7.** Utilisation d'un composé selon l'une quelconque des revendications 1 à 3 pour la production d'un médicament pour une utilisation dans le traitement et/ou la prophylaxie de maladies.

**8.** Utilisation d'un composé selon l'une quelconque des revendications 1 à 3 pour la production d'un médicament pour une utilisation dans le traitement et/ou la prophylaxie de l'insuffisance cardiaque (ICFEr, ICFEm et ICFEp), de l'hypertension (HTA), de la maladie rénale chronique et diabétique (MRC, MRD), de l'hypertension pulmonaire (HP), de la sclérodermie systémique (SSc), de la drépanocytose (SCD), des maladies neurodégénératives et des démences, et de l'ulcère du pied diabétique (UPD).

**9.** Médicament comprenant un composé selon l'une quelconque des revendications 1 à 3 en combinaison avec un excipient inerte, non toxique, pharmaceutiquement acceptable.

**10.** Médicament selon la revendication 9 pour une utilisation dans le traitement et/ou la prophylaxie de l'insuffisance cardiaque (ICFEr, ICFEm et ICFEp), de l'hypertension (HTA), de la maladie rénale chronique et diabétique (MRC, MRD), de l'hypertension pulmonaire (HP), de la sclérodermie systémique (SSc), de la drépanocytose (SCD), des maladies neurodégénératives et des démences, et de l'ulcère du pied diabétique (UPD).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012139888 A
- WO 2012058132 A
- WO 2010105770 A
- WO 2011104322 A
- WO 2016071212 A
- WO 0006568 A
- WO 0006569 A
- WO 0242301 A
- WO 03095451 A
- WO 2011147809 A
- WO 2012004258 A
- WO 2012028647 A
- WO 2012059549 A
- WO 0119355 A
- WO 0119776 A
- WO 0119778 A
- WO 0119780 A
- WO 02070462 A
- WO 02070510 A


### Non-patent literature cited in the description


- **O.V. EVGENOV et al.** *Nature Rev. Drug Disc.,* 2006, vol. 5, 755
- **J.P. STASCH et al.** *J. Clin. Invest.,* 2006, vol. 116, 2552
- **HAHN et al.** *Drugs Future,* 2018, vol. 43, 738
- **HASSAN J. et al.** *Chem. Rev.,* 2002, vol. 102, 1359-1469
- *CHEMICAL ABSTRACTS,* 1445085-55-1
- *CHEMICAL ABSTRACTS,* 564483-18-7
- *CHEMICAL ABSTRACTS,* 161265-03-8
- *CHEMICAL ABSTRACTS,* 1070663-78-3
- *CHEMICAL ABSTRACTS,* 657408-07-6
- *CHEMICAL ABSTRACTS,* 787618-22-8
- **S. L. BUCHWALD et al.** *Chem. Sci.,* 2013, vol. 4, 916
- **D. MONTANA ; G. SIMONNEAU et al.** Pulmonary Circulation. Diseases and their treatment,. Hodder Amold Publ, 2011, 197-206
- **M.M. HOEPER et al.** *J. Am. Coll. Cardiol.,* 2009, vol. 54 (1), S85-S96
- *CHEMICAL ABSTRACTS,* 148893-10-1
- *CHEMICAL ABSTRACTS,* 51364-51-3
- *CHEMICAL ABSTRACTS,* 14221-01-3
- *CHEMICAL ABSTRACTS,* 1445085-77-7
- *CHEMICAL ABSTRACTS,* 989-36-7
- *CHEMICAL ABSTRACTS,* 5331-43-1
- *CHEMICAL ABSTRACTS,* 695-96-5
- *CHEMICAL ABSTRACTS,* 78-84-2
- **F. WUNDER et al.** *Anal. Biochem.,* 2005, vol. 339, 104-112
- **STASCH et al.** *Br. J. Pharmacol.,* 2002, vol. 135, 344-355
- **KLAUS WITTE ; KAI HU ; JOHANNA SWIATEK ; CLAUDIA MÜSSIG ; GEORG ERTL ; BJÖRN LEMMER.** Experimental heart failure in rats: effects on cardiovascular circadian rhythms and on myocardial β-adrenergic signaling. *Cardiovasc Res,* 2000, vol. 47 (2), 203-405
- **KOZO OKAMOTO.** Spontaneous hypertension in rats. *Int Rev Exp Pathol,* 1969, vol. 7, 227-270
- **MAARTEN VAN DEN BUUSE.** Circadian Rhythms of Blood Pressure, Heart Rate, and Locomotor Activity in Spontaneously Hypertensive Rats as Measured With Radio-Telemetry. *Physiology & Behavior,* 1994, vol. 55 (4), 783-787
- **ARTURSSON, P. ; KARLSSON, J.** Correlation between oral drug absorption in humans and apparent drug permeability coefficients in human intestinal epithelial (Caco-2) cells. *Biochem. Biophys.,* 1991, vol. 175 (3), 880-885